# EUROPEAN PATENT SPECIFICATION

(11) **EP 2 262 911 B1**
(45) Date of publication and mention of the grant of the patent: **12.10.2016**
(21) Application number: 09732614.4
(22) Date of filing: 17.04.2009
(51) Int. Cl.: C12N 1/06, C12N 15/10, C12Q 1/68

(54) **COMPOSITIONS, METHODS, AND KITS USING SYNTHETIC PROBES FOR DETERMINING THE PRESENCE OF A TARGET NUCLEIC ACID**
ZUSAMMENSETZUNGEN, VERFAHREN UND KITS MIT SYNTHETISCHEN SONDEN ZUM NACHWEIS DER ANWESENHEIT EINER TARGET-NUKLEINSÄURE
COMPOSITIONS, PROCÉDÉ ET TROUSSES UTILISANT DES SONDES SYNTHÉTIQUES POUR DÉTERMINER LA PRÉSENCE D'UN ACIDE NUCLÉIQUE VOULU

(30) Priority: 17.04.2008 US 45952 P; 12.11.2008 US 113841 P; 28.01.2009 US 147862 P
(43) Date of publication of application: 22.12.2010
(73) Proprietor: QIAGEN Gaithersburg, Inc., Gaithersburg, MD 20878 (US)
(72) Inventor: NAZARENKO, Irina, Gaithersburg, MD 20878 (US); O'NEIL, Dominic, Gaithersburg, MD 20878 (US); PACHOWICZ, Karolina, Gaithersburg, MD 20878 (US)
(74) Representative: Roth, Carla
(86) International application number: PCT/US2009/041033
(87) International publication number: WO 2009/129505

(56) References cited:
- EP-A2- 0 163 220
- WO-A1-93/10263
- US-A- 4 743 535
- US-A1- 2006 160 188
- NAZARENKO I ET AL: "A novel method of HPV genotyping using Hybrid Capture<(>R) sample preparation method combined with GP5+/6+ PCR and multiplex detection on Luminex<(>R) XMAP<(>R)", JOURNAL OF VIROLOGICAL METHODS, ELSEVIER BV, NL LNKD- DOI:10.1016/J.JVIROMET.2008.09.002, vol. 154, no. 1-2, 22 October 2008 (2008-10-22), pages 76-81, XP0025680302, ISSN: 0166-0934 [retrieved on 2008-10-22]
- GENTECH DIAGNOSTICS: "Digene TM HBV Test Hybrid Capture TM II", INTERNET CITATION , 6 June 2008 (2008-06-06), pages 1-3, XP002560368, Retrieved from the Internet: URL:http://www.gentechin.com/hbvdnatestkit .htm [retrieved on 2009-12-11]
- HUANG SHANG-LANG ET AL: "Comparison between the Hybrid Capture II Test and an SPF1/GP6+ PCR-based assay for detection of human papillomavirus DNA in cervical swab samples.", JOURNAL OF CLINICAL MICROBIOLOGY MAY 2006 LNKD- PUBMED:16672400, vol. 44, no. 5, May 2006 (2006-05), pages 1733-1739, XP002665103, ISSN: 0095-1137
- SANDRI MARIA T ET AL: "Comparison of the Digene HC2 assay and the Roche AMPLICOR human papillomavirus (HPV) test for detection of high-risk HPV genotypes in cervical samples", 1 June 2006 (2006-06-01), JOURNAL OF CLINICAL MICROBIOLOGY, AMERICAN SOCIETY FOR MICROBIOLOGY, WASHINGTON, DC, US, PAGE(S) 2141 - 2146, XP002560370, ISSN: 0095-1137 * page 2141, right-hand column, paragraph 2 - page 2142, left-hand column, paragraph 2 * * page 2142, left-hand column, paragraph 5 *
- HANTZ S ET AL: "[Evaluation of accuracy of three assays for human papillomavirus detection and typing: Hybrid Capture 2, HPV Consensus kit and Amplicor HPV.]", 1 February 2008 (2008-02-01), PATHOLOGIE ET BIOLOGIE, L'EXPANSION SCIENTIFIQUE FRANCAISE, PARIS, FR, PAGE(S) 29 - 35, XP002560369, ISSN: 0369-8114 [retrieved on 2008-01-01] * page 30, right-hand column, paragraph 2 - page 32, left-hand column, paragraph 4; figure 1 *
- TAHA ET AL: "Universal Collection Medium (UCM)<(>R) is as suitable as the Standard Transport Medium (STM)<(>R) for Hybrid Capture II<(>R) (HC-2) assay", JOURNAL OF CLINICAL VIROLOGY, ELSEVIER, AMSTERDAM, NL LNKD- DOI:10.1016/J.JCV.2005.12.011, vol. 36, no. 1, 1 May 2006 (2006-05-01), pages 32-35, XP005367693, ISSN: 1386-6532
- KRISTJAN SIGURDSSON ET AL.: 'Human papillomavirus (HPV) in an icelandic population: the role of HPV DNA testing based on hybrid capture and PCR assays among women with screen- detected abnormal PAP smears.' INTERNATIONAL JOURNAL OF CANCER. vol. 72, no. 3, July 1997, pages 446 - 452, XP008142307
- ETHEL-MICHELE DE VILLIERS ET AL.: 'Classification of papillomaviruses.' VIROLOGY. vol. 324, no. 1, June 2004, pages 17 - 27, XP004512668
- DATABASE GENBANK 18 March 1994 'Human papillomavirus type 16 (HPV16), complete genome.', XP008142331 Database accession no. K02718
- DATABASE GENBANK 18 April 2005 'Human papillomavirus type 18 E6, E7, E1, E2, E4, E5, L1 & L2 genes.', XP008142317 Database accession no. X05015
- DATABASE GENBANK 18 April 2005 'Human papillomavirus type 45 genomic DNA.', XP008142326 Database accession no. X74479
- DATABASE GENBANK 18 March 1994 'Human papillomavirus type 31 (HPV-31), complete genome.', XP008142337 Database accession no. J04353
- DATABASE GENBANK 02 March 1994 'Human papillomavirus type 33, complete genome.', XP008142328 Database accession no. M12732
- DATABASE GENBANK 11 May 2002 'Human papillomavirus type 35, complete genome.', XP008142338 Database accession no. M74117
- DATABASE GENBANK 26 January 2001 'Human papillomavirus ORFs.', XP008142333 Database accession no. M62849
- DATABASE GENBANK 29 October 1999 'Human papillomavirus type 51 genomic DNA, partial sequence.', XP008142334 Database accession no. M62877
- DATABASE GENBANK 18 April 2005 'Human papillomavirus type 52 genomic DNA.', XP008142381 Database accession no. X74481
- DATABASE GENBANK 18 April 2005 'Human papillomavirus type 56 genomic DNA.', XP008142335 Database accession no. X74483
- DATABASE GENBANK 07 December 2007 'Human papillomavirus type 58, complete genome.', XP008142382 Database accession no. D90400
- DATABASE GENBANK 18 April 2005 'Human papilloma virus type 59, complete viral genome.', XP008142336 Database accession no. X77858
- DATABASE GENBANK 18 October 1995 'Human papillomavirus type 66, complete genome.', XP008142339 Database accession no. U31794
- DATABASE GENBANK 18 April 2005 'Human papilloma virus L1 gene for major capsid protein.', XP008142383 Database accession no. X67161
- DATABASE GENBANK 22 June 2000 'Human papillomavirus type 82 DNA, complete genome.', XP008142380 Database accession no. AB027021
- BERNHARD KLETER ET AL.: 'Development and clinical evaluation of a highly sensitive PCR- reverse hybridization line probe assay for detection and identification of anogenital human papillomavirus.' JOURNAL OF CLINICAL MICROBIOLOGY. vol. 37, no. 8, August 1999, pages 2508 - 2517, XP002247920

## Description

### FIELD OF THE INVENTION

The present disclosure relates to compositions, methods, and kits using synthetic probes for determining the presence of a target nucleic acid in a biological sample.

### BACKGROUND OF THE INVENTION

The detection and characterization of specific nucleic acid sequences and sequence changes have been utilized to detect the presence of viral or bacterial nucleic acid sequences indicative of an infection, the presence of variants or alleles of mammalian genes associated with disease and cancers, and the identification of the source of nucleic acids found in forensic samples, as well as in paternity determinations.

For example, the RNA or DNA for many microorganisms and viruses have been isolated and sequenced. Nucleic acid probes have been examined for a large number of infections. Detectable nucleic acid sequences that hybridize to complementary RNA or DNA sequences in a test sample have been previously utilized. Detection of the probe indicates the presence of a particular nucleic acid sequence in the test sample for which the probe is specific. In addition to aiding scientific research, DNA or RNA probes can be used to detect the presence of viruses and microorganisms such as bacteria, yeast and protozoa as well as genetic mutations linked to specific disorders in patient samples. Nucleic acid hybridization probes have the advantages of high sensitivity and specificity over other detection methods and do not require a viable organism. Hybridization probes can be labeled, for example with a radioactive substance that can be easily detected.

As nucleic acid sequence data for genes from humans and pathogenic organisms accumulates, the demand for fast, cost-effective, and easy-to-use tests increases. It would be desirable to provide novel and effective methods, compositions, and kits for determining a target nucleic acid in a sample.

### SUMMARY OF THE INVENTION

In one aspect, the present invention provides a method for determining the presence of a target nucleic acid in a sample, wherein the target nucleic acid is a DNA, the method comprising:
a) contacting a probe set comprising more than one polynucleotide probes with the sample under a hybridization condition sufficient for the more than one polynucleotide probes to hybridize to the target DNA in the sample to form double-stranded nucleic acid hybrids, wherein the polynucleotide probes do not hybridize to a variant of the target nucleic acid and wherein the polynucleotide probes of the probe set are short RNA probes having a length from 20 to 60 nucleotides; and
b) detecting the double-stranded nucleic acid hybrids, wherein detecting comprises contacting the double-stranded nucleic acid hybrids with a first anti-hybrid antibody that is immunospecific to double-stranded nucleic acid hybrids to capture the double-stranded nucleic acid hybrids, wherein the first anti-hybrid antibody is directly or indirectly immobilized onto a solid support and wherein the step of detecting further comprises providing a second anti-hybrid antibody that is immunospecific to double-stranded nucleic acid hybrids, wherein the second anti-hybrid antibody is detectably labelled either directly or indirectly, whereby detection of the double-stranded nucleic acid hybrids determines the target DNA in the sample.

In another aspect of the invention, the hybridization of the nucleic acids and detection of the double-stranded nucleic acid hybrids are performed at the same time.

In a further aspect of the invention, after the double-stranded nucleic acid hybrids are contacted with a first anti-hybrid antibody that is immunospecific to double-stranded nucleic acid hybrids, a second anti-hybrid antibody is added to detect the double-stranded nucleic acid hybrids whereby detection of the double-stranded nucleic acid hybrids by these second anti-hybrid antibodies determines the presence of target nucleic acid in the sample.

In another aspect of the invention, synthetic RNA probes corresponding to more than one HPV type are used to detect for the presence of HPV infection.

In certain embodiments, the detecting further comprises providing a second anti-hybrid antibody that is immunospecific to double-stranded nucleic acid hybrids, wherein the second anti-hybrid antibody is detectably labeled.

In certain embodiments, the at least one probe and the anti-hybrid antibody are added in the same step.

The target DNA may be an HPV nucleic acid and in certain embodiments, it is a high risk HPV type and the variant is a low risk type or another high risk type HPV nucleic acid. In certain embodiments, the hrHPV type is 16, 18 and/or 45.

In certain embodiments the polynucleotide probes consist essentially of a sequence or a complement thereof selected from the group consisting of SEQ ID NOs: 1-2026.

The present invention provides for a method of determing the presence of an HPV target nucleic acid in a sample wherein if the target nucleic acid is HPV 16, the polynucleotide probes is a set of nucleic acid probes comprising at least one nucleic acid sequence chosen from the group consisting of: SEQ ID NOs: 1-162. A method for determining the presence of HPV 16 DNA in a sample is provided, the method comprising:
a) contacting more than one polynucleotide probes or a complement thereof with the sample under a hybridization condition sufficient to allow the polynucleotides to anneal to a corresponding complementary nucleic acid sequence in the sample to form double-stranded nucleic acid hybrids, wherein the more than one polynucleotide probes is a set of nucleic acid probes, wherein the polynucleotides comprised in the probe set are short RNA probes having a length from 20 to 60 nucleotides and wherein the set comprises at least one nucleic acid sequence chosen from the group consisting of: SEQ ID NOs:I-162; and
b) detecting double-stranded nucleic acid hybrids, wherein detecting comprises contacting the double-stranded nucleic acid hybrids with a first anti-hybrid antibody that is immunospecific to double-stranded nucleic acid hybrids to capture the double-stranded nucleic acid hybrids, wherein the first anti-hybrid antibody is directly or indirectly immobilized onto a solid support and wherein the step of detecting further comprises providing a second anti-hybrid antibody that is immunospecific to double-stranded nucleic acid hybrids, wherein the second anti-hybrid antibody is detectably labelled either directly or indirectly, whereby detection of the double-stranded nucleic acid hybrids indicates the presence of HPV 16 DNA in the sample.

When the target nucleic acid is HPV 18, the polynucleotide probes is a set of nucleic acid probes comprising at least one nucleic acid sequence chosen from the group consisting of SEQ ID NOs: 163-309. A method for determining the presence of HPV 18 DNA in a sample is provided, the method comprising:
a) contacting more than one polynucleotide probes or a complement thereof with the sample under a hybridization condition sufficient to allow the polynucleotides to anneal to a corresponding complementary nucleic acid sequence in the sample to form double-stranded nucleic acid hybrids, wherein the more than one polynucleotide probes is a set of nucleic acid probes, wherein the polynucleotides comprised in the probe set are short RNA probes having a length from 20 to 60 nucleotides and wherein the probe set comprises at least one nucleic acid sequence chosen from the group consisting of: SEQ ID NOs: 163-309; and
b) detecting double-stranded nucleic acid hybrids, wherein detecting comprises contacting the double-stranded nucleic acid hybrids with a first anti-hybrid antibody that is immunospecific to double-stranded nucleic acid hybrids to capture the double-stranded nucleic acid hybrids, wherein the first anti-hybrid antibody is directly or indirectly immobilized onto a solid support and wherein the step of detecting further comprises providing a second anti-hybrid antibody that is immunospecific to double-stranded nucleic acid hybrids, wherein the second anti-hybrid antibody is detectably labelled either directly or indirectly, whereby detection of the double-stranded nucleic acid hybrids indicates the presence of HPV 18 DNA in the sample.

When the target nucleic acid is HPV 45, the polynucleotide probes is a set of nucleic acid probes comprising at least one nucleic acid sequence chosen from the group consisting of: SEQ ID NOs: 842-974. A method for determining the presence of HPV 45 DNA in a sample is provided, the method comprising:
a) contacting more than one polynucleotide probes or a complement thereof with the sample under a hybridization condition sufficient to allow the polynucleotides to anneal to a corresponding complementary nucleic acid sequence in the sample to form double-stranded nucleic acid hybrids, wherein the more than one polynucleotide probes is a set of nucleic acid probes, wherein the polynucleotides comprised in the probe set are short RNA probes having a length from 20 to 60 nucleotides and wherein the set comprises at least one nucleic acid sequence chosen from the group consisting of: SEQ ID NOs:842-974; and
b) detecting double-stranded nucleic acid hybrids, wherein detecting comprises contacting the double-stranded nucleic acid hybrids with a first anti-hybrid antibody that is immunospecific to double-stranded nucleic acid hybrids to capture the double-stranded nucleic acid hybrids, wherein the first anti-hybrid antibody is directly or indirectly immobilized onto a solid support and wherein the step of detecting further comprises providing a second anti-hybrid antibody that is immunospecific to double-stranded nucleic acid hybrids, wherein the second anti-hybrid antibody is detectably labelled either directly or indirectly, whereby detection of the double-stranded nucleic acid hybrids indicates the presence of HPV 45 DNA in the sample.

When the target nucleic acid is HPV 31, the polynucleotide probes is a set of nucleic acid probes comprising at least one nucleic acid sequence chosen from the group consisting of: SEQ ID NOs: 310-454.

When the target nucleic acid is HPV 33, the polynucleotide probes is a set of nucleic acid probes comprising at least one nucleic acid sequence chosen from the group consisting of: SEQ ID NOs: 455-579.

When the target nucleic acid is HPV 35, the polynucleotide probes is a set of nucleic acid probes comprising at least one nucleic acid sequence chosen from the group consisting of: SEQ ID NOs: 580-722.

When the target nucleic acid is HPV 39, the polynucleotide probes is a set of nucleic acid probes comprising at least one nucleic acid sequence chosen from the group consisting of: SEQ ID NOs: 723-841.

When the target nucleic acid is HPV 51, the polynucleotide probes is a set of nucleic acid probes comprising at least one nucleic acid sequence chosen from the group consisting of: SEQ ID NOs: 975-1120.

When the target nucleic acid is HPV 52, the polynucleotide probes is a set of nucleic acid probes comprising at least one nucleic acid sequence chosen from the group consisting of: SEQ ID NOs: 1121-1252.

When the target nucleic acid is HPV 56, the polynucleotide probes is a set of nucleic acid probes comprising at least one nucleic acid sequence chosen from the group consisting of SEQ ID NOs: 1253-1367.

When the target nucleic acid is HPV 58, the polynucleotide probes is a set of nucleic acid probes comprising at least one nucleic acid sequence chosen from the group consisting of: SEQ ID NOs: 1368-1497.

When the target nucleic acid is HPV 59, the polynucleotide probes is a set of nucleic acid probes comprising at least one nucleic acid sequence chosen from the group consisting of: SEQ ID NOs: 1498-1646.

When the target nucleic acid is HPV 66, the polynucleotide probes is a set of nucleic acid probes comprising at least one nucleic acid sequence chosen from the group consisting of: SEQ ID NOs: 1647-1767.

When the target nucleic acid is HPV 68, the polynucleotide probes is a set of nucleic acid probes comprising at least one nucleic acid sequence chosen from the group consisting of: SEQ ID NOs: 1768-1875.

When the target nucleic acid is HPV 82, the polynucleotide probes is a set of nucleic acid probes comprising at least one nucleic acid sequence chosen from the group consisting of: SEQ ID NOs: 1876-2026.

In certain embodiments, the polynucleotide probes comprises the whole set of probes for that HPV type provided herein. In certain embodiments, the polynucleotide probes consists essentially of or consists of the whole set of probes for that HPV type provided herein.

The present invention further provides probe sets of SEQ ID NO: 1-162 (HPV 16); 163-309(HPV 18); 842-974(HPV 45); 310-454(HPV 31); 455-579(HPV 33); 580-722(HPV 35); 723-841(HPV 39); 975-1120(HPV 51); 1121-1252(HPV 52); 1253-1367(HPV 56); 1368-1497(HPV 58); 1498-1646(HPV 59); 1647-1767(HPV 66); 1768-1875(HPV 68); and 1876-2026(HPV 82).

The present invention further provides probe sets of SEQ ID NO: 1-161 (HPV 16); 163-299 (HPV 18); and 842-968 (HPV 45). In certain embodiments the polynucleotide probes is a mixture of probe sets comprising the probes set forth in SEQ ID NO: 1-2026.

In certain embodiments the polynucleotide probes is a mixture of probe sets comprising the probes set forth in SEQ ID NO: 1-19, 21-23, 25-53, 55-65, 67-71, 73-92, 94-116, 118-130, 132-241, 244-274, 276, 277, 279, 280, 282-849, 851-893, 895-917, 919-929, 931, 933-936, 938-2026.

In certain embodiments the hybridization is performed at about 45 to about 55 °C.

Also described are kits comprising any one of the probes disclosed herein from SEQ ID NO: 1-2026. In certain embodiments the kits comprise the probes set forth from the group consisting of SEQ ID NO: 1-162 (HPV 16); 163-309(HPV 18); 842-974(HPV 45); 310-454(HPV 31); 455-579(HPV 33); 580-722(HPV 35); 723-841(HPV 39); 975-1120(HPV 51); 1121-1252(HPV 52); 1253-1367(HPV 56); 1368-1497(HPV 58); 1498-1646(HPV 59); 1647-1767(HPV 66); 1768-1875(HPV 68); and 1876-2026(HPV 82). In another embodiment, the kit comprises the probes set forth in SEQ ID NO: 1-161 (HPV 16); 163-299 (HPV 18); and 842-968 (HPV 45). In another embodiment, the kit comprises the probes set forth in SEQ ID NO: 1-2026. In yet another embodiment, the kit comprises the 2,007 probes set forth in SEQ ID NO: 1-19, 21-23, 25-53, 55-65, 67-71, 73-92, 94-116, 118-130, 132-241, 244-274, 276, 277, 279, 280, 282-849, 851-893, 895-917, 919-929, 931, 933-936, 938-2026. Advantages and benefits of the present invention will be apparent to one skilled in the art from reading this specification.

### BRIEF DESCRIPTION OF THE FIGURES

Figure 1a shows the sequence conservation across 20 HPV genomes.
Figure 1b shows location of RNA probes along HPV18 genome.
Figure 2 shows performance of RNA probes specific for HPVs 16, 18, 31, or 45.
Figure 3 shows detection of 5,000 copies of HPV18 plasmid with synRNA coverage of 3.7Kb. synRNA = ((1.5kb coverage; 30mers) or (3.7kb coverage; 25mers)) @ 1.34 nM
Figure 4 shows that increasing the concentration of synRNA increased sensitivity of detection.
Figure 5 shows that 50mer synRNA gave higher signal than 25mer synRNA; synRNA =0.5kb of coverage; 25 or 50mers @ concentrations listed above; at about 40 min hybridization @ about 50°C.
Figure 6 shows the effect of contiguous synRNA coverage on sensitivity of detection; 40 min hybridization @ 50°C; synRNA = 1.5kb of coverage; 30 mers @ 2.24 nM.
Figure 7 shows HPV16 and HPV18 detection with synRNA is comparable; 55°C hybridization; synRNA = 3.7kb (coverage for HPV 18) or 3.175kb (coverage for HPV 16); 25 mers @ 1.34 nM.
Figure 8 shows comparison of synRNA prepared by different chemistries.
Figure 9 shows hybridization of synRNAs at different temperatures; synRNA = 3.7kb of coverage; 25mers @ 1.34 nM.
Figure 10 shows detection in the presence or absence of exogenous RNase A.
Figure 11 shows sensitivity of detection.
Figure 12 shows amplification time course.
Figure 13 shows enhancing sensitivity by increasing target amplification.
Figure 14 shows specificity.
Figure 15 represents another embodiment of a method in accordance with the present invention.
Figure 16 shows that diluting the sample collected in PreservCyt® with a suitable collection medium ("DCM" - Digene Collection Medium) enhances the signal.
Figure 17 shows that synRNA probes have the same signal and dynamic range as the full length probes.
Figure 18 shows that synRNA probes detected all specific targets (15 hrHPV target nucleic acids) with robust S/N and low variability.
Figure 19 shows that even with 10⁸ copies of low-risk HPV mixed with 10⁸ copies of positive control, the mixture of 2,007 hrHPV probes were specific enough not to provide a positive signal for the low risk HPV types and were still able to provide a strong signal for the positive control.
Figures 20A and B shows that decreasing hybridization temperature increases the detection signal where the biological sample containing the target nucleic acid has been collected in PreverveCyt®.

### DETAILED DESCRIPTION

The present inventors have discovered novel methods, compositions, and kits using synthetic probes for determining the presence of a target nucleic acid in a biological sample. The present disclosure also provides synthetic probes useful for detecting a target nucleic acid in a sample. The present disclosure includes use of novel detection methods, compositions, and kits for, among other uses, clinical diagnostic purposes, including but not limited to the detection and identification of pathogenic organisms.

In one aspect, the present disclosure provides a method for determining the presence of a target nucleic acid in a sample, wherein the target nucleic acid is a DNA, the method comprising:
a) contacting a probe set comprising more than one polynucleotide probes with the sample under a hybridization condition sufficient for the more than one polynucleotide probes to hybridize to the target DNA in the sample to form double-stranded nucleic acid hybrids, wherein the polynucleotide probes do not hybridize to a variant of the target nucleic acid and wherein the polynucleotide probes of the probe set are short RNA probes having a length from 20 to 60 nucleotides; and
b) detecting the double-stranded nucleic acid hybrids, wherein detecting comprises contacting the double-stranded nucleic acid hybrids with a first anti-hybrid antibody that is immunospecific to double-stranded nucleic acid hybrids, whereby detection of the double-stranded nucleic acid hybrids determines the target DNA in the sample.

The sample includes, without limitation, a specimen or culture (*e.g.,* microbiological and viral cultures) including biological and environmental samples. Biological samples may be from an animal, including a human, fluid, solid (*e.g.,* stool) or tissue, as well as liquid and solid food and feed products and ingredients such as dairy items, vegetables, meat and meat by-products, and waste. Environmental samples include environmental material such as surface matter, soil, water and industrial samples, as well as samples obtained from food and dairy processing instruments, apparatus, equipment, utensils, disposable and non-disposable items. Particularly preferred are biological samples including, but not limited to cervical samples (*e.g.,* a sample obtained from a cervical swab), blood, saliva, cerebral spinal fluid, pleural fluid, milk, lymph, sputum and semen. The sample may comprise a single- or double-stranded nucleic acid molecule, which includes the target nucleic acid and may be prepared for hybridization analysis by a variety of methods known in the art, *e.g.,* using proteinase K/SDS, chaotropic salts, or the like. These examples are not to be construed as limiting the sample types applicable to the present invention.

For example, a sample such as blood or an exfoliated cervical cell specimen can be collected and subjected to alkaline pH to denature the target nucleic acid and, if necessary, nick the nucleic acid that may be present in the sample. The treated, or hydrolyzed, nucleic acids can then be subjected to hybridization with a probe or group of probes diluted in a neutralizing buffer.

In certain embodiments, the sample is an exfoliated cell sample, such as an exfoliated cervical cell sample. The sample can be collected with a chemically inert collection device such as, but not limited to, a dacron tipped swab, cotton swap, cervical brush, etc. The sample and collection device can be stored in a transport medium that preserves nucleic acids and inhibits nucleases, for example in a transport medium comprising a chaotropic salt solution, a detergent solution such as sodium dodecyl sulfate (SDS), preferably 0.5% SDS, or a chelating agent solution such as ethylenediaminetetraacetic acid (EDTA), preferably 100 mM, to prevent degradation of nucleic acids prior to analysis. In certain embodiments, the sample is a cervical cell sample and in this situation, both the cell sample and the collection device are stored in the chaotropic salt solution provided as the Sample Transport Medium™ in the digene Hybrid Capture^{®} 2 High-Risk HPV DNA Test^{®} kit (Qiagen Gaithersburg, Inc., Gaithersburg, MD). Alternatively, the sample can be collected and stored in a base hydrolysis solution, for example.

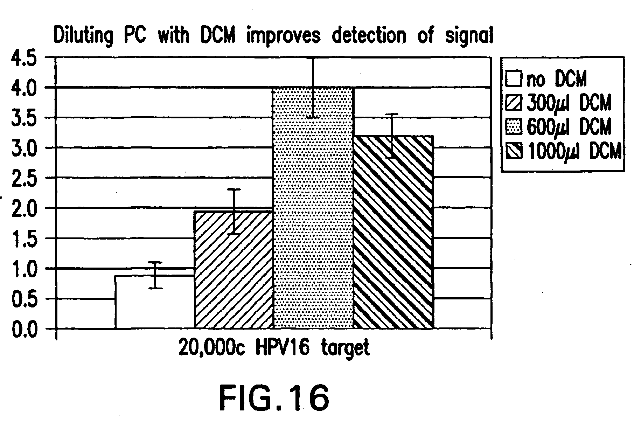

The sample may be collected and stored in a liquid based cytology collection medium such as, but not limited to, PreservCyt® and Surepath™. When such collection mediums are used (methanol based), it is preferable that the sample is diluted prior to performing methods of the present invention relating to detecting at target nucleic acid to obtain a stronger detection signal. A suitable solution is one that dilutes the methanol concentration, but still allows the rest of the reaction to proceed (i.e. allows hybridization of the probe to the target nucleic acid, allows binding of the hybrid capture antibody to the DNA:RNA, etc.). A useful solution is a collection medium comprising NP-40, sodium deoxycholate, Tris-HCl, EDTA, NaCl and sodium azide. In certain embodiments, the medium comprises or consists essentially of 1% NP-40, 0.25% sodium deoxycholate, 50mM Tris-HCl, 25 mM EDTA, 150 mM NaCl and 0.09% sodium azide. This medium is often referred to herein and in the figures as Digene Collection Medium or DCM. Figure 16 shows that diluting a methanol based collection medium, such as PreserveCyt® (or noted as "PC" in the figure) with a suitable solution such as DCM, produces a stronger signal and as such signals and hence detection of a target nucleic acid can be obtained even when the target nucleic acid has been collected in a relatively large volume of solution (i.e. ≥ 1ml). Preferably the methanol based collection medium or PreserveCyt® is diluted in the following ratios of PC to DCM:

| Amount of PreserveCyt® (PC) in ml | Amount of Digene Collection Medium (DCM) in µl |
|---|---|
| 1 | about 100 to about 1500 |
| 1 | about 200 to about 1300 |
| 1 | about 300 to about 1200 |
| 1 | about 400 to about 1100 |
| 1 | about 500 to about 1000 |
| 1 | about 600 to about 1000 |
| 1 | about 600 to about 900 |
| 1 | about 600 to about 800 |

In other embodiments 1 ml of PC is diluted with at least 200 of DCM, in other embodiments, 1 ml of PC is diluted with at least 300 µl of DCM, and in other embodiments, 1 ml of PC is diluted with at least 500 µl of DCM. In certain embodiments, 1 ml of PC is diluted with at least 500 DCM but no more than 1000 µl DCM. By diluting the PC containing the biological sample, the methods of the present invention are able to provide results and detect a target nucleic acid from a relative large sample volume (i.e. a biological sample collected in ≥ 1 ml).

If the nucleic acids to be determined are present in blood, a blood sample can be collected with a syringe, for example, and the serum separated by conventional methods. Preferably, serum is incubated for approximately 20 minutes at approximately 65° C with a protease, such as proteinase K prior to a base treatment.

In some embodiments, the sample is treated with a base, or hydrolyzed, to render the target nucleic acid accessible to hybridization. Nucleic acids can be denatured and, if necessary, nicked by incubating the sample and collection device, if present, in 0.1 to 2.0 M base at about 20 to about 100° C for 5 to 120 minutes. Preferably, treatment is achieved with 0.2 to 0.8 M NaOH, or a similar base such as KOH, at 60-70° C for 30 to 60 minutes. Most preferably, the sample and swab are incubated in 0.415 M NaOH for 65° C for 45 minutes. Approximately one volume of sample can be treated with about one-half volume of base, also referred to herein as the hydrolysis reagent. The pH will typically be about 13. This basic pH will both nick and denature a majority of the nucleic acid in the specimen. In addition, base treatment can disrupt interactions between peptides and nucleic acids to improve accessibility of the target nucleic acid and degrade protein. Base treatment effectively homogenizes the specimen to ensure reproducibility of analysis results for a given sample. Base treatment also can reduce the viscosity of the sample to increase kinetics, homogenize the sample, and reduce background by destroying any existing DNA-RNA or RNA-RNA hybrids in the sample. Base treatment also can help inactivate enzymes such as RNAases that may be present in the sample.

The variant of the target DNA includes genetic variants of the target. A variant includes polymorphisms, mutants, derivatives, modified, altered, or the like forms of the target nucleic acid. By way of example with respect to a human papillomavirus (HPV), variants include the various types. Thus, for example, wherein the target nucleic acid corresponds to HPV type 18 nucleic acid, the variant can be a corresponding nucleic acid sequence of a type of HPV other than type 18.

In one embodiment, the target DNA is an HPV nucleic acid. In another embodiment, the HPV nucleic acid is HPV DNA of an HPV type. In some embodiments, the HPV type is HPV 18, wherein the variant is nucleic acid of a type selected from the group consisting of: HPV 1, 2, 3, 4, 5, 6, 8, 11, 13, 16, 26, 30, 31, 33, 34, 35, 39, 40, 42, 43, 44, 45, 51, 52, 53, 54, 56, 58, 59, 61, 62, 66 , 67, 68, 69, 70, 71, 72, 73, 74, 81, 82, 83, 84, and 89.

In other embodiments, the HPV type is HPV 16, wherein the variant is nucleic acid of a type selected from the group consisting of: HPV 1, 2, 3, 4, 5, 6, 8, 11, 13, 18, 26, 30, 31, 33, 34, 35, 39, 40, 42, 43, 44, 45, 51, 52, 53, 54, 56, 58, 59, 61, 62, 66 , 67, 68, 69, 70, 71, 72, 73, 74, 81, 82, 83, 84, and 89.

In other embodiments, the HPV type is HPV 45, wherein the variant is nucleic acid of a type selected from the group consisting of: HPV 1, 2, 3, 4, 5, 6, 8, 11, 13, 16, 18, 26, 30, 31, 33, 34, 35, 39, 40, 42, 43, 44, 51, 52, 53, 54, 56, 58, 59, 61, 62, 66,67,68,69,70, 71, 72, 73, 74, 81, 82, 83, 84, and 89.

In other embodiments, the HPV type is HPV 31, wherein the variant is nucleic acid of a type selected from the group consisting of: HPV 1, 2, 3, 4, 5, 6, 8, 11, 13, 16, 18, 26, 30, 33, 34,35, 39, 40, 42, 43, 44, 45, 51, 52, 53, 54, 56, 58, 59, 61,62,66,67, 68, 69, 70, 71, 72, 73, 74, 81, 82, 83, 84, and 89.

In other embodiments, the HPV type is HPV 33, wherein the variant is nucleic acid of a type selected from the group consisting of: HPV 1, 2, 3, 4, 5, 6, 8, 11, 13, 16, 18, 26, 30, 31, 34, 35, 39, 40, 42, 43, 44, 45, 51, 52, 53, 54, 56, 58, 59, 61, 62, 66, 67, 68, 69, 70, 71, 72, 73, 74, 81, 82, 83, 84, and 89.

In other embodiments, the HPV type is HPV 35, wherein the variant is nucleic acid of a type selected from the group consisting of: HPV 1, 2, 3, 4, 5, 6, 8, 11, 13, 16, 18, 26, 30, 31, 33, 34, 39, 40, 42, 43, 44, 45, 51, 52, 53, 54, 56, 58,59, 61, 62, 66, 67, 68, 69, 70, 71, 72, 73, 74, 81, 82, 83, 84, and 89.

In other embodiments, the HPV type is HPV 39, wherein the variant is nucleic acid of a type selected from the group consisting of: HPV 1, 2, 3, 4, 5, 6, 8, 11, 13, 16, 18, 26, 30, 31, 33, 34, 35, 40, 42, 43, 44, 45, 51, 52, 53, 54, 56, 58, 59, 61, 62, 66 ,67, 68, 69, 70, 71, 72, 73, 74, 81, 82, 83, 84, and 89.

In other embodiments, the HPV type is HPV 51, wherein the variant is nucleic acid of a type selected from the group consisting of: HPV 1, 2, 3, 4, 5, 6, 8, 11, 13, 16, 18, 26, 30, 31, 33, 34, 35, 39, 40, 42, 43, 44, 45, 52, 53, 54, 56, 58,59, 61, 62, 66 , 67, 68, 69, 70, 71, 72, 73, 74,81,82, 83, 84, and 89.

In other embodiments, the HPV type is HPV 52, wherein the variant is nucleic acid of a type selected from the group consisting of: HPV 1, 2, 3, 4, 5, 6, 8, 11, 13, 16, 18, 26, 30, 31, 33, 34, 35, 39, 40, 42, 43, 44, 45, 51, 53, 54, 56, 58, 59, 61, 62, 66 , 67, 68, 69, 70, 71, 72, 73, 74, 81, 82, 83, 84, and 89.

In other embodiments, the HPV type is HPV 56, wherein the variant is nucleic acid of a type selected from the group consisting of: HPV 1, 2, 3, 4, 5, 6, 8, 11, 13, 16,18,26, 30, 31, 33, 34, 35, 39, 40, 42, 43,44, 45, 1,52, 3, 4, 58, 59, 61, 62, 66 , 67, 68, 69, 70, 71, 72, 73, 74, 81, 82, 83, 84, and 89.

In other embodiments, the HPV type is HPV 58, wherein the variant is nucleic acid of a type selected from the group consisting of: HPV 1, 2, 3, 4, 5, 6, 8, 11, 13, 16, 18, 26, 30, 31, 33, 34, 35, 39, 40, 42, 43, 44, 45, 51, 52, 53, 54, 56, 59, 61, 62, 66 , 67, 68,69, 70, 71, 72, 73, 74, 81, 82, 83, 84, and 89.

In other embodiments, the HPV type is HPV 59, wherein the variant is nucleic acid of a type selected from the group consisting of: HPV 1, 2, 3, 4, 5, 6, 8, 11, 13, 16, 18, 26, 30, 31, 33, 34, 35, 39, 40, 42, 43, 44, 45, 51, 52, 53, 54, 56, 58, 61, 62, 66 , 67, 68, 69, 70, 71, 72, 73, 74, 81, 82, 83, 84, and 89.

In other embodiments, the HPV type is HPV 66, wherein the variant is nucleic acid of a type selected from the group consisting of: HPV 1, 2, 3, 4, 5, 6, 8, 11, 13, 16, 18, 26, 30, 31, 33, 34, 35, 39, 40, 42, 43, 44, 45, 51, 52, 53, 54, 56, 58, 59, 61, 62, 67, 68, 69, 70, 71, 72, 73, 74, 81, 82, 83, 84, and 89.

In other embodiments, the HPV type is HPV 68, wherein the variant is nucleic acid of a type selected from the group consisting of: HPV 1, 2, 3, 4, 5, 6, 8, 11, 13, 16, 18, 26, 30, 31, 33, 34, 35, 39, 40, 42, 43, 44, 45, 51, 52, 53, 54, 56, 58, 59, 61, 62, 66, 67, 69, 70, 71, 72, 73, 74, 81, 82, 83, 84, and 89.

In other embodiments, the HPV type is HPV 82, wherein the variant is nucleic acid of a type selected from the group consisting of: HPV 1, 2, 3, 4, 5, 6, 8, 11, 13, 16, 18, 26, 30, 31, 33, 34, 35, 39, 40, 42, 43, 44, 45, 51, 52, 53, 54, 56, 58, 59, 61, 62, 66, 67, 68, 69, 70, 71, 72, 73, 74, 81, 83, 84, and 89.

In other embodiments, the HPV type is HPV 16, 18 and 45, wherein the variant is nucleic acid of a low risk HPV type.

In other embodiments, the HPV type is a high risk HPV type (hrHPV), wherein the variant is nucleic acid of a low risk HPV type.

In other embodiments, the HPV type is 16, 18, 31, 33, 35, 39, 45, 51, 52, 56, 58, 59, 66, 68, and 82 wherein the wherein the variant is nucleic acid of a low risk HPV type (such as 1, 2, 3, 4, 5, 6, 8, 11, 13, 26, 30, 34, 53, 54, 61, 62, 67, 69, 70, 71, 72, 73, 74, 81, 83, 84, and 89).

Thus, the present disclosure provides methods, compositions, and kit for determining a target nucleic acid in a sample. The sample can be collected with a chemically inert device and optionally treated with a base or other denaturing solution. The sample is incubated with more than one polynucleotide probes that are specific for the target nucleic acid but not for any other member of the population (i.e. will not bind to a variant). For example, the target nucleic acid to be determined can be an oncogenic or non-oncogenic HPV DNA sequence, HBV DNA sequence, Gonorrhea DNA, Chlamydia DNA, or other pathogen DNA or RNA. The target nucleic acid may be from cells for the detection of cancer.

In one embodiment, the target DNA is an HPV nucleic acid, wherein the target and the variant nucleic acids correspond to an HPV high risk or low risk type. HPV types characterized as low risk and high risk are known to one of ordinary skill in the art. Presently HPV types 16, 18, 31, 33, 35, 39, 45, 51, 52, 56, 58, 59, 66, 68, and 82 are considered hrHPVs and HPV types 1, 2, 3, 4, 5, 6, 8, 11, 13, 26, 30, 34, 53, 54,61, 62, 67, 69, 70, 71, 72, 73, 74, 81, 83, 84, and 89 are considered low risk HPVs.

Thus, for example, the target DNA to be determined can be nucleic acid of a microorganism such as, *e.g.,* a disease-causing pathogen, preferably a virus or bacteria, preferably HPV, however, the invention is not restricted thereto and the description following is merely illustrated by reference to determining an HPV DNA in a sample.

### Polynucleotide probes ("synprobes")

In accordance with the present invention, more than one polynucleotide probes are contacted with the sample under conditions sufficient for the polynucleotide probes to hybridize to the target nucleic acid in the sample to form double-stranded nucleic acid hybrids. The target nucleic acid is DNA and the probes are RNA. The RNA probes are short probes as opposed to full length transcribed RNA probes. These short probes are often referred to herein as synthetic RNA probes or "synRNA"

A set of polynucleotide probes is used (i.e. more than one probe). For example, if the target nucleic acid to be detected is HPV 16, a set of probes designed to specifically (i.e. only) bind to HPV 16 as opposed to binding to other HPV types is used. In certain embodiments a set of probes is used to ensure coverage of about 3-4 kb of the target nucleic acid, which ensures a strong, readable signal. In certain embodiments, detection of HPV 16 using the methods of the present invention may use a probe set comprising all of the HPV 16 probes disclosed herein (see Table 1). In other embodiments, a set of probes designed to specifically bind to another HPV type is used. For example, for HPV 18, the set of probes comprises the probes disclosed in Table 2, for HPV 45- the set of probes comprises the probes disclosed in Table 3; for HPV 31- the set of probes comprises the probes disclosed in Table 4; for HPV 33 - the set of probes comprises the probes disclosed in Table 5; for HPV 35 - the set of probes comprises the probes disclosed in Table 6; for HPV 39 - the set of probes comprises the probes disclosed in Table 7; for HPV 51 - the set of probes comprises the probes disclosed in Table 8 ; for HPV 52 - the set of probes comprises the probes disclosed in Table 9; for HPV 56 - the set of probes comprises the probes disclosed in Table 10; for HPV 58 - the set of probes comprises the probes disclosed in Table 11; for HPV 59 - the set of probes comprises the probes disclosed in Table 12; for HPV 66 - the set of probes comprises the probes disclosed in Table 13; for HPV 68 - the set of probes comprises the probes disclosed in Table 14; for HPV 15 - the set of probes comprises the probes disclosed in Table 15.

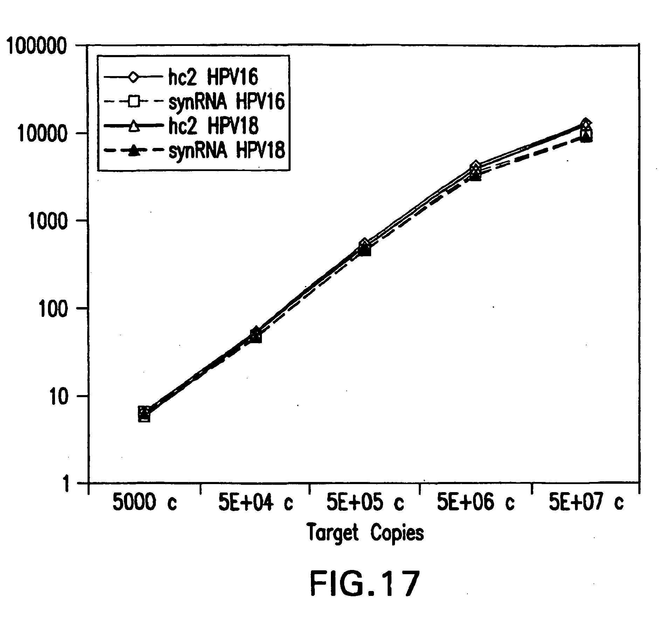

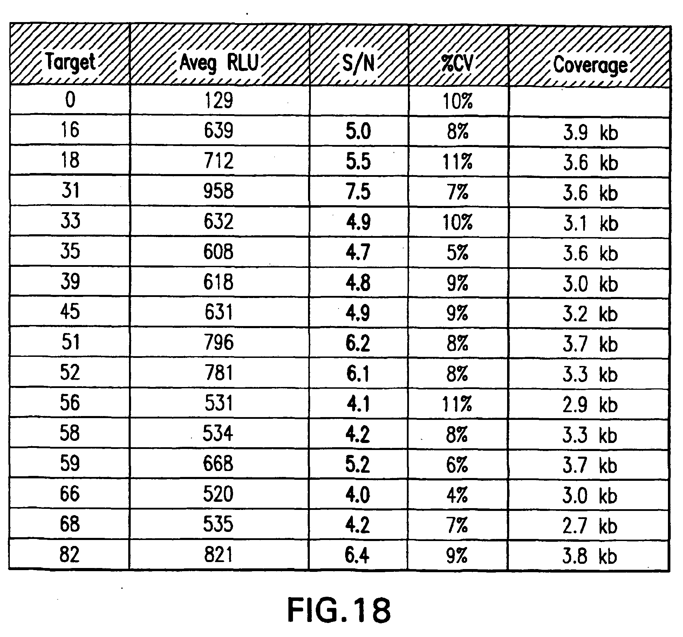

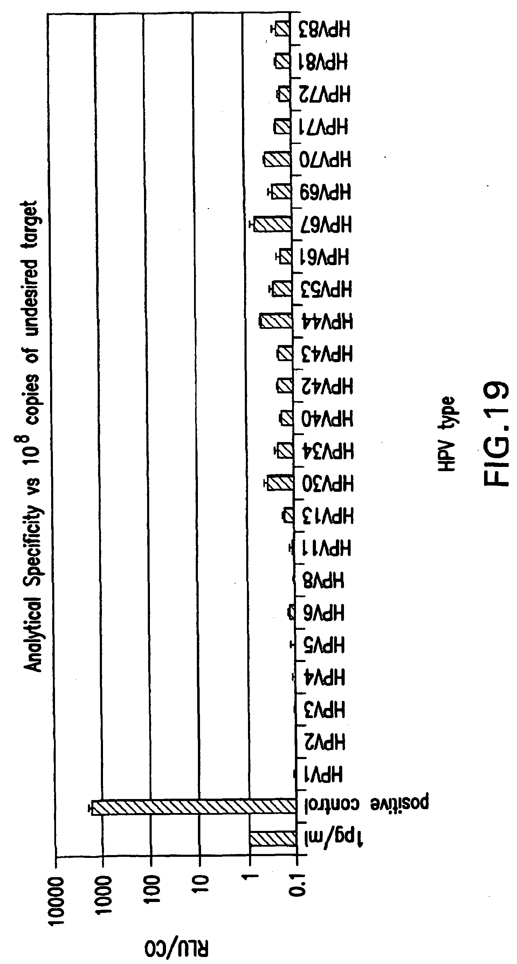

In certain embodiments a probe mixture comprising multiple sets of probes is used to simultaneously screen for any one of a mixture of desired target nucleic acids. For example, it may be desirable to screen a biological sample for the presence of any hrHPV type. In such a situation, a probe mixture of some, and in some cases, all of the probes provided in Tables 1-15 are used. For example, a probe mixture can be designed to provide a probe set for every high risk HPV (hrHPV) so one test can be run to identify whether the sample had any hrHPV target nucleic acid. For example, a probe mixture of 2,007 type-specific probes for the detection of 15 hrHPV types was used and was able to detect 5,000 copies/assay of each target genome (see Figures 17 and 18). Figure 17 shows that the synthetic probes have the same signal and dynamic range as traditional full length probes. Figure 19 provides the results of an analytical specificity test, which shows a good signal for the positive control having 10⁸ copies, whereas the low risk HPV types had a signal below the cutoff, even when they were present at 10⁸ copies. Thus, figures 17-19 show that the methods of the present utilizing the synthetic RNA probes ("synRNA") provide analytical specificity and are equivalent in limit of detection and dynamic range to full-length transcribed probes and do not suffer any loss of sensitivity with clinical samples. The probes of the present invention enable sensitive detection of a set of target genomes, while also achieving excellent specificity against even very similar related species. For example, the methods of the invention using the synprobes are able to distinguish HPV 67 from HPV 52 and 58 (HPV67 is greater than 72% identical to HPV 52 and 56). See Figure 19.

If a positive signal is obtained in the example above, it may then be desirable to further test the sample to identify the actual hrHPV type target nucleic acid present. In such a situation, the sample would be further tested with one probe specific for the HPV type or a set of probes for the specific HPV type. For example, if one were testing the sample to determine whether the sample contained an HPV 16 target nucleic acid, then at least one probe from Table 1 (HPV 16 probes) would be used, or alternatively the entire set of probes from Table 1 would be used to increase the signal strength. Alternatively, it may be desirable to test for certain hrHPV types such as HPV 16, 18 and 45 and not necessarily test for each individual hrHPV types. In this situation, the mixture of probes would employ at least one probe from the HPV 16, 18 and 45 probe sets (or alternatively, all of the probes from the 16, 18 and 45 HPV probe sets are used).

The polynucleotide probes are designed so that they do not hybridize to a variant of the target nucleic acid under the hybridization conditions utilized. The number of different polynucleotide probes employed per set can depend on the desired sensitivity. Higher coverage of the nucleic acid target with the corresponding polynucleotide probes can provide a stronger signal (as there will be more DNA-RNA hybrids for the antibodies to bind).

In one embodiment, the method further comprises determining the polynucleotide probes, wherein determining comprises identifying a contiguous nucleotide sequence of the target nucleic acid, wherein the contiguous nucleotide sequence is not present in the variant. By way of example, relatively short regions (*e.g.,* about 25mers) of the HPV genome with sufficient sequence specificity can be determined to provide the polynucleotide probes for HPV type-specific hybridization.

Thus, depending on the target DNA of interest, and the corresponding variant(s), the polynucleotide probes can be prepared to have lengths sufficient to provide target-specific hybridization. The polynucleotide probes each have a length of about 20 to about 60, about 20 to about 40, about 20 to about 20 and about 25 to about 30 nucleotides. In one embodiment, the polynucleotide probes each have a length of about 25 to about 50 nucleotides. In certain embodiments, the probes have a length of 25 nucleotides. In certain embodiments, all of the probes in a set will have the same length, such as 25 nucleotides, and will have very similar melting temperatures to allow hybridization of all of the probes in the set under the same hybridization conditions.

Bioinformatics tools can be employed to determine the polynucleotide probes. For example, Oligoarray 2.0, a software program that designs specific oligonucleotides can be utilized. Oligoarray 2.0 is described by Rouillard et al., Nucleic Acids Research, 31: 3057-3062 (2003). Oligoarray 2.0 is a program which combines the functionality of BLAST (Basic Local Alignment Search Tool) and Mfold (Genetics Computer Group, Madison, WI). BLAST, which implements the statistical matching theory by Karlin and Altschul (Proc. Natl. Acad. Sci. USA 87:2264 (1990); Proc. Natl. Acad. Sci. USA 90:5873 (1993), is a widely used program for rapidly detecting nucleotide sequences that match a given query sequence One of ordinary skill in the art can provide a database of sequences, which are to be checked against, for example HPV high risk and low risk types 1, 2, 3, 4, 5, 6, 8, 11, 13, 16, 26, 30, 31, 33, 34, 35, 39, 40, 42, 43, 44, 45, 51, 52, 53, 54, 56, 58, 59,61,62,66, 67, 68, 69, 70, 71, 72, 73, 74, 81, 82, 83, 84, and 89. The target sequence of interest, *e.g.* HPV 18, can then be BLASTed against that database to search for any regions of identity. Melting temperature (Tm) and %GC can then be computed for polynucleotide probes of a specified length and compared to the parameters, after which secondary structure also can be examined. Once all parameters of interest are satisfied, cross hybridization can be checked with the Mfold package, using the similarity determined by BLAST. The various programs can be adapted to determine the polynucleotide probes meeting the desired specificity requirements. For example, the parameters of the program can be set to prepare polynucleotides of 25nt length, Tm range of 55-95°C, a GC range of 35-65%, and no secondary structure or cross-hybridization at 55°C or below.

Accordingly in other aspects, the present invention utilizes bioinformatics to provide sequence information sufficient to design and/or prepare polynucleotide probes for determining the target in the sample.

In addition to using the synprobes in a method of the present invention, one aspect of the invention comprises the probes disclosed herein.

Also described is an isolated polynucleotide for specific hybridization to HPV 16 consisting essentially of a sequence or a complement thereof selected from the group consisting of SEQ ID NOs: 1-162 (See Table 1). Also described is a set of polynucleotides for specific hybridization to HPV 16, wherein the set comprises at least one polynucleotide consisting essentially of a sequence or a complement thereof selected from the group consisting of: SEQ ID NOs: 1-162. Also described is a set of polynucleotides for specific hybridization to HPV 16, wherein the set comprises at least one polynucleotide consisting essentially of a sequence or a complement thereof selected from the group consisting of: SEQ ID NOs: 1-161. In certain embodiments, the methods of the present invention utilize a set of polynucleotide probes for specific hybridization to HPV 16 comprising SEQ ID NOs: 1-162. In certain embodiments, the methods of the present invention utilize a set of polynucleotide probes for specific hybridization to HPV 16 comprising SEQ ID NO: 1-19, 21-23, 25-53, 55-65, 67-71, 73-92, 94-116, 118-130, 132-162.

**Table 1: Polyribonucleotide probes for determining HPV 16 nucleic acid.**

| SEQ ID NO: | Name | Sequence |
|---|---|---|
| 1 | HPV16_25_HR&LR_7866 | GGGUUACACAUUUACAAGCAACUUA |
| 2 | HPV16_25_HR&LR_7841 | ACAUGGGUGUGUGCAAACCGAUUUU |
| 3 | HPV16_25_HR&LR_7799 | CUGUGUAAAGGUUAGUCAUACAUUG |
| 4 | HPV_16_25_HR&LR_7774 | AAUGUCACCCUAGUUCAUACAUGAA |
| 5 | HPV16_25_HR&LR_7749 | AGGUUUAAACUUCUAAGGCCAACUA |
| 6 | HPV16_25_HR&LR_7712 | GGCUUGUUUUAACUAACCUAAUUGC |
| 7 | HPV16_25_HR&LR_7676 | CAACGCCUUACAUACCGCUGUUAGG |
| 8 | HPV16_25_HRBLR_7629 | CUGAAUCACUAUGUACAUUGUGUCA |
| 9 | HPV16_25_HRBLR_7577 | GCACUGCUUGCCAACCAUUCCAUUG |
| 10 | HPV16_25_HR&LR_7552 | UGCCAAAUCCCUGUUUUCCUGACCU |
| 11 | HPV16_25_HRBLR_7527 | UUGUACGUUUCCUGCUUGCCAUGCG |
| 12 | HPV16_25_HR&LR_7502 | CUAUGUCAGCAACUAUGGUUUAAAC |
| 13 | HPV16_25_HR&LR_7433 | CCCAUUUUGUAGCUUCAACCGAAUU |
| 14 | HPV16_25_HR&LR_7408 | AUAUACUAUAUUUUGUAGCGCCAGG |
| 15 | HPV16_25_HR&LR_7371 | UAUAAACUAUAUUUGCUACAUCCUG |
| 16 | HPV16_25_HR&LR_7340 | CCUACUAAUUGUGUUGUGGUUAUUC |
| 17 | HPV16_25_HR&LR_7293 | GUGUAACUAUUGUGUCAUGCAACAU |
| 18 | HPV16_25_HR&LR_7250 | UGUAUGGUAUAAUAAACACGUGUGU |
| 19 | HPV16_25_HR&LR_7225 | AUAUUAAGUUGUAUGUGUGUUUGUA |
| 20 | HPV16_25_HR&LR_7201 | GUAUGUGCUUGUAUGUGCUUGUAAA |
| 21 | HPV16_25_HR&LR_7175 | UAGUGUUGUUUGUUGUGUAUAUGUU |
| 22 | HPV16_25_HR&LR_7150 | UGUAAGUAUUGUAUGUAUGUUGAAU |
| 23 | HPV16_25_HR&LR_7112 | AUCUACCUCUACAACUGCUAAACGC |
| 24 | HPV16_25_HR&LR_7087 | AACGAAAAGCUACACCCACCACCUC |
| 25 | HPV16_25_HR&LR_7061 | GGCCAAACCAAAAUUUACAUUAGGA |
| 26 | HPV16_25_HR&LR_6935 | AGCACCUAAAGAAGAUGAUCCCCUU |
| 27 | HPV16_25_HR&LR_6894 | UUUGUAACCCAGGCAAUUGCUUGUC |
| 28 | HPV16_25_HR&LR_6869 | AGGCACACUAGAAGAUACUUAUAGG |
| 29 | HPV16_25_HR&LR_6790 | CAGACGUUAUGACAUACAUACAUUC |
| 30 | HPV16_25_HR&LR_6675 | GCCAUAUCUACUUCAGAAACUACAU |
| 31 | HPV16_25_HR&LR_6541 | CUGAUGCCCAAAUAUUCAAUAAACC |
| 32 | HPV16_25_HR&LR_6496 | CCAGUUCAAAUUAUUUUCCUACACC |
| 33 | HPV16_25_HR&LR_6471 | GGCUCUGGGUCUACUGCAAAUUUAG |
| 34 | HPV16_25_HR&LR_6438 | GGUGAAAAUGUACCAGACGAUUUAU |
| 35 | HPV16_25_HR&LR_6350 | GUCAGAACCAUAUGGCGACAGCUUA |
| 36 | HPV16_25_HR&LR_6294 | GUUCCACUGGAUAUUUGUACAUCUA |
| 37 | HPV16_25_HR&LR_6192 | CCACCAUUAGAGUUAAUAAACACAG |
| 38 | HPV16_25_HR&LR_6165 | AAUGUUGCAGUAAAUCCAGGUGAUU |
| 39 | HPV16_25_HR&LR_6052 | CAGGUGUGGAUAAUAGAGAAUGUAU |
| 40 | HPV16_25_HR&LR_6022 | CAGAAAAUGCUAGUGCUUAUGCAGC |
| 41 | HPV16_25_HR&LR_5851 | UAUUUAGAAUACAUUUACCUGACCC |
| 42 | HPV16_25_HR&LR_5825 | UAAAGUAUCAGGAUUACAAUACAGG |
| 43 | HPV16_25_HR&LR_5800 | CUAACAAUAACAAAAUAUUAGUUCC |
| 44 | HPV16_25_HR&LR_5745 | GCAGGAACAUCCAGACUACUUGCAG |
| 45 | HPV16_25_HR&LR_5586 | GUUAUUACAUGUUACGAAAACGACG |
| 46 | HPV16_25_HR&LR_5546 | ACAAUUAUUGCUGAUGCAGGUGACU |
| 47 | HPV16_25_HR&LR_5521 | UAUAGUUCCAGGGUCUCCACAAUAU |
| 48 | HPV16_25_HR&LR_5496 | CUGACCAAGCUCCUUCAUUAAUUCC |
| 49 | HPV16_25_HR&LR_5469 | CAGGUCCUGAUAUACCCAUUAAUAU |
| 50 | HPV16_25_HR&LR_5442 | GUGGUGCAUACAAUAUUCCUUUAGU |
| 51 | HPV16_25_HR&LR_5406 | CAGGUUAUAUUCCUGCAAAUACAAC |
| 52 | HPV16_25_HR&LR_5381 | CCAUCUGUACCCUCUACAUCUUUAU |
| 53 | HPV16_25_HR&LR_5356 | UACAGAUACUUCUACAACCCCGGUA |
| 54 | HPV16_25_HR&LR_5336 | AUUUAUGCAGAUGACUUUAUUACAG |
| 55 | HPV16_25_HR&LR_5301 | CCUCACCUACUUCUAUUAAUAAUGG |
| 56 | HPV16_25_HR&LR_5276 | ACAUAUACUACCACUUCACAUGCAG |
| 57 | HPV16_25_HR&LR_5228 | ACUAUUGAUCCUGCAGAAGAAAUAG |
| 58 | HPV16_25_HR&LR_5182 | UGGAAAAUCUAUAGGUGCUAAGGUA |
| 59 | HPV16_25_HR&LR_5153 | GGUAAUAAACAAACACUACGUACUC |
| 60 | HPV16_25_HR&LR_5122 | UAGGCGUACUGGCAUUAGGUACAGU |
| 61 | HPV16_25_HR&LR_5051 | AAUAGUAUUAAUAUAGCUCCAGAUC |
| 62 | HPV16_25_HR&LR_5000 | GCAUAUGAAGGUAUAGAUGUGGAUA |
| 63 | HPV16_25_HR&LR_4965 | CCACUCCCACUAAACUUAUUACAUA |
| 64 | HPV16_25_HR&LR_4910 | GGAUUAUAUAGUCGCACAACACAAC |
| 65 | HPV16_25_HR&LR_4854 | CUAACACAGUAACUAGUAGCACACC |
| 66 | HPV16_25_HR&LR_4829 | GAUACAUUUAUUGUUAGCACAAACC |
| 67 | HPV16_25_HR&LR_4771 | GCAUUUUACACUUUCAUCAUCCACU |
| 68 | HPV16_25_HR&LR_4706 | CAUAAUAAUCCCACUUUCACUGACC |
| 69 | HPV16_25_HR&LR_4681 | UAAUACUGUUACUACUGUUACUACA |
| 70 | HPV16_25_HR&LR_4640 | ACUACUUCAACUGAUACCACACCUG |
| 71 | HPV16_25_HR&LR_4588 | UGCACCAACAUCUGUACCUUCCAUU |
| 72 | HPV16_25_HR&LR_4562 | GAAGAAACUAGUUUUAUUGAUGCUG |
| 73 | HPV16_25 HR&LR 4480 | UACAGAUACACUUGCUCCUGUAAGA |
| 74 | HPV16_25_HR&LR_4435 | CGGACGCACUGGGUAUAUUCCAUUG |
| 75 | HPV16_25_HR&LR_4369 | AUUACAAUAUGGAAGUAUGGGUGUA |
| 76 | HPV16_25_HR&LR_4275 | CGGCUACCCAACUUUAUAAAACAUG |
| 77 | HPV16_25 HR&LR 4232 | ACAAUGCGACACAAACGUUCUGCAA |
| 78 | HPV16_25 HR&LR_4131 | AAUUGUUGUAUACCAUAACUUACUA |
| 79 | HPV16_25_HR&LR_4103 | AUAUGUACAUAAUGUAAUUGUUACA |
| 80 | HPV16_25_HR&LR_4009 | CUCUGCGUUUAGGUGUUUUAUUGUA |
| 81 | HPV16_25_HR&LR_3984 | UAUUACUAUUGUGGAUAACAGCAGC |
| 82 | HPV16_25_HR&LR_3942 | UGCUUUUGUCUGUGUCUACAUACAC |
| 83 | HPV16 25_HR&LR_3866 | UGCAUCCACAACAUUACUGGCGUGC |
| 84 | HPV16_25_HR&LR_3824 | CAGUGUCUACUGGAUUUAUGUCUAU |
| 85 | HPV16 25_HR&LR_3765 | UGAUAGUGAAUGGCAACGUGACCAA |
| 86 | HPV16_25_HR&LR_3712 | CAUUGGACAGGACAUAAUGUAAAAC |
| 87 | HPV16_25_HR&LR_3686 | UGUAUACUGCAGUGUCGUCUACAUG |
| 88 | HPV16_25_HR&LR_3638 | CUAAUACUUUAAAAUGUUUAAGAUA |
| 89 | HPV16_25_HR&LR_3602 | GUAACACUACACCCAUAGUACAUUU |
| 90 | HPV16_25_HR&LR_3577 | CACAAAGGACGGAUUAACUGUAAUA |
| 91 | HPV16_25_HR&LR_3552 | AAUCCUCACUGCAUUUAACAGCUCA |
| 92 | HPV16_25_HR&LR_3520 | UUGUUGCACAGAGACUCAGUGGACA |
| 93 | HPV16_25_HR&LR_3495 | CGGAAACCCCUGCCACACCACUAAG |
| 94 | HPV16_25_HR&LR_3460 | ACGACUAUCCAGCGACCAAGAUCAG |
| 95 | HPV16 25_HR&LR_3417 | GACCCAUACCAAAGCCGUCGCCUUG |
| 96 | HPV16_25_HR&LR_3378 | UGAAAUUAUUAGGCAGCACUUGGCC |
| 97 | HPV16_25_HR&LR_3323 | GUCAGGUAAUAUUAUGUCCUACAUC |
| 98 | HPV16_25_HR&LR_3241 | GGAAUACGAACAUAUUUUGUGCAGU |
| 99 | HPV16_25_HR&LR_3201 | GGGUCAAGUUGACUAUUAUGGUUUA |
| 100 | HPV16_25_HR&LR_3176 | AAGAAGCAUCAGUAACUGUGGUAGA |
| 101 | HPV16_25_HR&LR_3145 | UAUACAAACUGGACACAUAUAUAUA |
| 102 | HPV16_25_HR&LR_3103 | GUGGAAGUGCAGUUUGAUGGAGACA |
| 103 | HPV16_25_HR&LR_3043 | GUUAGCCUUGAAGUGUAUUUAACUG |
| 104 | HPV16_25_HR&LR_3018 | UAAUGAAAAGUGGACAUUACAAGAC |
| 105 | HPV16_25_HR&LR_2974 | GAACUGCAACUAACGUUAGAAACAA |
| 106 | HPV16_25_HR&LR_2938 | CUGGCUGUAUCAAAGAAUAAAGCAU |
| 107 | HPV16_25_HR&LR_2890 | GCCAGAGAAAUGGGAUUUAAACAUA |
| 108 | HPV16_25_HR&LR_2863 | CGCCUAGAAUGUGCUAUUUAUUACA |
| 109 | HPV16_25_HR&LR_2828 | ACCUACGUGACCAUAUAGACUAUUG |
| 110 | HPV16_25_HR&LR_2794 | AAAAUACUAACACAUUAUGAAAAUG |
| 111 | HPV16_25_HR&LR_2630 | UAAUGAGUUUCCAUUUGACGAAAAC |
| 112 | HPV16_25_HR&LR_2602 | AUAAUAGAUUGGUGGUGUUUACAUU |
| 113 | HPV16_25_HR&LR_2555 | UACAUCUAACAUUAAUGCUGGUACA |
| 114 | HPV16_25_HR&LR_2501 | UAUGGAUGUAAAGCAUAGACCAUUG |
| 115 | HPV16_25_HR&LR_2444 | CUGUUGGAACUACAUAGAUGACAAU |
| 116 | HPV16_25_HR&LR_2345 | GCAAGGGUCUGUAAUAUGUUUUGUA |
| 117 | HPV16_25_HR&LR_2324 | UAUGAGUUUAAUGAAAUUUCUGCAA |
| 118 | HPV16_25_HR&LR_2282 | AUUACUAUAUGGUGCAGCUAACACA |
| 119 | HPV16_25_HR&LR_2171 | AGGUGAUUGGAAGCAAAUUGUUAUG |
| 120 | HPV16_25_HR&LR_2139 | AUAAAAUAUAGAUGUGAUAGGGUAG |
| 121 | HPV16_25_HR&LR_1957 | ACGAUAAUGACAUAGUAGACGAUAG |
| 122 | HPV16_25_HR&LR_1914 | AAUGAUUGUACAUUUGAAUUAUCAC |
| 123 | HPV16_25_HR&LR_1827 | UAUAAAACAGGUAUAUCAAAUAUUA |
| 124 | HPV16_25_HR&LR_1775 | UAUGAUGAUAGAGCCUCCAAAAUUG |
| 125 | HPV16_25_HR&LR_1750 | AACUAUUAUGUGUGUCUCCAAUGUG |
| 126 | HPV16_5_HR&LR_1676 | GGGAAUGGUUGUGUUACUAUUAGUA |
| 127 | HPV16_25_HR&LR_584 | UUUGGACUUACACCCAGUAUAGCUG |
| 128 | HPV16_25 HR&LR 1559 | GUGUUGCGAUUGGUGUAUUGCUGCA |
| 129 | HPV16_25_HR&LR_1534 | GACCAUUUAAAAGUAAUAAAUCAAC |
| 130 | HPV16_25_HR&LR_1492 | AAUUUAAAGAGUUAUACGGGGUGAG |
| 131 | HPV16_25_HR&LR_1417 | CUAUAUGCCAAACACCACUUACAAA |
| 132 | HPV16_25_HR&LR_1364 | UUGCAGUCAGUACAGUAGUGGAAGU |
| 133 | HPV16_25_HR&LR_1331 | AUGUAGUCAGUAUAGUGGUGGAAGU |
| 134 | HPV16_25_HR&LR_1306 | AAGGGCGCCAUGAGACUGAAACACC |
| 135 | HPV16_25_HR&LR_1238 | AUUAUUUGAAAGCGAAGACAGCGGG |
| 136 | HPV16_25_HR&LR_1185 | CCUAGAUUAAAAGCUAUAUGUAUAG |
| 137 | HPV16_25_HR&LR_1150 | GUGAUAUUAGUGGAUGUGUAGACAA |
| 138 | HPV16_25_HR&LR_1101 | UAGAGAUGCAGUACAGGUUCUAAAA |
| 139 | HPV16_25_HR&LR_1076 | UUACUGCACAGGAAGCAAAACAACA |
| 140 | HPV16_25_HR&LR_1029 | UAAUGAUUAUUUAACACAGGCAGAA |
| 141 | HPV16_25_HR&LR_1004 | AUUUGGUAGAUUUUAUAGUAAAUGA |
| 142 | HPV16_25_HR&LR_984 | UGACAGUGAUACAGGUGAAGAUUUG |
| 143 | HPV16_25_HR&LR_848 | AGAAACCAUAAUCUACCAUGGCUGA |
| 144 | HPV16_25_HR&LR_790 | CGUACUUUGGAAGACCUGUUAAUGG |
| 145 | HPV16_25_HR&LR_732 | UUGUUGCAAGUGUGACUCUACGCUU |
| 146 | HPV16_25_HR&LR_702 | GGACAGAGCCCAUUACAAUAUUGUA |
| 147 | HPV16_25_HR&LR_569 | GAGAUACACCUACAUUGCAUGAAUA |
| 148 | HPV16_25_HRBLR_524 | AGAUCAUCAAGAACACGUAGAGAAA |
| 149 | HPV16_25_HR&LR_477 | UCCAUAAUAUAAGGGGUCGGUGGAC |
| 150 | HPV16_25_HR&LR_412 | UAUUAACUGUCAAAAGCCACUGUGU |
| 151 | HPV16_25_HR&LR_366 | UAGAACAGCAAUACAACAAACCGUU |
| 152 | HPV16_25_HR&LR_334 | ACAUUAUUGUUAUAGUUUGUAUGGA |
| 153 | HPV16_25_HR&LR_306 | AGUUUUAUUCUAAAAUUAGUGAGUA |
| 154 | HPV16_25_HR&LR_281 | UAUGCUGUAUGUGAUAAAUGUUUAA |
| 155 | HPV16_25_HR&LR_245 | CGGGAUUUAUGCAUAGUAUAUAGAG |
| 156 | HPV16_25_HR&LR 209 | CAGUUACUGCGACGUGAGGUAUAUG |
| 157 | HPV16_25_HR&LR_155 | GAGCUGCAAACAACUAUACAUGAUA |
| 158 | HPV16_25_HR&LR_130 | CAGAAAGUUACCACAGUUAUGCACA |
| 159 | HPV16_25_HR&LR_92 | AAGAGAACUGCAAUGUUUCAGGACC |
| 160 | HPV16_25_HR&LR_57 | CCGGUUAGUAUAAAAGCAGACAUUU |
| 161 | HPV16_25_HR&LR_18 | AUAAAACUAAGGGCGUAACCGAAAU |
| 162 | HPV16_7200 | UGUAUGUGCUUGUAUGUGCUUGUAA |

Also described is an isolated polynucleotide for specific hybridization to HPV 18 consisting essentially of a sequence or a complement thereof selected from the group consisting of: SEQ ID NOs: 163-309 (See Table 2). Also described is a set of polynucleotides for specific hybridization to HPV 18, wherein the set comprises at least one polynucleotide consisting essentially of a sequence or a complement thereof selected from the group consisting of: SEQ ID NOs: 163-309. Also described is a set of polynucleotides for specific hybridization to HPV 18, wherein the set comprises at least one polynucleotide consisting essentially of a sequence or a complement thereof selected from the group consisting of: SEQ ID NOs: 163-299. In certain embodiments, the methods of the present invention utilize a set of polynucleotide probes for specific hybridization to HPV 18 comprising SEQ ID NOs: 163-309. In certain embodiments, the methods of the present invention utilize a set of polynucleotide probes for specific hybridization to HPV 18 comprising SEQ ID NO: 163-241, 244-274, 276, 277, 279, 280, 282-309.

**Table 2: Polyribonucleotide probes for determining HPV 18 nucleic acid.**

| SEQ ID NO: | Name | Sequence |
|---|---|---|
| 163 | HPV18_25_HR&LR(-45)_7833 | UUGGGCAGCACAUACUAUACUUUUC |
| 164 | HPV18_25_HR&LR(-45)_7796 | UAAGCUGUGCAUACAUAGUUUAUGC |
| 165 | HPV18_25_HR&LR(-45)_7764 | CUGUCUACCCUUAACAUGAACUAUA |
| 166 | HPV18_25_HR&LR(-45)_7738 | GUACAACUACUUUCAUGUCCAACAU |
| 167 | HPV18_25_HR&LR(-45)_7658 | AUCCACUCCCUAAGUAAUAAAACUG |
| 168 | HPV18_25_HR&LR(-45)_7632 | GCUACAACAAUUGCUUGCAUAACUA |
| 169 | HPV18_25_HR&LR(-45)_7561 | UUGAACAAUUGGCGCGCCUCUUUGG |
| 170 | HPV18_25_HR&LR(-45)_7536 | CUUUUGGGCACUGCUCCUACAUAUU |
| 171 | HPV18_25_HR&LR(-45)_7501 | CAAUACAGUACGCUGGCACUAUUGC |
| 172 | HPV18_25_HR&LR(-45)_7476 | UGGCUUAUGUCUGUGGUUUUCUGCA |
| 173 | HPV18_25_HR&LR(-45)_7423 | CCAUUUUAUCCUACAAUCCUCCAUU |
| 174 | HPV18_25_HR&LR(-45)_7398 | UAUAAAACUGCACACCUUACAGCAU |
| 175 | HPV18_25_HR&LR(-45)_7370 | GGGCUAUAUAUUGUCCUGUAUUUCA |
| 176 | HPV18_25_HR&LR(-45)_7345 | GUUUGUGGUAUGGGUGUUGCUUGUU |
| 177 | HPV18_25_HR&LR(-45)_7320 | CCUAGUGAGUAACAACUGUAUUUGU |
| 178 | HPV18_25_HR&LR(-45)_7291 | UUGUGGUUCUGUGUGUUAUGUGGUU |
| 179 | HPV18_25_HR&LR(-45)_7249 | GUUACUAUAUUUGUUGGUAUGUGGC |
| 180 | HPV18_25_HR&LR(-45)_7211 | CAUUGUAUGGUAUGUAUGGUUGUUG |
| 181 | HPV18_25_HR&LR(-45)_7184 | CCUGUGUUUGUGUUUGUUGUAUGAU |
| 182 | HPV18_25_HR&LR(-45)_7123 | GUGCCAGGAAGUAAUAUGUGUGUGU |
| 183 | HPV18_25_HR&LR(-45)_7098 | AAACCUGCCAAGCGUGUGCGUGUAC |
| 184 | HPV18_25_HR&LR(-45)_7073 | UGCUCCAUCUGCCACUACGUCUUCU |
| 185 | HPV18_25_HR&LR(-45)_6982 | CUUUAGACUUAGAUCAAUAUCCCCU |
| 186 | HPV18_25_HR&LR(-45)_6911 | UGCACCGGCUGAAAAUAAGGAUCCC |
| 187 | HPV18_25_HR&LR(-45)_6876 | GUACAAUCUGUUGCUAUUACCUGUC |
| 188 | HPV18_25_HR&LR(-45)_6698 | GCAGUAUAGCAGACAUGUUGAGGAA |
| 189 | HPV18_25_HR&LR(-45)_6672 | GGGCAAUAUGAUGCUACCAAAUUUA |
| 190 | HPV18_25_HR&LR(-45)_6625 | CCAGUACCAAUUUAACAAUAUGUGC |
| 191 | HPV18_25_HR&LR(-45)_6482 | GUAUUCUCCCUCUCCAAGUGGCUCU |
| 192 | HPV18_25_HR&LR(-45)_6425 | GCCUCAAUCCUUAUAUAUUAAAGGC |
| 193 | HPV18_25_HR&LR(-45)_6254 | AGAUACUAAAUGUGAGGUACCAUUG |
| 194 | HPV18_25_HR&LR(-45)_6188 | CACAGUUUUGGAAGAUGGUGAUAUG |
| 195 | HPV18_25_HR&LR(-45)_6137 | UAAAUCGCGUCCUUUAUCACAGGGC |
| 196 | HPV18_25_HR&LR(-45)_6029 | UUCUGAGGACGUUAGGGACAAUGUG |
| 197 | HPV18_25_HR&LR(-45)_6004 | GUUCCCAUGCCGCCACGUCUAAUGU |
| 198 | HPV18_25_HR&LR(-45)_5766 | GUUCCUGCAGGUGGUGGCAAUAAGC |
| 199 | HPV18_25_HR&LR(-45)_5667 | GCAAGAGUUGUAAAUACCGAUGAUU |
| 200 | HPV18_25_HR&LR(-45)_5642 | CGUAUAUCUUCCACCUCCUUCUGUG |
| 201 | HPV18_25_HR&LR(-45)_5519 | CAGUAUAUUGGUAUACAUGGUACAC |
| 202 | HPV18_25_HR&LR(-45)_5487 | CCAUUGUAUCACCCACGGCCCCUGC |
| 203 | HPV18_25_HR&LR(-45)_5462 | UUACCAUCUACUACCUCUGUAUGGC |
| 204 | HPV18_25_HR&LR(-45)_5437 | UGUAUACACGGGUCCUGAUAUUACA |
| 205 | HPV18_25_HR&LR(-45)_5409 | UCCCUUUAACCUCCUCUUGGGAUGU |
| 206 | HPV18_25_HR&LR(-45)_5384 | GCCUCUUCCUAUAGUAAUGUAACGG |
| 207 | HPV18_25_HR&LR(-45)_5329 | AUCGCGUUCUACUACCUCCUUUGCA |
| 208 | HPV18_25_HR&LR(-45)_5304 | ACAUGGACCCUGCAGUGCCUGUACC |
| 209 | HPV18_25_HR&LR(-45)_5249 | CAGCCUUUAGUAUCUGCCACGGAGG |
| 210 | HPV18_25_HR&LR(-45)_5224 | ACCUUCCCCAGAAUAUAUUGAACUG |
| 211 | HPV18_25_HR&LR(-45)_5160 | UUACCCGCAGCGGUACACAAAUAGG |
| 212 | HPV18_25_HR&LR(-45)_5118 | GGACUGUUCGCUUUAGUAGAUUAGG |
| 213 | HPV18_25_HR&LR(-45)_5021 | GACACUACAUUAACAUUUGAUCCUC |
| 214 | HPV18_25_HR&LR(-45)_4971 | CACGUCCAUCCUCUUUAAUUACAUA |
| 215 | HPV18_25_HR&LR(-45)_4946 | UCAGUGGCUAACCCUGAGUUUCUUA |
| 216 | HPV18_25_HR&LR(-45)_4833 | UACAAACAUUUGCUUCUUCUGGUAC |
| 217 | HPV18_25_HR&LR(-45)_4737 | CGUCCAUUAUUGAAGUUCCACAAAC |
| 218 | HPV18_25_HR&LR(-45)_4701 | CCACAACCAAUUUUACCAAUCCUGC |
| 219 | HPV18_25_HR&LR(-45)_4676 | CCUUCGUCUACCUCUGUGUCUAUUU |
| 220 | HPV18_25_HR&LR(-45)_4634 | ACAUCUGCGGGUACAACUACACCUG |
| 221 | HPV18_25_HR&LR(-45)_4591 | UGCACCUAGGCCUACGUUUACUGGC |
| 222 | HPV18_25_HR&LR(-45)_4566 | AGGACUCCAGUGUGGUUACAUCAGG |
| 223 | HPV18_25_HR&LR(-45)_4483 | AGUGGUGGAUGUUGGUCCUACACGU |
| 224 | HPV18_25_HR&LR(-45)_4455 | ACAUUCCAUUGGGUGGGCGUUCCAA |
| 225 | HPV18_25_HR&LR(-45)_4375 | AUUGCAAUGGUCAAGCCUUGGUAUA |
| 226 | HPV18_25_HR&LR(-45)_4276 | GGCUUCGGUAACUGACUUAUAUAAA |
| 227 | HPV18_25_HR&LR(-45)_4234 | UAAUAAAAGUAUGGUAUCCCACCGU |
| 228 | HPV18_25_HR&LR(-45)_4113 | CCCAUGUUACUAUUGCAUAUACAUG |
| 229 | HPV18_25_HR&LR(-45)_4072 | CUGCCACAGCAUUCACAGUAUAUGU |
| 230 | HPV18_25_HR&LR(-45)_4047 | GUGUAUAUUGUGGUAAUAACGUCCC |
| 231 | HPV18_25_HR&LR(-45)_3971 | AUGCAUGUAUGUGUGCUGCCAUGUC |
| 232 | HPV18_25_HR&LR(-45)_3922 | GCUGUAGUACCAAUAUGUUAUCACU |
| 233 | HPV18_25_HR&LR(-45)_3888 | AUAUUGGUGGGAUACAUGACAAUGU |
| 234 | HPV18_25_HR&LR(-45)_3863 | UGUUGCAAUUCCAGAUAGUGUACAA |
| 235 | HPV18_25_HR&LR(-45)_3823 | CAUACCAUAGUGAAACACAAAGAAC |
| 236 | HPV18_25_HR&LR(-45)_3752 | CUAUAGAGAUAUAUCAUCCACCUGG |
| 237 | HPV18_25_HR&LR(-45)_3727 | ACAGAUUGCGAAAACAUAGCGACCA |
| 238 | HPV18_25_HR&LR(-45)_3647 | AAGACGGAAACUCUGUAGUGGUAAC |
| 239 | HPV18_25_HR&LR(-45)_3622 | CAGCUACACCUACAGGCAACAACAA |
| 240 | HPV18_25_HR&LR(-45)_3597 | GGACCUGUCAACCCACUUCUCGGUG |
| 241 | HPV18_25_HR&LR(-45)_3572 | UGGACUCGCGGAGAAGCAGCAUUGU |
| 242 | HPV18_25_HR&LR(-45)_3547 | CGGCUGCUACACGACCUGGACACUG |
| 243 | HPV18_25_HR&LR(-45)_3499 | AUUCCAGCACCGUGUCCGUGGGCAC |
| 244 | HPV18_25_HR&LR(-45)_3454 | CCGCUACUCAGCUUGUUAAACAGCU |
| 245 | HPV18_25_HR&LR(-45)_3382 | GGGAAGUACAUUUUGGGAAUAAUGU |
| 246 | HPV18_25_HR&LR(-45)_3315 | GAAGGGUACAACACGUUUUAUAUAG |
| 247 | HPV18_25_HR&LR(-45)_3269 | CAAAACCGCUACCUGUGUAAGUCAC |
| 248 | HPV18_25_HR&LR(-45)_3244 | AUAUGACUGAUGCAGGAACAUGGGA |
| 249 | HPV18_25_HR&LR(-45)_3219 | UAUGUAGCAUGGGACAGUGUGUAUU |
| 250 | HPV18_25_HR&LR(-45)_3168 | GGCCAAACAGUACAAGUAUAUUUUG |
| 251 | HPV18_25_HR&LR(-45)_3134 | GAAUACAGAACCUACUCACUGCUUU |
| 252 | HPV18_25_HR&LR(-45)_3080 | AAGUCGAUACAAAACCGAGGAUUGG |
| 253 | HPV18_25_HR&LR(-45)_2972 | ACAUGGCAUACAGACAUUAAACCAC |
| 254 | HPV18_25_HR&LR(-45)_2938 | GUUGGGAAAAUGCAAUAUUCUUUGC |
| 255 | HPV18_25_HR&LR(-45)_2903 | CAUAGACAGCCAAAUACAGUAUUGG |
| 256 | HPV18_25_HR&LR(-45)_2645 | GCAAAGGAUAAUAGAUGGCCAUAUU |
| 257 | HPV18_25_HR&LR(-45)_2612 | CCUCCAAUACUACUAACCACAAAUA |
| 258 | HPV18_25_HR&LR(-45)_2527 | CUUUGAUACCUAUAUGAGAAAUGCG |
| 259 | HPV18_25_HR&LR(-45)_2475 | CAGAUACUAAGGUGGCCAUGUUAGA |
| 260 | HPV18_25_HR&LR(-45)_2270 | CUGCGAUACCAACAAAUAGAGUUUA |
| 261 | HPV18_25_HR&LR(-45)_2202 | CACAGUGGAUACGAUUUAGAUGUUC |
| 262 | HPV18_25_HR&LR(-45)_2065 | UGAAUAUGCCUUAUUAGCAGACAGC |
| 263 | HPV18_25_HR&LR(-45)_2036 | GAGCUGACAGAUGAAAGCGAUAUGG |
| 264 | HPV18_25_HR&LR(-45)_1944 | CUGAGUGGAUACAAAGACUUACUAU |
| 265 | HPV18_25_HR&LR(-45)_1918 | UAUUAGUGAAGUAAUGGGAGACACA |
| 266 | HPV18_25_HR&LR(-45)_1829 | CACGUACCUGAAACUUGUAUGUUAA |
| 267 | HPV18_25_HR&LR(-45)_1802 | GUUGCUAAAGGUUUAAGUACGUUGU |
| 268 | HPV18_25_HR&LR(-45)_1777 | CAAAUGUGGUAAGAGUAGACUAACA |
| 269 | HPV18_25_HR&LR(-45)_1751 | GUAUUAAUAUUAGCCCUGUUGCGUU |
| 270 | HPV18_25_HR&LR(-45)_1726 | UCAAUGUCUAGACUGUAAAUGGGGA |
| 271 | HPV18_25_HR&LR(-45)_1572 | ACACAUAUGGGCUAUCAUUUACAGA |
| 272 | HPV18_25_HR&LR(-45)_1536 | ACAAUAAACAAGGAGCUAUGUUAGC |
| 273 | HPV18_25_HR&LR(-45)_1493 | CCACAAUGUACCAUAGCACAAUUAA |
| 274 | HPV18_25_HR&LR(-45)_1455 | ACGGUACAAGUGACAAUAGCAAUAU |
| 275 | HPV18_25_HR&LR(-45)_1429 | CACAGAGGGCAACAACAGCAGUGUA |
| 276 | HPV18_25_HR&LR(-45)_1399 | CGGCAGUACGGAGGCUAUAGACAAC |
| 277 | HPV18_25_HR&LR(-45)_1360 | AACUACAAAUGGCGAACAUGGCGGC |
| 278 | HPV18_25_HR&LR(-45)_1216 | GCGGCUGGAGGUGGAUACAGAGUUA |
| 279 | HPV18_25_HR&LR(-45)_1149 | CACAAGUGUUGCAUGUUUUAAAACG |
| 280 | HPV18_25_HR&LR(-45)_1072 | ACAAGGAACAUUUUGUGAACAGGCA |
| 281 | HPV18_25_HR&LR(-45)_959 | GGCUGGUUUUAUGUACAAGCUAUUG |
| 282 | HPV18_25_HR&LR(-45)_885 | CGUGGUGUGCAUCCCAGCAGUAAGC |
| 283 | HPV18_25_HR&LR(-45)_857 | UUUCUGAACACCCUGUCCUUUGUGU |
| 284 | HPV18_25_HR&LR(-45)_816 | UAGAAAGCUCAGCAGACGACCUUCG |
| 285 | HPV18_25_HR&LR(-45)_791 | UGUGAAGCCAGAAUUGAGCUAGUAG |
| 286 | HPV18_25_HR&LR(-45)_695 | GAAGAAAACGAUGAAAUAGAUGGAG |
| 287 | HPV18_25_HR&LR(-45)_670 | UCACGAGCAAUUAAGCGACUCAGAG |
| 288 | HPV18_25_HR&LR(-45)_645 | AUGAAAUUCCGGUUGACCUUCUAUG |
| 289 | HPV18_25_HR&LR(-45)_620 | AUUGUAUUGCAUUUAGAGCCCCAAA |
| 290 | HPV18_25_HR&LR(-45)_589 | UAUGCAUGGACCUAAGGCAACAUUG |
| 291 | HPV18_25_HR&LR(-45)_554 | CCAACGACGCAGAGAAACACAAGUA |
| 292 | HPV18_25_HR&LR(-45)_529 | GCAACCGAGCACGACAGGAACGACU |
| 293 | HPV18_25_HR&LR(-45)_489 | AACAUAGCUGGGCACUAUAGAGGCC |
| 294 | HPV18_25_HR&LR(-45)_344 | UUAUUCAGACUCUGUGUAUGGAGAC |
| 295 | HPV18_25_HR&LR(-45)_264 | GUGGUGUAUAGAGACAGUAUACCCC |
| 296 | HPV18_25_HR&LR(-45)_216 | GUAUUGGAACUUACAGAGGUAUUUG |
| 297 | HPV18_25_HR&LR(-45)_179 | GCAAGACAUAGAAAUAACCUGUGUA |
| 298 | HPV18_25_HR&LR(-45)_154 | UGUGCACGGAACUGAACACUUCACU |
| 299 | HPV18_25_HR&LR(-45)_92 | ACACCACAAUACUAUGGCGCGCUUU |
| 300 | HPV18_7601 | CCUGGUAUUAGUCAUUUUCCUGUCC |
| 301 | HPV18_6850 | CUAGUUUGGUGGAUACAUAUCGUUU |
| 302 | HPV18_5697 | ACUCCCACAAGCAUAUUUUAUCAUG |
| 303 | HPV18_5046 | GUAGUGAUGUUCCUGAUUCAGAUUU |
| 304 | HPV18_2877 | GACCACUAUGAAAAUGACAGUAAAG |
| 305 | HPV18_1298 | CUGUUUACAAUAUCAGAUAGUGGCU |
| 306 | HPV18_1241 | AGUCCACGGUUACAAGAAAUAUCUU |
| 307 | HPV18_739 | AGCCCGACGAGCCGAACCACAACGU |
| 308 | HPV18_405 | UUAUUAAUAAGGUGCCUGCGGUGCC |
| 309 | HPV18_289 | AUGCUGCAUGCCAUAAAUGUAUAGA |

Also described is an isolated polynucleotide for specific hybridization to HPV 45 consisting essentially of a sequence or a complement thereof selected from the group consisting of: SEQ ID NOs: 842-974 (See Table3). Also described is a set of polynucleotides for specific hybridization to HPV 45, wherein the set comprises at least one polynucleotide consisting essentially of a sequence or a complement thereof selected from the group consisting of: SEQ ID NOs: 842-974. Also described is a set of polynucleotides for specific hybridization to HPV 45, wherein the set comprises at least one polynucleotide consisting essentially of a sequence or a complement thereof selected from the group consisting of: SEQ ID NOs: 842-968. In certain embodiments, the methods of the present invention utilize a set of polynucleotide probes for specific hybridization to HPV 45 comprising SEQ ID NOs: 842-974. In certain embodiments, the methods of the present invention utilize a set of polynucleotide probes for specific hybridization to HPV 45 comprising SEQ ID NO: 842-849, 851-893, 895-917, 919-929, 931, 933-936, 938-974.

**Table 3: Polyribonucleotide probes for determining HPV 45 nucleic acid.**

| SEQ ID NO: | Name | Sequence |
|---|---|---|
| 842 | HPV45_25_HR&LR(-18)_7834 | GGCCCUAUAACACAUACCUUUUCUU |
| 643 | HPV45_25_HR&LR(-18)_7754 | CCAACAAUCUGUCUACUUGUUACAU |
| 844 | HPV45_25_HR&LR(-18)_7726 | UAAUUGGCGUGUAGAACCACUUUCU |
| 845 | HPV45_25_HR&LR(-18)_7646 | GCACAACUGUAUCCACACCCUAUGU |
| 846 | HPV45_25_HR&LR(-18)_7552 | ACAUAGUUUAACCUACUGGCGCGCC |
| 847 | HPV45_25_HR&LR(-18)_7527 | CUAAACUGGCACAUUUACAACCCCU |
| 848 | HPV45_25_HR&LR(-18)_7495 | GUGGCUUAUAUGUGACCUUUUAAAC |
| 849 | HPV45_25_HR&LR(-18)_7440 | GCAUCCAUUUUACUUAUAAUCCUCC |
| 850 | HPV45_25_HR&LR(-18)_7385 | CUUUGUACCCUAUAUUCUUUCCUGU |
| 851 | HPV45_25_HR&LR(-18)_7322 | UAAUAGUGUUGUGUAGGGUUGCACC |
| 852 | HPV45_25_HR&LR(-18)_7282 | GGUGUUACUGUACAUAAUUGUGGUA |
| 853 | HPV45_25_HR&LR(-18)_7250 | GUGUAUGUAUGAAUGUGCCUUGUGG |
| 854 | HPV45_25_HR&LR(-18)_7225 | UACUGUAUUUUGUUUGUUUGCGUGC |
| 855 | HPV45_25_HR&LR(-18)_7106 | CAUCUAGGCCUGCCAAACGUGUACG |
| 856 | HPV45_25_HR&LR(-18)_7081 | GCUUCCACGUCUACUGCAUCUACUG |
| 857 | HPV45_25_HR&LR(-18)_7052 | CUACCAUAGGACCUCGUAAGCGUCC |
| 858 | HPV45_25_HR&LR(-18)_7027 | GUUCAGGCUGGGUUACGUCGUAGGC |
| 859 | HPV45_25_HR&LR(-18)_6911 | AUACUACACCUCCAGAAAAGCAGGA |
| 860 | HPV45_25_HR&LR(-18)_6885 | AUCAGUUGCUGUUACCUGUCAAAAG |
| 861 | HPV45_25_HR&LR(-18)_6697 | UUUAAGCAGUAUAGUAGACAUGUGG |
| 862 | HPV45_25_HR&LR(-18)_6672 | GCCAAGUACAUAUGACCCUACUAAG |
| 863 | HPV45_25_HR&LR(-18)_6505 | GGCUCUAUUAUUACUUCUGAUUCUC |
| 864 | HPV45_25_HR&LR(-18)_6479 | GUUGUGUGUAUUCCCCUUCUCCCAG |
| 865 | HPV45_25_HR&LR(-18)_6454 | GCUAAUAUGCGUGAAACCCCUGGCA |
| 866 | HPV45_25_HR&LR(-18)_6426 | UACGGACCUAUAUAUUAAAGGCACU |
| 867 | HPV45_25_HR&LR(-18)_6272 | CAUUAGACAUUUGUCAAUCCAUCUG |
| 868 | HPV45_25_HR&LR(-18)_6247 | UUGCAGGAUACAAAGUGCGAGGUUC |
| 869 | HPV45_25_HR&LR(-18)_6142 | GCACAAUUGCAACCUGGUGACUGUC |
| 870 | HPV45_25_HR&LR(-18)_6018 | AGCUGUUAUUACGCAGGAUGUUAGG |
| 871 | HPV45_25_HR&LR(-18)_5833 | GUAGCUUUACCCGAUCCUAAUAAAU |
| 872 | HPV45_25_HR&LR(-18)_5791 | GCUGUUCCUAAGGUAUCCGCAUAUC |
| 873 | HPV45_25_HR&LR(-18)_5766 | ACCUAAUGGUGCAGGUAAUAAACAG |
| 874 | HPV45_25_HR&LR(-18)_5741 | UAGGCAAUCCAUAUUUUAGGGUUGU |
| 875 | HPV45_25_HR&LR(-18)_5654 | CUUCUGUGGCCAGAGUUGUCAGCAC |
| 876 | HPV45_25_HR&LR(-18)_5534 | CACACAAUAUUAUUUAUGGCCAUGG |
| 877 | HPV45_25_HR&LR(-18)_5490 | UCUCCUACCAAUGCUUCCACCACCA |
| 878 | HPV45_25_HR&LR(-18)_5465 | CCAUACUCCUAUGUGGCCUAGUACA |
| 879 | HPV45_25_HR&LR(-18)_5437 | AUACUGGCCCGGACAUUAUAUUGCC |
| 880 | HPV45_25_HR&LR(-18)_5402 | AGUACCAUUAACAUCUGCAUGGGAU |
| 881 | HPV45_25_HR&LR(-18)_5372 | UACUGCUGCAUCCUCUUACAGUAAU |
| 882 | HPV45_25_HR&LR(-18)_5347 | CAAAGUAUUCCUUGACCAUGCCUUC |
| 883 | HPV45_25_HR&LR_(-18)_5314 | CACCUAGCACUAUACACAAAUCAUU |
| 884 | HPV45_25_HR&LR_(-18)_5289 | GACUUCCCACCUCCUGCGUCCACUA |
| 885 | HPV45_25_HR&LR(-18)_5254 | CUACAAAUGAUAGUGACCUGUUUGA |
| 886 | HPV45_25_HR&LR(-18)_5209 | CCAUUGCUGCUACAGAGGAAAUUGA |
| 887 | HPV45_25_HR&LR(-18)_5111 | CACUGUUAGAUUUAGUAGAUUGGGU |
| 888 | HPV45_25_HR&LR(-18)_5038 | CCAGUAAUGUUCCUGAUUCCGAUUU |
| 889 | HPV45_25_HR&LR(-18)_5013 | GACACCACACUAUCCUUUGAGCCUA |
| 890 | HPV45_25_HR&LR(-18)_4974 | UCGUUGGUUACAUUUGAUAAUCCAG |
| 891 | HPV45_25_HR&LR(-18)_4926 | AAUCAACAGGUCCGUGUGUCCACCU |
| 892 | HPV45_25_HR&LR(-18)_4837 | CAUCUUCUGGGUCAGGUACGGAACC |
| 893 | HPV45_25_HR&LR(-18)_4781 | UGGUACACCAACAUCGGGCAGCCAU |
| 894 | HPV45_25_HR&LR(-18)_4716 | GCAUUUUCUGAUCCCUCUAUUAUUG |
| 895 | HPV45_25_HR&LR(-18)_4679 | CUCUGUUUCUAUUUCGUCAACUAGU |
| 896 | HPV45_25_HR&LR(-18)_4654 | UGUUGGACAUCACACCUACCGUGGA |
| 897 | HPV45_25_HR&LR(-18)_4573 | UUGCCUCUGGUGCUCCGGUUCCCAC |
| 898 | HPV45_25_HR&LR(-18)_4463 | CAGGUCUAAUACUGUUGUGGAUGUU |
| 899 | HPV45_25_HR&LR(-18)_4367 | UUUACAGUGGUCUAGCCUUGGGAUA |
| 900 | HPV45_25_HR&LR(-18)_4224 | GUUUAAUAAACCAUGGUAUCCCACC |
| 901 | HPV45_25_HR&LR(-18)_4158 | AUACCUGUGAUGUGCAUGUUGUUGU |
| 902 | HPV45_25_HR&LR(-18)_4106 | GCAUGCUUUACACACCAUACAAUAA |
| 903 | HPV45_25_HR&LR(-18)_4053 | GCAUUUGCUGUAUACAUUUGUUGCU |
| 904 | HPV45_25_HR&LR(-18)_3989 | UGUGUGUGCUUUUGCUUGGUUGUUG |
| 905 | HPV45_25_HR&LR(-18)_3944 | GUGCCUUUAUGUGUGCUGCAAUGUC |
| 906 | HPV45_25_HR&LR(-18)_3857 | GGGAUACAUGACUAUAUGAAUCUGU |
| 907 | HPV45_25_HR&LR(-18)_3832 | UUCCUAACAGUGUACAAAUCUCGGU |
| 908 | HPV45_25_HR&LR(-18)_3717 | UACUCAGAAAUAUCCUCCACCUGGC |
| 909 | HPV45_25_HR&LR(-18)_3685 | UAAGAUAUAGGCUACGCAAAUAUGC |
| 910 | HPV45_25_HR&LR(-18)_3612 | AGAAGGAAAGUGUGUAGUGGUAACA |
| 911 | HPV45_25_HR&LR(-18)_3585 | CUGUGUUCAAGUACAAGUAACAACA |
| 912 | HPV45_25_HR&LR(-18)_3535 | UCACAGAGCAGCACCACGGACGUGU |
| 913 | HPV45_25_HR&LR(-18)_3492 | CACAUCCAGACGCCGGCUACUAAGC |
| 914 | HPV45_25_HR&LR(-18)_3429 | AGACAGCUACAACACGCCUCCACGU |
| 915 | HPV45_25_HR&LR(-18)_3325 | GAAAUAGUAAUACGUGGGAAGUACA |
| 916 | HPV45_25_HR8LR(-18)_3241 | GUGUUAGCUAUUGGGGUGUAUAUUA |
| 917 | HPV45_25_HR&LR(-18)_3216 | GGGAUAUGGGACAAAACAGCAGCAU |
| 918 | HPV45_25_HR&LR(-18)_3173 | GAACUAUGUAGUAUGGGACAGUAUA |
| 919 | HPV45_25_HR&LR(-18)_3134 | CGUGCACGUAUACUUUGAUGGCAAC |
| 920 | HPV45_25_HR&LR(-18)_3092 | GAAUACAGAACCGUCGCAGUGUUUU |
| 921 | HPV45_25_HR&LR(-18)_3039 | AGCAAGUAUAACAAUGAGGAAUGGA |
| 922 | HPV45_25_HR&LR(-18)_2918 | UACAGCAAGGGAACAUGGUAUUACC |
| 923 | HPV45_25_HR&LR(-18)_2883 | UGGCAACUUAUACGUUUGGAAAAUG |
| 924 | HPV45_25_HR&LR(-18)_2850 | GACAGUAAAGACAUAAACAGCCAAA |
| 925 | HPV45_25_HR&LR(-18)_2765 | GACGAUGAAGAUGCAGACACCGAAG |
| 926 | HPV45_25_HR&LR(-18)_2642 | ACGGUAUUUACAUUUCCACAUGCAU |
| 927 | HPV45_25_HR&LR(-18)_2586 | CAUCCAAUAUUGAUCCAGCAAAAGA |
| 928 | HPV45_25_HR&LR(-18)_2560 | GCUAAAAUGUCCUCCAAUCCUAUUA |
| 929 | HPV45_25_HR&LR(-18)_2431 | AGCAGAUACUAAGGUAGCCAUGUUG |
| 930 | HPV45_25_HR&LR(-18)_2358 | GUUUUAUACAUUUCCUACAAGGUGC |
| 931 | HPV45_25_HR&LR(-18)_2266 | GGCACUAAAGGAAUUUCUUAAAGGA |
| 932 | HPV45_25_HR&LR(-18)_1781 | UUGUUGCACGUACCUGAAACAUGUA |
| 933 | HPV45_25_HR&LR(-18)_1754 | CUAACUGUUGCAAAAGGCUUAAGCA |
| 934 | HPV45_25_HR&LR(-18)_1676 | GCCCAUAUCCAAUGUUUAGAUUGUA |
| 935 | HPV45_25_HR&LR(-18)_1599 | GGGUAAUGGCUAUAUUUGGAGUUAA |
| 936 | HPV45_25_HR&LR(-18)_1541 | CUGUCAUUUACGGAUUUGGUUAGAA |
| 937 | HPV45_25_HR&LR(-18)_1516 | GGCAGUAUUUAAAGACAUAUAUGGG |
| 938 | HPV45_25_HR&LR(-18)_1474 | AAAGGAGCUAUUACAAGCAAGUAAC |
| 939 | HPV45_25_HR&LR(-18)_1449 | AUCCGCAUUGCAGUAUUACAGAACU |
| 940 | HPV45_25_HR&LR(-18)_1424 | AGUAGUGACAAUGCAGAAAAUGUAG |
| 941 | HPV45_25_HR&LR(-18)_1399 | UAGUACACAAAGUAGUGGUGGGGAU |
| 942 | HPV45_25_HR&LR(-18)_1365 | UAAACACUAAUGCGGAAAAUGGCGG |
| 943 | HPV45_25_HR&LR(-18)_1338 | UGGAAGCUGCAGAGACUCAGGUAAC |
| 944 | HPV45_25_HR&LR(-18)_1242 | GUCCACGGUUACAAGAAAUUUCAUU |
| 945 | HPV45_25_HR&LR(-18)_1217 | CAGCUAAGUGUGGAUACGGAUCUAA |
| 946 | HPV45_25 HR&LR(-18)_1153 | GGUGUUGCAUCUUUUAAAACGAAAG |
| 947 | HPV45_25_HR&LR(-18)_1124 | CAUGCGCAGGAAGUUCAGAAUGAUG |
| 948 | HPV45_25_HR&LR(-18)_1072 | ACAAUUAUCCAUUUGUGAACAGGCA |
| 949 | HPV45_25_HR&LR(-18)_954 | GUAAUGGCUGGUUCUUUGUAGAAAC |
| 950 | HPV45_25_HR&LR(-18)_897 | CUAACCAAUAAUCUACAAUGGCGGA |
| 951 | HPV45_25_HR&LR(-18)_832 | GGACCUUAGAACACUACAGCAGCUG |
| 952 | HPV45_25_HR&LR(-18)_799 | CAGAAUUGAGCUUACAGUAGAGAGC |
| 953 | HPV45_25_HR&LR(-18)_649 | AGAUCCUGUUGACCUGUUGUGUUAC |
| 954 | HPV45_25_HR&LR(-18)_624 | UGCAUUUGGAACCUCAGAAUGAAUU |
| 955 | HPV45_25_HR&LR(-18)_596 | CCCCGGGAAACACUGCAAGAAAUUG |
| 956 | HPV45_25_HR&LR(-18)_570 | CAAGUAUAGCAAUAAGUAUGCAUGG |
| 957 | HPV45_25_HR&LR(-18)_536 | ACGGCAAGAAAGACUUCGCAGACGU |
| 958 | HPV45_25_HR&LR(18)_511 | AGUGUAAUACAUGUUGUGACCAGGC |
| 959 | HPV45_25_HR&LR(-18)_486 | AGCAUAGCUGGACAGUACCGAGGGC |
| 960 | HPV45_25_HR&LR(-18)_461 | CCUUAAGGACAAACGAAGAUUUCAC |
| 961 | HPV45_25_HR&LR(-18)_348 | AACUCUGUAUAUGGAGAGACACUGG |
| 962 | HPV45_25_HR&LR(-18)_265 | UGUAUAGAGACUGUAUAGCAUAUGC |
| 963 | HPV45_25_HR&LR(-18)_218 | GGAACGCACAGAGGUAUAUCAAUUU |
| 964 | HPV45_25_HR&LR(-18)_188 | UAUUGCCUGUGUAUAUUGCAAAGCA |
| 965 | HPV45_25_HR&LR(-18)_163 | UGAAUACAUCACUACAAGACGUAUC |
| 966 | HPV45_25_HR&LR(-18)_138 | AAGCUACCAGAUUUGUGCACAGAAU |
| 967 | HPV45_25_HR&LR(-18)_113 | UGACGAUCCAAAGCAACGACCCUAC |
| 968 | HPV45_25_HR&LR(-18)_87 | AAAGUGCAUUACAGGAUGGCGCGCU |
| 969 | HPV45_7599 | CCUGGUAUUAGUCAUUUUCCUGUCC |
| 970 | HPV45_6860 | UGGUGGAUACAUAUCGUUUUGUGCA |
| 971 | HPV45_2617 | AUGGCCAUAUUUAGAAAGUAGGGUG |
| 972 | HPV45_1297 | GUUGUUUACAAUAUCAGAUAGUGGC |
| 973 | HPV45_733 | ACUACCAGCCCGACGAGCCGAACCA |
| 974 | HPV45_414 | UGCCUGCGGUGCCAGAAACCAUUGA |

Also described is an isolated polynucleotide for specific hybridization to HPV 31 consisting essentially of a sequence or a complement thereof selected from the group consisting of SEQ ID NOs: 310-454 (See Table4). Also described is a set of polynucleotides for specific hybridization to HPV 31, wherein the set comprises at least one polynucleotide consisting essentially of a sequence or a complement thereof selected from the group consisting of: SEQ ID NOs: 310-454. In certain embodiments, the methods of the present invention utilize a set of polynucleotide probes for specific hybridization to HPV 31 comprising SEQ ID NOs: 310-454.

**Table 4: Polyribonucleotide probes for determining HPV 31 nucleic acid.**

| SEQ ID NO: | Name | Sequence |
|---|---|---|
| 310 | HPV31_7871 | GUUUUCGGUUACAGUUUUACAAGCA |
| 311 | HPV31_7799 | CCAAGGUUGUGUCAUGCAUUAUAAA |
| 312 | HPV31_7760 | CCUUGAUUGCAGUGCUGGCUUUUGC |
| 313 | HPV31_7709 | CCUACACACCUUAAACUGCUUUUAG |
| 314 | HPV31_7670 | UGUAGUUCAACUAUGUGUCAUGCAC |
| 315 | HPV31_7620 | CCAGUCCAACUUUGCAAUUAUACUA |
| 316 | HPV31_7595 | CUAACACACCUUGCCAACAUAUAAU |
| 317 | HPV31_7570 | AACAUUCUGGCUUGUAGUUUCCUGC |
| 318 | HPV31_7502 | CAUGCUAGUACAACUAUGCUGAUGC |
| 319 | HPV31_7462 | CAUUUUAAAUCCCUAACCGUUUUCG |
| 320 | HPV31_7437 | CUACUCCAUUUUGAUUUUAUGCAGC |
| 321 | HPV31_7396 | UAGUAAAAGUUGUACACCCGGUCCG |
| 322 | HPV31_7350 | CAAUAGUCAUGUACUUAUUUCUGCC |
| 323 | HPV31_7325 | UGUUCCUACUUGUUCCUGCUCCUCC |
| 324 | HPV31_7261 | GUUGUCCUUAUAUACACCCUAUUAG |
| 325 | HPV31_7232 | AUAUGUGUAUACCUGUGUGUGUUGU |
| 326 | HPV31_7111 | GCUGUAUUGUAUAUGUGUGUGUUUG |
| 327 | HPV31_7086 | UGUGUCUGUAUGUGUAUGUGCUUGU |
| 328 | HPV31_7024 | AUCUACCACUACACCAGCAAAACGU |
| 329 | HPV31_6984 | GUCCUAAAUUUAAAGCAGGUAAACG |
| 330 | HPV31_6860 | CCCAAGGAAGAUCCAUUUAAAGAUU |
| 331 | HPV31_6786 | CAGGUUCUUUGGAGGAUACCUAUAG |
| 332 | HPV31_6593 | GCAAUUGCAAACAGUGAUACUACAU |
| 333 | HPV31_6567 | GUAGUACCAAUAUGUCUGUUUGUGC |
| 334 | HPV31_6424 | AUACUUUCCUACACCUAGCGGCUCC |
| 335 | HPV31_6390 | GCUCCGGUUCAACAGCUACUUUAGC |
| 336 | HPV31_6358 | UGAAUCGGUCCCUACUGACUUAUAU |
| 337 | HPV31_6197 | GACACUAAAAGUAAUGUUCCUUUGG |
| 338 | HPV31_6089 | GCUAUUACCCCUGGUGAUUGUCCUC |
| 339 | HPV31_6017 | CAACUGUGUUUACUUGGUUGCAAAC |
| 340 | HPV31_5962 | CGGUGGUCCUGGCACUGAUAAUAGG |
| 341 | HPV31_5701 | UUCCAUACCUAAAUCUGACAAUCCU |
| 342 | HPV31_5666 | AGUGCUAGGCUGCUUACAGUAGGCC |
| 343 | HPV31_5640 | GAACCAACAUAUAUUAUCACGCAGG |
| 344 | HPV31_5596 | UGUCCCAGUGUCUAAAGUUGUAAGC |
| 345 | HPV31_5571 | GCGAGGCUACUGUCUACUUACCACC |
| 346 | HPV31_5440 | GCCCCUACAACGCCACAAGUGUCUA |
| 347 | HPV31_5415 | UACACAGGUUUUCCCAUUUCCUUUG |
| 348 | HPV31_5390 | CUGAUGUACCUAUAGAGCAUGCACC |
| 349 | HPV31_5364 | UUUUGACAUUCCCAUAUUUUCUGGG |
| 350 | HPV31_5337 | AAAUACCACUGUGCCACUAAGUACA |
| 351 | HPV31_5294 | CUGCUGUACAGUCCACAUCUGCUGU |
| 352 | HPV31_5258 | UGGAUACACCUGCCACACAUAAUGU |
| 353 | HPV31_5173 | AUGCAACCUUUAGGGGCGUCUGCAA |
| 354 | HPV31_5148 | UAAUCCUGCAGGUGAAAGUAUUGAA |
| 355 | HPV31_5097 | UGGUGCUACUAUUGGUGCAAGGGUG |
| 356 | HPV31_5072 | AUAAACAAACUUUGCGCACUCGUAG |
| 357 | HPV31_5046 | CACUGUUAGAUAUAGUAGACUAGGU |
| 358 | HPV31_4990 | CCCGACUUUCUAGAUAUUAUAGCAU |
| 359 | HPV31_4965 | UACAUCGCAUAAUAUAGCCCCUGAU |
| 360 | HPV31_4922 | CCUAUGAAACUGUAAAUGCUGAAGA |
| 361 | HPV31_4888 | GCUCCAAAACAGCUAAUUACAUAUG |
| 362 | HPV31_4841 | GUAAGGCUACACAACAAGUAAAAGU |
| 363 | HPV31_4782 | CAUAACAAGUAGCACACCCAUUCCA |
| 364 | HPV31_4688 | CAGGUCAUUUACUACUUUCAUCAUC |
| 365 | HPV31_4663 | CAGCCUCCUACACCUGCAGAAACAU |
| 366 | HPV31_4622 | GCACACAUGAAAAUCCUACUUUUAC |
| 367 | HPV31_4583 | CAGACACAACACCUGCAAUUUUAGA |
| 368 | HPV31_4558 | UCUGGGUUUGACAUUGCUACAACUG |
| 369 | HPV31_4533 | UCCUAUACCACACCCUCCUACAACA |
| 370 | HPV31_4508 | GAAUUGUUGAUGUUGGUGCCCCUGC |
| 371 | HPV31_4478 | CCUCUAUAGUAAGUCUUGUUGAAGA |
| 372 | HPV31_4442 | CACCAGUUAGCAUUGACCCUGUAGG |
| 373 | HPV31_4417 | UCUGAGGCAAGUAUACCUAUUAGAC |
| 374 | HPV31_4392 | UCUUAGUACACGUCCUUCUACAGUA |
| 375 | HPV31_4303 | AUAUUAAGGUAUGGUAGUAUGGGUG |
| 376 | HPV31_4255 | CCAUCAGACGUUAUACCUAAAAUAG |
| 377 | HPV31_4182 | ACGCUCUACAAAACGCACUAAACGU |
| 378 | HPV31_3967 | UUAUUGCAACCUCUCCAUUACGUUG |
| 379 | HPV31_3923 | GUCGGUAUAUGCAACACUACUAUUA |
| 380 | HPV31_3898 | UCAUACGUCCACUUGUGCUGUCUGU |
| 381 | HPV31_3873 | UGUGUGCUACUAUUUGUGUGUCUUG |
| 382 | HPV31_3789 | CAACAGGAUAUAUGACUAUUUAGCC |
| 383 | HPV31_3673 | UUGGACAUGUACAGAUGGAAAACAU |
| 384 | HPV31_3645 | UGUAUGAACAAGUGUCAUCUACAUG |
| 385 | HPV31_3561 | CUGCAACUACACCUAUAAUACACUU |
| 386 | HPV31_3536 | AACCAAACAAGGGCUGUCAGUUGUC |
| 387 | HPV31_3506 | UGUGGGGUUAUCAGUGCAGCUGCAU |
| 388 | HPV31_3428 | CCAAGAACAGAGCCAGAGCACAGAA |
| 389 | HPV31_3361 | GAAUUCCAAAACCUGCGCCUUGGGC |
| 390 | HPV31_3308 | UCCUUUGCUGGGAUUGUUACAAAGC |
| 391 | HPV31_3281 | GAAUCUGUAUUUAGCAGUGACGAAA |
| 392 | HPV31_3158 | GGCAUUUAUUAUGUACAUGAAGGAC |
| 393 | HPV31_3133 | UGUGGAAGGGCAAGUUAAUUGUAAG |
| 394 | HPV31_3108 | UAUGUAUAGAUGGCCAAUGUACUGU |
| 395 | HPV31_3073 | CACCAUGCAUUAUACUAACUGGAAA |
| 396 | HPV31_3046 | GGUGCAAUUUGAUGGUGAUGUACAC |
| 397 | HPV31_2988 | UUGAACUGUAUUUAACUGCACCUAC |
| 398 | HPV31_2963 | GACUGGACAAUGCAGCAAACAAGUC |
| 399 | HPV31_2897 | GCCUUACAAGCUAUUGAACUACAAA |
| 400 | HPV31_2870 | CCAGCGUUGUCAGUAUCAAAGGCCA |
| 401 | HPV31_2839 | GGGAAUACACAGUAUUAACCACCAG |
| 402 | HPV31_2783 | GACUAUUGGAAACAUAUUCGACUUG |
| 403 | HPV31_2698 | GACUCUUUCUCAACGUUUAAAUGUG |
| 404 | HPV31_2660 | UAAAUUUGCACGAGGAAGAGGACAA |
| 405 | HPV31_2520 | UGACAGAUGGCCAUACCUACAUAGC |
| 406 | HPV31_2430 | CCCUGUAUCUAUAGAUGUAAAGCAU |
| 407 | HPV31_2402 | AUUACCUACGAAAUGCACUAGAUGG |
| 408 | HPV31_2222 | UAAUACAUGGUGCACCUAAUACAGG |
| 409 | HPV31_2109 | AGGUGACUGGAGGGACAUAGUAAAG |
| 410 | HPV31_2084 | GUAGAUGUGACAAAGUUAGUGACGA |
| 411 | HPV31_1949 | CUGACAGUGAUAGUAAUGCAUGUGC |
| 412 | HPV31_1855 | GACACAACAUUUGAUUUGUCCCAAA |
| 413 | HPV31_1712 | GUAUGUUAAUUCAGCCACCCAAAUU |
| 414 | HPV31_1591 | UUACAAAGUUUAGCAUGUUCCUGGG |
| 415 | HPV31_1566 | GCAACCAUAUUGUUUGUAUUGCCAU |
| 416 | NPV31_1540 | GUUGCAGAAGGAUUUAAAACCCUAU |
| 417 | HPV31_1515 | AGCUGCGUUUGGAGUUACAGGUACA |
| 418 | HPV31_1490 | AAAGCACAUGUACUGAUUGGUGUGU |
| 419 | HPV31_1462 | GAACUAAUUAGGCCAUUUCAAAGCA |
| 420 | HPV31_1408 | GGUAAAGCUGCUAUGUUAGGUAAAU |
| 421 | HPV31_1369 | CCAACACGUAAUAUAUUGCAAGUGU |
| 422 | HPV31_1344 | ACAUAGUGAACGAGAGAAUGAAACU |
| 423 | HPV31_1319 | UAAGUUGUAAUGGUAGUGACGGGAC |
| 424 | HPV31_1294 | CAGGUAGAGGAGCAACAAACAACAU |
| 425 | HPV31_1269 | UGAAGUGGAAACGCAGCAGAUGGUA |
| 426 | HPV31_1233 | ACUCUUUGAACUUCCAGACAGCGGG |
| 427 | HPV31_1181 | CACGGUUAAAAGCUAUAUGCAUAGA |
| 428 | HPV31_1084 | GCGGAGGAACAUGCAGAGGCUGUGC |
| 429 | HPV31_994 | GAGGAUAUGGUUGACUUUAUUGACA |
| 430 | HPV31_965 | ACGAAAAUGAAGACAGUAGUGAUAC |
| 431 | HPV31_940 | CAGACAGGGGACAACAUUUCAGAGG |
| 432 | HPV31_907 | GGUUGGUUUUAUGUAGAAGCAGUAA |
| 433 | HPV31_848 | AGACUGUAACUACAAUGGCUGAUCC |
| 434 | HPV31_814 | CUCAUUUGGAAUCGUGUGCCCCAAC |
| 435 | HPV31_789 | GCAUAUUGCAAGAGCUGUUAAUGGG |
| 436 | HPV31_764 | GUACAGAGCACACAAGUAGAUAUUC |
| 437 | HPV31_727 | CUUUUGUUGUCAGUGUAAGUCUACA |
| 438 | HPV31_700 | GGACACAUCCAAUUACAAUAUCGUU |
| 439 | HPV31_662 | GAGGAUGUCAUAGACAGUCCAGCUG |
| 440 | HPV31_629 | UGUUAUGAGCAAUUACCCGACAGCU |
| 441 | HPV31_594 | UGUUAGAUUUGCAACCUGAGGCAAC |
| 442 | HPV31_569 | GAAACACCUACGUUGCAAGACUAUG |
| 443 | HPV31_535 | CUCGUACUGAAACCCAAGUGUAAAC |
| 444 | HPV31_510 | CGUUGCAUAGCAUGUUGGAGAAGAC |
| 445 | HPV31_478 | GAUUCCACAACAUAGGAGGAAGGUG |
| 446 | HPV31_340 | GGUAUAGAUAUAGUGUGUAUGGAAC |
| 447 | HPV31_287 | CGGAGUGUGUACAAAAUGUUUAAGA |
| 448 | HPV31_262 | UAGUAUAUAGGGACGACACACCACA |
| 449 | HPV31_220 | CAGAAACAGAGGUAUUAGAUUUUGC |
| 450 | HPV31_186 | AGAUUGAAUUGUGUCUACUGCAAAG |
| 451 | HPV31_161 | AUUGGAAAUACCCUACGAUGAACUA |
| 452 | HPV31_136 | GGAAAUUGCAUGAACUAAGCUCGGC |
| 453 | HPV31_89 | GUGCAAACCUACAGACGCCAUGUUC |
| 454 | HPV31_60 | CGGUUGGUAUAUAAAGCACAUAGUA |

Also described is an isolated polynucleotide for specific hybridization to HPV 33 consisting essentially of a sequence or a complement thereof selected from the group consisting of: SEQ ID NOs: 455-579 (See Table 5). Also described is a set of polynucleotides for specific hybridization to HPV 33, wherein the set comprises at least one polynucleotide consisting essentially of a sequence or a complement thereof selected from the group consisting of: SEQ ID NOs: 455-579. In certain embodiments, the methods of the present invention utilize a set of polynucleotide probes for specific hybridization to HPV 33 comprising SEQ ID NOs:455-579.

**Table 5: Polyribonucleotide probes for determining HPV 33 nucleic acid.**

| SEQ ID NO: | Name | Sequence |
|---|---|---|
| 455 | HPV33_7867 | CCGUUUUAGGUCAUAUUGGUCAUUU |
| 456 | HPV33_7831 | UGAGUCACUACCUGUUUAUUACCAG |
| 457 | HPV33_7805 | GUAUGCCAAACUAUGCCUUGUAAAA |
| 458 | HPV33_7780 | CAGUUUUGGCUUACACAAUUGCUUU |
| 459 | HPV33_7680 | UCAUAUAUACAUGCAGUGCAAUUGC |
| 460 | HPV33_7655 | GUUUGUCUGUACUUGCUGCAUUGAC |
| 461 | HPV33_7630 | UUAAUCCUUUUCUUUCCUGCACUGU |
| 462 | HPV33_7605 | AUACCCUAUGACAUUGGCAGAACAG |
| 463 | HPV33_7576 | GUUUGUCUGUACUUGCUGCAUUGGC |
| 464 | HPV33_7551 | UUAAUCCUUUUCUUUCCUGCACUGU |
| 465 | HPV33_7526 | AUACCCUAUGACAUUGGCAGAACAG |
| 466 | HPV33_7465 | GUCCAUAUUGUACAAUUUCCUCCAU |
| 467 | HPV33_7425 | CCUACAUGUUUAGUAUUGCUUUACC |
| 468 | HPV33_7389 | CAAUGUACCUACCUUUAUUUCCCUA |
| 469 | HPV33_7364 | GUAUUGCUUGCCCUACCCUGCAUUG |
| 470 | HPV33_7339 | GGUGUACCUAUAUGAGUAAGGAGUU |
| 471 | HPV33_7228 | UGUACUUGUUUGUGUGCAUGUUCUA |
| 472 | HPV33_7129 | CUGUCUAUGUACUUUGUGUUGUUGU |
| 473 | HPV33_7051 | CACCCGCACAUCGUCUGCAAAACGC |
| 474 | HPV33_7016 | GCAAAACCUAAACUUAAACGUGCAG |
| 475 | HPV33_6914 | GGUAAAUAUACAUUUUGGGAAGUGG |
| 476 | HPV33_6804 | GUUUAACACCUCCUCCAUCUGCUAG |
| 477 | HPV33_6630 | CACAAGUAACUAGUGACAGUACAUA |
| 478 | HPV33_6490 | GGUUACUUCCGAAUCUCAGUUAUUU |
| 479 | HPV33_6434 | GGAACUACUGCCUCUAUUCAAAGCA |
| 480 | HPV33_6405 | UUCCCGAUGACCUGUACAUUAAAGG |
| 481 | HPV33_6380 | AGGGCUGGUACAUUAGGAGAGGCUG |
| 482 | HPV33_6135 | CUGCCAAUGAUUGUCCACCUUUAGA |
| 483 | HPV33_6109 | AGGUGUUGCUUGUACUAAUGCAGCA |
| 484 | HPV33_6063 | UAUGUUUACUUGGAUGUAAGCCUCC |
| 485 | HPV33_6004 | UGGACAACCGGGUGCUGAUAAUAGG |
| 486 | HPV33_5979 | ACACUGAAACCGGUAACAAGUAUCC |
| 487 | HPV33_5902 | UGUAGGCCUUGAAAUAGGUAGAGGG |
| 488 | HPV33_5839 | UAAAUUUGGAUUUCCUGACACCUCC |
| 489 | HPV33_5783 | CCCAAAGUAUCAGGCUUGCAAUAUA |
| 490 | HPV33_5521 | GCUGACUUUGUUUUACAUCCUAGUU |
| 491 | HPV33_5496 | UUUUGACACCAUUGUUGUAGACGGU |
| 492 | HPV33_5462 | CUAGCCCAUUUGUUCCUAUUUCGCC |
| 493 | HPV33_5412 | UACUCCUGUUAUGUCUGGCCCUGAU |
| 494 | HPV33_5375 | CCAGCAAUGUGUCUAUACCUUUAAA |
| 495 | HPV33_5349 | AUACAGUACGUUUGCAACAACACGU |
| 496 | HPV33_5324 | AUGUACACACCCCAAUGCAACACUC |
| 497 | HPV33_5299 | GAUGUUUAUGCUGACGAUGUGGAUA |
| 498 | HPV33_5249 | CUUUACAUGAUACUUCUACAUCGUC |
| 499 | HPV33_5219 | CCGUGCCAAAUGAACAAUAUGAAUU |
| 500 | HPV33_5194 | AGUCCUAUUGUGCCUUUAGACCACA |
| 501 | HPV33_5164 | GGAGCUAGAAUACAUUAUUAUCAGG |
| 502 | HPV33_5092 | CGUAGACAUACUGUGCGUUUUAGUA |
| 503 | HPV33_4993 | CCUGAAGACACAUUACAAUUUCAAC |
| 504 | HPV33_4888 | UUAUAUAGUCGCAAUACCCAACAGG |
| 505 | HPV33_4836 | UGUAACAUCAAGCACGCCCAUUCCA |
| 506 | HPV33_4811 | UUGUUGUUUCCACAGACAGUAGUAA |
| 507 | HPV33_4775 | GCACACAAAGUUAUGAAAACAUACC |
| 508 | HPV33_4742 | CUGGACAUUUUAUAUUUUCUUCCCC |
| 509 | HPV33_4715 | UACACCCUCCAGCGCCUGCAGAAGC |
| 510 | HPV33_4652 | GGGAGUCAUCUAUUCAAACUAUUUC |
| 511 | HPV33_4603 | ACUACAUCUGCAGAUACUACACCUG |
| 512 | HPV33_4568 | CCCCAUCUAUUCCUACACCAUCAGG |
| 513 | HPV33_4510 | GACUCGUCUAUAGUGUCAUUAAUAG |
| 514 | HPV33_4485 | UACUGUAGACACUGUUGGACCUUUA |
| 515 | HPV33_4460 | CCUUGCAGCCUAUACGUCCUCCGGU |
| 516 | HPV33_4435 | ACUGACCCACCUACAGCUGCAAUCC |
| 517 | HPV33_4317 | AGGAAGUACCAUAGCAGAUCAAAUU |
| 518 | HPV33_4119 | CAUGGUGGUGUUUUAACAUUGUUGU |
| 519 | HPV33_4060 | GCAUAUGACACAACAAGAGUAAUGU |
| 520 | HPV33_3969 | UUUGGGUGUUUGUGGGAUCUCCUUU |
| 521 | HPV33_3944 | UGGUUGCUGGUGUUGGUAUUGCUGC |
| 522 | HPV33_3773 | CUACUGUGCAAAUAAGUACUGGAUU |
| 523 | HPV33_3719 | CAUUUGUAACUGAACAGCAACAACA |
| 524 | HPV33_3646 | UAUAGUUCUAUGUCAUCCACCUGGC |
| 525 | HPV33_3555 | UAGUUCUAACGUUGCACCUAUAGUG |
| 526 | HPV33_3530 | GCACAAACAAGCAGCGGACUGUGUG |
| 527 | HPV33_3497 | UGGACAAUAGAACAGCACGUACUGC |
| 528 | HPV33_3463 | CCCCUUACAAAGCUGUUCUGUGCAG |
| 529 | HPV33_3408 | ACCACAAGCAGCGGCCAAACGACGA |
| 530 | HPV33_3380 | ACAUACAGACAGACAACGAUAACCG |
| 531 | HPV33_3338 | CGUCUAUAUCUAGCAACCAAAUAUC |
| 532 | HPV33_3185 | CUAUGGUUACAGGGAAAGUAGAUUA |
| 533 | HPV33_3135 | GGAUUAUACAAACUGGGGUGAAAUA |
| 534 | HPV33_3096 | AGUAACUGUGCAAUAUGACAAUGAC |
| 535 | HPV33_3008 | AUAGUACAAGCCAAUGGACAUUGCA |
| 536 | HPV33_2939 | CAUCAAAGACCAAAGCAUUUCAAGU |
| 537 | HPV33_2895 | GGGAUUUUCACAUUUAUGCCACCAG |
| 538 | HPV33_2867 | GUGCUUUAUUGUAUACAGCCAAACA |
| 539 | HPV33_2809 | GCUGAUAAAACUGAUUUACCAUCAC |
| 540 | HPV33_2654 | CCCAGUGUAUGCAAUAAAUGAUGAA |
| 541 | HPV33_2576 | CUCUAGAUGGCCAUAUUUACAUAGU |
| 542 | HPV33_2526 | UUAAAAUGUCCACCACUGCUUCUUA |
| 543 | HPV33_2454 | GAUGAUUACAUGAGAAAUGCGUUAG |
| 544 | HPV33_2419 | UAGAUGAUGUAACGCCAAUAAGUUG |
| 545 | HPV33_2269 | GCUGUAUGCUAAUUUGUGGACCAGC |
| 546 | HPV33_2174 | GAGACCAAUAGUACAGUUGUUAAGA |
| 547 | HPV33_2004 | GCAGAUUCAAAUAGUAAUGCUGCUG |
| 548 | HPV33_1951 | AUGAUAACGAGUUAACGGACGAUAG |
| 549 | HPV33_1795 | GGAGCCAAACAUGUGCAUUGUAUUG |
| 550 | HPV33_1763 | AACAUGUAUGGUUAUAGAGCCACCA |
| 551 | HPV33_1715 | CAGGUUAACAGUAGCAAAACUAAUG |
| 552 | HPV33_1567 | GUAUAACAGGAUAUGGAAUUAGUCC |
| 553 | HPV33_1496 | GGCCUAUGGAAUAAGUUUUAUGGAA |
| 554 | HPV33_1426 | CGUUGCAGGAAAUUAGUAAUGUUCU |
| 555 | HPV33_1395 | GAGACAAAUGUAGAUAGCUGUGAAA |
| 556 | HPV33_1345 | UAAAUGACUUAGAAUCUAGUGGGGU |
| 557 | HPV33_1320 | GAAAGUCAAAAUGGCGACACAAACU |
| 558 | HPV33_1295 | AACUCAGCAGAUGGUACAACAGGUA |
| 559 | HPV33_1183 | AUCGUGCUGCAAACCCGUGUAGAAC |
| 560 | HPV33_1154 | UUCACAAAGUGCUGCGGAGGACGUU |
| 561 | HPV33_1009 | GCACGGAUUUACUAGAGUUUAUAGA |
| 562 | HPV33_984 | GAGGAUGAAACAGCAGAUGACAGUG |
| 563 | HPV33_870 | UCAUCUACAAUGGCCGAUCCUGAAG |
| 564 | HPV33_830 | AGUGAAUAUUGUGUGCCCUACCUGU |
| 565 | HPV33_805 | CCAUACAGCAACUACUUAUGGGCAC |
| 566 | HPV33_780 | AACAGUACAGCAAGUGACCUACGAA |
| 567 | HPV33_742 | GUUGUCACACUUGUAACACCACAGU |
| 568 | HPV33_717 | ACAGCUGAUUACUACAUUGUAACCU |
| 569 | HPV33_617 | AUAUCCUGAACCAACUGACCUAUAC |
| 570 | HPV33_575 | GAGAGGACACAAGCCAACGUUAAAG |
| 571 | HPV33_539 | GUAGAGAAACUGCACUGUGACGUGU |
| 572 | HPV33_490 | AUUUCGGGUCGUUGGGCAGGGCGCU |
| 573 | HPV33_457 | CGACAUGUGGAUUUAAACAAACGAU |
| 574 | HPV33_424 | UGUCAAAGACCUUUGUGUCCUCAAG |
| 575 | HPV33_301 | CUGUGUUUGCGGUUCUUAUCUAAAA |
| 576 | HPV33_274 | GAGGGAAAUCCAUUUGGAAUAUGUA |
| 577 | HPV33_214 | CCUUUGCAACGAUCUGAGGUAUAUG |
| 578 | HPV33_183 | CAUUGAACUACAGUGCGUGGAAUGC |
| 579 | HPV33_103 | ACGACUAUGUUUCAAGACACUGAGG |

Also described is an isolated polynucleotide for specific hybridization to HPV 35 consisting essentially of a sequence or a complement thereof selected from the group consisting of: SEQ ID NOs: 580-722 (See Table 6). Also described is a set of polynucleotides for specific hybridization to HPV 35, wherein the set comprises at least one polynucleotide consisting essentially of a sequence or a complement thereof selected from the group consisting of: SEQ ID NOs:580-722. In certain embodiments, the methods of the present invention utilize a set of polynucleotide probes for specific hybridization to HPV 35 comprising SEQ ID NOs:580-722.

**Table 6: Polyribonucleotide probes for determining HPV 35 nucleic acid.**

| SEQ ID NO: | Name | Sequence |
|---|---|---|
| 580 | HPV35_7767 | CACAUUGUUAUAUGCACACAGGUGU |
| 581 | HPV35_7737 | CAUGCAUGUAAAACAUUACUCACUG |
| 582 | HPV35_7711 | CACAUCCUGCCAACUUUAAGUUAAA |
| 583 | HPV35_7648 | CUAAAGGGCUUUAAUUGCACACCUU |
| 584 | HPV35_7606 | AACACUUGUAACAGUGCUUUUAGGC |
| 585 | HPV35_7549 | CUUGAUUCAUCUUGCAGUAUUAGUC |
| 586 | HPV35_7493 | GUGUUCCUGAUAUAUAUUGUUUGCC |
| 587 | HPV35_7424 | GGUUGCUGUUGGUAAGCUUUAUAUG |
| 588 | HPV35_7393 | GUGUCCUUUACAUUACCUUUCAACC |
| 589 | HPV35_7327 | GAGCUUACAUAAUUACAUGACAGCU |
| 590 | HPV35_7302 | UUGUAUGACUAUGGUGCACCGAUAU |
| 591 | HPV35_7261 | GCCAUAAAGUGAUGUGUGUGUUUAU |
| 592 | HPV35_7236 | GUACUUAGUGUGUAGUAGUUCAGUA |
| 593 | HPV35_7206 | UUGUGCAAUGUGUUGUACGUGGGUG |
| 594 | HPV35_7180 | CAUGGCGUGUAAAUGUGUGUAUAAU |
| 595 | HPV35_7155 | UGUUGUGGUGCCUGUUUGUGUUGUA |
| 596 | HPV35_7108 | CAUGUAUACUGUGUGUUAUGUGUUG |
| 597 | HPV35_7028 | GCGUGCAGCUCCAGCAUCUACAUCU |
| 598 | HPV35_6874 | CACCAAAACCUAAAGAUGAUCCAUU |
| 599 | HPV35_6825 | ACAUAUCGCUAUGUAACAUCACAGG |
| 600 | HPV35_6759 | AACCCGUCCAUUUUAGAGGAUUGGA |
| 601 | HPV35_6592 | GUACAAAUAUGUCUGUGUGUUCUGC |
| 602 | HPV35_6474 | GUAACCUCCGAUGCACAAAUAUUUA |
| 603 | HPV35_6439 | GUACUAGUUAUUUUCCUACUCCUAG |
| 604 | HPV35_6392 | AGUACCUGCAGACCUAUAUAUUAAG |
| 605 | HPV35_6296 | UUCUGAGCCAUAUGGAGAUAUGUUA |
| 606 | HPV35_6245 | CCUAGAUAUAUGCAGUUCCAUUUGC |
| 607 | HPV35_6141 | CCUUUGGAGUUACUAAACACUGUAC |
| 608 | HPV35_6115 | ACCAGGUAAAAGCAGGAGAAUGUCC |
| 609 | HPV35_6045 | CAAUUGUGUUUAAUAGGUUGUAGGC |
| 610 | HPV35_6006 | GAUAACAGGGAAUGCAUUUCUAUGG |
| 611 | HPV35_5981 | UGGUAACUCUGGUAACUCUGGUACA |
| 612 | HPV35_5877 | UGUACAGGAGUUGAAGUAGGUCGUG |
| 613 | HPV35_5849 | UCCCUGCCUCCAGCGUUUGGUUUGG |
| 614 | HPV35_5748 | AAAAUAGCAGUACCCAAGGUAUCUG |
| 615 | HPV35_5682 | GCAGGCAGUUCUAGGCUAUUAGCUG |
| 616 | HPV35_5589 | UCUAACGAAGCCACUGUCUACCUGC |
| 617 | HPV35_5465 | CCCACAGGUCCUAUAUAUUCUAUUA |
| 618 | HPV35_5440 | UAUUACUAACUCUGUACUACCGGUA |
| 619 | HPV35_5412 | GGCCAGACAUUGUAUUUAACUCUAA |
| 620 | HPV35_5387 | GGCUAUGAUAUUCCUAUAACAGCAG |
| 621 | HPV35_5354 | GUUCCUAGCAAUACUACUAUACCAU |
| 622 | HPV35_5274 | CUCCUAUAGAUACUGAGGAAGAUAU |
| 623 | HPV35_5223 | CACAUACCACUGUUUCAACAUCAUU |
| 624 | HPV35_5198 | UUACAACAUGUACCAUCCUCUUUAC |
| 625 | HPV35_5120 | AGUGGAAAAGCUAUAGGGGCACGGG |
| 626 | HPV35_5094 | GUAAUAAACGUACUAUGCAUACACG |
| 627 | HPV35_5050 | UGCACUAACAUCUAGGAAAGGCACU |
| 628 | HPV35_5024 | AUGGACAUUAUAGCUUUACAUAGGC |
| 629 | HPV35_4993 | GGAUAUUAGCUUAGCUCCGGAUCCU |
| 630 | HPV35_4967 | GAUACAACCUUACAAUUUGAGCAUG |
| 631 | HPV35_4909 | GACUUCUCCUGCAAAACUUAUUACA |
| 632 | HPV35_4857 | GAUUAUAUAGUAAAGGUACCCAGCA |
| 633 | HPV35_4800 | GCAAUAAUAUAACUAAUAGCACGCC |
| 634 | HPV35_4713 | CAGGUCAUUUUGUACUUUCAUCAUC |
| 635 | HPV35_4688 | CACCCACCCACGCCUGCAGAAACUU |
| 636 | HPV35_4634 | GUGACAUCCAUAAGUACACAUGAUA |
| 637 | HPV35_4605 | CUACAGAUACCACACCUGCUAUUUU |
| 638 | HPV35_4578 | CUACAACAGGUUUUACAAUAACCAC |
| 639 | HPV35_4553 | ICCUGUUGUUACACCAAGGGUCCCAC |
| 640 | HPV35_4506 | CUAUAGUGUCAUUAGUAGAGGAAAC |
| 641 | HPV35_4481 | GACACAAUUGGCCCUUUAGAUUCUU |
| 642 | HPV35_4426 | GGCUGCCACAAACAUUCCUAUACGA |
| 643 | HPV35_4401 | UUCCACUGGGUACAACACCUCCAAC |
| 644 | HPV35_4376 | GGCACAGGUGGAAGAUCUGGAUAUG |
| 645 | HPV35_4233 | AACUAUAUCGUACUUGCAAAGCUGC |
| 646 | HPV35_4190 | CACAAAAGGUCUACAAAACGUGUUA |
| 647 | HPV35_4068 | GUAACAUGUGUGUAUGGUGGUUUUA |
| 648 | HPV35_4026 | GGCAGUACAGUAAUUGUAUACAAAC |
| 649 | HPV35_3999 | GAUGAUUAACGCUCAUGCACAAUAU |
| 650 | HPV35_3958 | CUACUUGCUUUUGUUGUUUCUUGCU |
| 651 | HPV35_3933 | ACUGUGGGUUACUGUAGCAACACCA |
| 652 | HPV35_3889 | CUAUCUGUGUCAUUAUACUCAGCAU |
| 653 | HPV35_3864 | GUGUCUGCUUGUACGUUCGCUAUUG |
| 654 | HPV35_3839 | UGUGCUUUUGUGUGCUUUUGUGCUU |
| 655 | HPV35_3807 | AGCUUCCAGUACUGUGUUGCUGUGC |
| 656 | HPV35_3760 | CACAGUUACAGUGUCUAAAGGAUAU |
| 657 | HPV35_3705 | CUUACACAACAGAAUAUCAAAGGGA |
| 658 | HPV35_3652 | AUGGAGAUGGACAUGUACAAACGAU |
| 659 | HPV35_3513 | ACUGCACAAACAAAGACCGGUGUGG |
| 660 | HPV35_3481 | CAGUGUUGACAGAGGGGUCUACUCU |
| 661 | HPV35_3456 | AGCGAGUGCGACUCAGUGCCGUGGA |
| 662 | HPV35_3431 | ACCGAGCUCCCCUACAACCCCACCA |
| 663 | HPV35_3399 | AGAAGACAAAUCACAAACGACUUCG |
| 664 | HPV35_3360 | CCCAUACCAAAGCCUGCUCCGUGGG |
| 665 | HPV35_3293 | UUUAGCAGCACAGAACUAUCCACUG |
| 666 | HPV35_3196 | UUAUGUUACUUUUAGGGAAGAGGCU |
| 667 | HPV35_3171 | AUGUGCAUCAGGGUGUAGAAACAUA |
| 668 | HPV35_3123 | GUAUAUGUACUGUUGUAAAGGGACU |
| 669 | HPV35_3047 | GAAGCACAAUUUGAUGGUGAUAAAC |
| 670 | HPV35_2946 | CAACUGAGUAUAGCACAGAGGACUG |
| 671 | HPV35_2890 | AAAAGCCAAAGCAAUGCAAGCAAUU |
| 672 | HPV35_2865 | AAGUGGUUCCAACGCAGGCCAUUUC |
| 673 | HPV35_2840 | AUGGGAAUUAAAACUCUUAACCACC |
| 674 | HPV35_2788 | GUAUUGGAAACUGAUUCGUCUUGAA |
| 675 | HPV35_2763 | GCACAUGUUUGUCUGAUCACAUACA |
| 676 | HPV35_2679 | AGAGGUCAAAGAAAAUGAUGGAGAC |
| 677 | HPV35_2648 | GGACGUGGUGCAGAUUAAAUUUGCA |
| 678 | HPV35_2551 | GUAGUGGUCUUUACAUUUCACAAUG |
| 679 | HPV35_2526 | CAGGUGGCCAUACUUACAUAGCAGG |
| 680 | HPV35_2386 | CCAUGUGGCAUAUAUAGACCAAUAU |
| 681 | HPV35_2338 | CAGCCAUUAUAUGAUGCCAAAAUAG |
| 682 | HPV35_2275 | CUAAUGCAUUUCUUACAAGGAGCUA |
| 683 | HPV35_2220 | UUGCAUACUAAUAUAUGGAGCACCA |
| 684 | HPV35_2147 | GAUAUCAACAAGUAGAUUUUGUGGC |
| 685 | HPV35_2075 | CACAGUGGAUUAAAAGGCGAUGUGC |
| 686 | HPV35_1955 | CAGAAACUAAUAGUAAUGCAUGUGC |
| 687 | HPV35_1791 | UAUUAGUGAGGUUGAUGGAGAAACA |
| 688 | HPV35_1744 | CGUAGUACCCCAGCUGCGUUAUAUU |
| 689 | HPV35_1698 | GCUAUGUAUUUCAGCUGCAAGUAUG |
| 690 | HPV35_1619 | GGGCUAUGGUAAUUCUAGCAUUAUU |
| 691 | HPV35_1559 | GUGUGGCGAACUUUAAACAUAUAAC |
| 692 | HPV35_1534 | GUGGCCGCAUUUGGAAUAGCCCCAA |
| 693 | HPV35_1391 | CAACGCGAGACAUAAUACAAAUACU |
| 694 | HPV35_1366 | AGCGAUGAAAGACAUGAUGAGACUC |
| 695 | HPV35_1341 | CAGUGGGGAUAGUAUAACCUCUAGU |
| 696 | HPV35_1316 | AUACAGUUGAACAAUGUAGUAUGGG |
| 697 | HPV35_1286 | UACACGAGAUACAACAGGUAGAGGG |
| 698 | HPV35_1237 | CGAUUAUUUGAACUACCAGACAGCG |
| 699 | HPV35_1136 | CUAGUAGUCCACUUAGCAGCGUGAG |
| 700 | HPV35_1101 | CAAAGAGGCUGUACAGGUCCUAAAA |
| 701 | HPV35_1051 | GAAACAGAGACAGCACAAGCAUUAU |
| 702 | HPV35_970 | GACGAAAAUGAAGAUGACUGUGACA |
| 703 | HPV35_945 | UAGACGUACGGGAUCCAGUGUAGAG |
| 704 | HPV35_858 | AUAAUCUACAAUGGCUGAUCCUGCA |
| 705 | HPV35_828 | AAUAGUGUGCCCCGGCUGUUCACAG |
| 706 | HPV35_781 | CACAUUGACAUACGUAAAUUGGAAG |
| 707 | HPV35_739 | UGUAAAUGUGAGGCGACACUACGUC |
| 708 | HPV35_703 | CCAGACACCUCCAAUUAUAAUAUUG |
| 709 | HPV35_669 | AGAUACUAUUGACGGUCCAGCUGGA |
| 710 | HPV35_592 | UAUGUUUUAGAUUUGGAACCCGAGG |
| 711 | HPV35_554 | GUGUAAUCAUGCAUGGAGAAAUAAC |
| 712 | HPV35_529 | GAAACCAACACGUAGAGAAACCGAG |
| 713 | HPV35_443 | CCAGUUGAAAAGCAAAGACAUUUAG |
| 714 | HPV35_350 | UAUAGUGUGUAUGGAGAAACGUUAG |
| 715 | HPV35_284 | CCAUAUGGAGUAUGCAUGAAAUGUU |
| 716 | HPV35_259 | GUGUAUAGUAUAUAGAGAAGGCCAG |
| 717 | HPV35_232 | GGUAUAUGACUUUGCAUGCUAUGAU |
| 718 | HPV35_207 | GCAAACAAGAAUUACAGCGGAGUGA |
| 719 | HPV35_163 | GGUAGAAGAAAGCAUCCAUGAAAUU |
| 720 | HPV35_131 | CGACCUUACAAACUGCAUGAUUUGU |
| 721 | HPV35_106 | CGGUAUGUUUCAGGACCCAGCUGAA |
| 722 | HPV35_46 | ACGGUUGCCAUAAAAGCAGAAGUGC |

Also described is an isolated polynucleotide for specific hybridization to HPV 39 consisting essentially of a sequence or a complement thereof selected from the group consisting of SEQ ID NOs: 723-841 (See Table 7). Also described is a set of polynucleotides for specific hybridization to HPV 39, wherein the set comprises at least one polynucleotide consisting essentially of a sequence or a complement thereof selected from the group consisting of: SEQ ID NOs: 723-841. In certain embodiments, the methods of the present invention utilize a set of polynucleotide probes for specific hybridization to HPV 39 comprising SEQ ID NOs: 723-841.

**Table 7: Polyribonucleotide probes for determining HPV 39 nucleic acid.**

| SEQ ID NO: | Name | Sequence |
|---|---|---|
| 723 | HPV39_7780 | CACACAAUAGUUUAUGCAACCGAAA |
| 724 | HPV39_7735 | CAGGAAUGUGUCUUACAGUAUAAGU |
| 725 | HPV39_7692 | CUUGCUUAAUUAAAUAGUUGGCCUG |
| 726 | HPV39_7642 | CCACCCUAUGUAAUAAAACUGCUUU |
| 727 | HPV39_7617 | CAAUACUUUGGCAACAUCCAUAUCU |
| 728 | HPV39_7581 | CCUUAUUACUCAUCAUCCUGUCCAG |
| 729 | HPV39_7538 | UUCACCCUGCAUAGUUGGCACUGGU |
| 730 | HPV39_7429 | CAUUUUAUACUUCGCCAUUUUGUGG |
| 731 | HPV39_7349 | UCAUACAUAAUCUAUAUGCCCUACC |
| 732 | HPV39_7273 | AUGACAGUUUCAUGUGUGAUUGCAC |
| 733 | HPV39_7203 | CCUUAUGUGUUGAGUGUAUAUGUGU |
| 734 | HPV39_7173 | CCUUGUUAUGUGUGUGUAUGUUGUU |
| 735 | HPV39_7146 | CGUGUGUCUAAAUAAUGCAUGUGUA |
| 736 | HPV39_7111 | CUUCCUCGUCCUCAGCUACUAAACA |
| 737 | HPV39_7072 | GCCCUACUAUAGGUCCCCGAAAGCG |
| 738 | HPV39_7012 | UGGAACUUGAUCAAUUCCCUUUGGG |
| 739 | HPV39_6956 | AGAUCCAUAUGACGGUCUAAAGUUU |
| 740 | HPV39_6902 | CCUACAGUCUGCAGCCAUUACAUGU |
| 741 | HPV39_6877 | CCAGUUUGGUAGACACUUACAGAUA |
| 742 | HPV39_6851 | UUUUGCUGUAGCUCCUCCACCAUCU |
| 743 | HPV39_6824 | GAAUUCCUCUAUAUUGGACAAUUGG |
| 744 | HPV39_6696 | CCUUCUACAUAUGAUCCUUCUAAGU |
| 745 | HPV39_6671 | AUCUACCUCUAUAGAGUCUUCCAUA |
| 746 | HPV39_6511 | ACUGCCCCUCUCCCAGCGGUUCCAU |
| 747 | HPV39_6486 | CGUGCAAACCCCGGUAGUUCUGUAU |
| 748 | HPV39_6458 | CCAAUUGUAUAUUAAGGGCACAGAU |
| 749 | HPV39_6370 | ACAGUAUGUUCUUCUGUUUACGUAG |
| 750 | HPV39_6204 | GAACUAGUAAACACCCCUAUUGAGG |
| 751 | HPV39_6160 | CAUGCAAGCCCAAUAAUGUAUCUAC |
| 752 | HPV39_6039 | CCAUUUUCAUCAACCACCAAUAAGG |
| 753 | HPV39_5998 | GACACCCAUUAUAUAAUAGACAGGA |
| 754 | HPV39_5908 | CCUUAUAUAAUCCAGAAACACAACG |
| 755 | HPV39_5875 | CCGAUCCUAAUAAAUUCAGUAUUCC |
| 756 | HPV39_5850 | UAUAGGGUAUUUCGCGUGACAUUGC |
| 757 | HPV39_5792 | UAAAGUGGGUAUGAAUGGUGGUCGC |
| 758 | HPV39_5758 | GCUCUAGAUUAUUAACAGUAGGACA |
| 759 | HPV39_5543 | ACAACAUAUGCAAUAACCAUUCAGG |
| 760 | HPV39_5512 | GUUGCCAUUGGUGCCUUCUGGACCA |
| 761 | HPV39_5487 | UUGCUUUACCAAGUACUACUCCACA |
| 762 | HPV39_5462 | AUGCCUGUAAAUACUGGUCCUGAUA |
| 763 | HPV39_5436 | CUAUUCCUUUUAGUACCUCAUGGAA |
| 764 | HPV39_5409 | CAGCAUCUACUAAAUAUGCCAAUAC |
| 765 | HPV39_5384 | GGCUCACUACCUUCUGUGGCUUCUU |
| 766 | HPV39_5359 | GGAUUCGGGCACUACAUAUAACACA |
| 767 | HPV39_5305 | AUAUGCUGAUGUGGACAAUAACACA |
| 768 | HPV39_5264 | CACGCUGAGCCCUCUGAUGCUUCAG |
| 769 | HPV39_5239 | AAGCAUUGAAUUACAGCCCCUAGUU |
| 770 | HPV39_5209 | CCAUGACAUUAGUAGUAUUGCUCCU |
| 771 | HPV39_5178 | GCACACAAAUUGGAGCGCAAGUACA |
| 772 | HPV39_5121 | AAGGAACAGUAAGGUUUAGUAGGCU |
| 773 | HPV39_5018 | GAGCCUGUUGAUACUACAUUAACAU |
| 774 | HPV39_4928 | UAUAGUAGAGCACAUCAGCAGGUUC |
| 775 | HPV39_4889 | CCUACACCUGGAAUCAGUCGUGUGG |
| 776 | HPV39_4778 | UCGGGUAAUAUAUUUGUCAGUACCC |
| 777 | HPV39_4736 | ACGGAUCCUUCCUUAAUUGAGGUUC |
| 778 | HPV39_4706 | ACCUCUACUAGUUAUACUAACCCUG |
| 779 | HPV39_4621 | CACCUCUGGAUUUGAAAUUACUUCU |
| 780 | HPV39_4596 | GAACACCAGUACCAACAUUUACAGG |
| 781 | HPV39_4571 | GAGGACUCAAGUGUUAUAACCUCUG |
| 782 | HPV39_4546 | UGAGCCAUCUAUUGUGCAAUUGGUG |
| 783 | HPV39_4487 | ACUGUUGUAGAUGUGUCUCCUGCAC |
| 784 | HPV39_4358 | GGUACUACACUUGCUGACAAAAUUU |
| 785 | HPV39_4333 | ACCAGACGUUGUUGAUAAAGUUGAG |
| 786 | HPV39_4297 | CCUAUAUAGAACCUGUAAACAAUCG |
| 787 | HPV39_4239 | UACUAAUAAACAUGGUUUCCCACCG |
| 788 | HPV39_4195 | AUUGUGCAUAACUACUGUACAUAGC |
| 789 | HPV39_4158 | GGCAAUGGAUAUGAUAUAGUACUGU |
| 790 | HPV39_4133 | UGCCCAUGUGGUUGUUGCAUAGACU |
| 791 | HPV39_4046 | CGUAUGUGUGGAUAAUUGUGUUUGU |
| 792 | HPV39_3888 | CAUUGGGUUACAUGACAUUGUAAAG |
| 793 | HPV39_3854 | GACACUGUUAAAAUACCUUCUAGUG |
| 794 | HPV39_3818 | ACAUAUGCCACAGAGUCACAACGCC |
| 795 | HPV39_3641 | AGACGGUACCUCAGUUGUGGUAACA |
| 796 | HPV39_3616 | CAGUAACAGUACAGGCCACAACACA |
| 797 | HPV39_3591 | UGGACCAUCUUAACAACCCACUCCA |
| 798 | HPV39_3556 | AGUCACAGAGCCCACUGAGCCCGAC |
| 799 | HPV39_3458 | GAAUUAUCAAACACCACCGCGACCC |
| 800 | HPV39_3426 | ACGGAUCGGUACCCACUACUGAACU |
| 801 | HPV39_3328 | UAUUCAAGAUGCGGAAAGGUAUGGG |
| 802 | HPV39_3301 | GCACCUAAAAGUAUACUAUGAAGUG |
| 803 | HPV39_3199 | GAACUAUGUAUUAUGGGGUGCUAUA |
| 804 | HPV39_3174 | AUGAUGGGGACAAAUGUAAUGCUAU |
| 805 | HPV39_3067 | UGAAUACAAUACAGAGGAGUGGACA |
| 806 | HPV39_2986 | GGUGCCAACCAUAAACAUUUCAAAA |
| 807 | HPV39_2636 | ACGAUAGGUGGCCAUAUUUACGUAG |
| 808 | HPV39_2542 | GGGUAUGCAAUAAGUUUAGAUAGGA |
| 809 | HPV39_2479 | UUAGAUGAUGCAACCGGUACCUGCU |
| 810 | HPV39_2412 | UAUUUCAUAUGUAAACUCCACCAGC |
| 811 | HPV39_2338 | GUUAUAUAUGGACCUGCGAAUACAG |
| 812 | HPV39_2235 | GAGACCCAUAGUACAAUUCUUAAGA |
| 813 | HPV39_2205 | GUGUAGUAAAUGUGAUGAAGGCGGG |
| 814 | HPV39_2056 | GCAAUGUUAGCAGAUUGUAACAGUA |
| 815 | HPV39_1974 | UAGUGUAUUUGACCUAUCGGACAUG |
| 816 | HPV39_1906 | AGUGUGGUAACAGGGGAUACGCCAG |
| 817 | HPV39_1881 | GUAUCGCACAGGUAUAUCCAAUAUU |
| 818 | HPV39_1835 | UUCUGGAGCCUCCUAAACUGCGCAG |
| 819 | HPV39_1789 | GGAAAGGGAUUAAGUACAUUGUUAC |
| 820 | HPV39_1716 | CUUAGACACAAAACAAGGAGUACUA |
| 821 | HPV39_1645 | GUACAUCCAACUAUUGCAGAAGGAU |
| 822 | HPV39_1568 | UAUCCUUUACUGACCUGGUACGUAC |
| 823 | HPV39_1531 | GCUGCAAUGCUAACACAAUUUAAAG |
| 824 | HPV39_1478 | CCAAAUCUCCAACUGCACAAAUUAA |
| 825 | HPV39_1453 | GCUAUAGAUAGUGAAAACCAGGAUC |
| 826 | HPV39_1390 | AAUGGGGAUGCUGAAGGGGAACAUG |
| 827 | HPV39_1283 | GCAGUACGCAGGCAACACAAACGGU |
| 828 | HPV39_1251 | GGGAACACUACAGGAAAUUUCAUUA |
| 829 | HPV39_1189 | AAGUAUACAGACAGCAGUGGCGACA |
| 830 | HPV39_1083 | UGAUUCCACAGAUAUUUGUGUACAG |
| 831 | HPV39_876 | CUCACUAGGAUUUGUGUGUCCGUGG |
| 832 | HPV39_839 | GGGAUACUCUGCGACAACUACAGCA |
| 833 | HPV39_803 | GUAACAACACACUGCAGCUGGUAGU |
| 834 | HPV39_595 | CGUGGACCAAAGCCCACCUUGCAGG |
| 835 | HPV39_567 | GAGAAACCCAAGUAUAACAUCAGAU |
| 836 | HPV39_464 | CACCUAAAUAGCAAACGAAGAUUUC |
| 837 | HPV39_336 | AGCUACGAUAUUACUCGGACUCGGU |
| 838 | HPV39_284 | GAACCACUAGCUGCAUGCCAAUCAU |
| 839 | HPV39_259 | UUUAUAUGUAGUAUAUAGGGACGGG |
| 840 | HPV39_212 | AGACGACCACUACAGCAAACCGAGG |
| 841 | HPV39_7808 | GUUGGGCAUACAUACCUAUACUUUU |

Also described is an isolated polynucleotide for specific hybridization to HPV 51 consisting essentially of a sequence or a complement thereof selected from the group consisting of SEQ ID NOs 975-1120: (See Table 8). Also described is a set of polynucleotides for specific hybridization to HPV 51, wherein the set comprises at least one polynucleotide consisting essentially of a sequence or a complement thereof selected from the group consisting of: SEQ ID NOs: 975-1120. In certain embodiments, the methods of the present invention utilize a set of polynucleotide probes for specific hybridization to HPV 51 comprising SEQ ID NOs: 975-1120.

**Table 8: Polyribonucleotide probes for determining HPV 51 nucleic acid.**

| SEQ ID NO: | Name | Sequence |
|---|---|---|
| 975 | HPV51_7766 | UUGUGUUCUGCCUAUGCUUGCAACA |
| 976 | HPV51_7716 | CCAUCUUACUCAUAUGCAGGUGUGC |
| 977 | HPV51_7689 | GUGCCAAGUUUCUAUCCUACUUAUA |
| 978 | HPV51_7593 | CCGCCCUAUAAUAAUUUAACUGCUU |
| 979 | HPV51_7566 | CUUUAACAAUUGUUGGCACACUGUU |
| 980 | HPV51_7536 | GCUAGUCAUACAACCUAUUAGUCAU |
| 981 | HPV51_7510 | CCUUGUACUUGGCGCGCCUUACCGG |
| 982 | HPV51_7485 | UAGUGCAUACAUCCGCCCGCCCACG |
| 983 | HPV51_7427 | AAGUUUUAAACCACAACUGCCAGUU |
| 984 | HPV51_7394 | GAUUUCGGUUCGUGUACUUUUAGUA |
| 985 | HPV51_7368 | CAGCUGCAGCCAUUUUGAGUGCAAC |
| 986 | HPV51_7265 | AGGGUGGUGUUUCGGUGGCGUCCCU |
| 987 | HPV51_7236 | UGUGGGUAUUACAUUAUCCCCGUAG |
| 988 | HPV51_7211 | CAUUUGUAUGACAUGUACGGGUGUA |
| 989 | HPV51_7131 | GUUGUUCCUGUAUGUAUGAGUUAUG |
| 990 | HPV51_7071 | GUAUGCCUGUAUGUAUAUGUUUGUG |
| 991 | HPV51_6979 | UCAUCGGCAUCCUCUUCCUCUUCCU |
| 992 | HPV51 6939 | CGUACAACGCAAGCCCAGACCAGGC |
| 993 | HPV51_6738 | AACAUUACCUCCGUCUGCUAGUUUG |
| 994 | HPV51_6707 | CUACCAUUCUUGAACAGUGGAAUUU |
| 995 | HPV51_6671 | CUACAGAGGUAAUGGCUUAUUUACA |
| 996 | HPV51 6597 | CUUUAAGCAAUAUAUUAGGCAUGGG |
| 997 | HPV51_6572 | UUUCCCCAACAUUUACUCCAAGUAA |
| 998 | HPV51_6543 | UUUAACUAUUAGCACUGCCACUGCU |
| 999 | HPV51_6514 | ACCUGUGUUGAUACUACCAGAAGUA |
| 1000 | HPV51_6385 | UAUAUAUACUCUGCUACUCCCAGUG |
| 1001 | HPV51_6360 | UAAUGGCCGUGACCCUAUAGAAAGU |
| 1002 | HPV51_6307 | CUUGUAGGUGUUGGGGAAGACAUUC |
| 1003 | HPV51_6160 | GCCACCAAAUCAGACGUCCCUUUGG |
| 1004 | HPV51_6084 | ACUUGUAUCCUCUGUCAUUCAGGAU |
| 1005 | HPV51_5962 | GUUGACAACAAACAGACUCAGUUAU |
| 1006 | HPV51_5922 | AAAUGGCAAUGCACAACAAGAUGUU |
| 1007 | HPV51_5897 | AUGACACAGAAAAUUCACGCAUAGC |
| 1008 | HPV51_5773 | CCGGAUCCAAAUUUAUAUAAUCCAG |
| 1009 | HPV51_5707 | AAAGUAUCUGCAUUUCAAUACAGGG |
| 1010 | HPV51_5682 | AACCUCAACGCGUGCUGCUAUUCCU |
| 1011 | HPV51_5641 | AGACUAAUAACAUUAGGACAUCCCU |
| 1012 | HPV51_5590 | ACAGAAGAAUAUAUCACACGCACCG |
| 1013 | HPV51_5565 | UGCACCUGUGUCUCGAAUUGUGAAU |
| 1014 | HPV51_5469 | UAUACACAUUUACUACGCAAACGCC |
| 1015 | HPV51_5444 | AGGUGGGGAUUACUAUUUGUGGCCC |
| 1016 | HPV51_5418 | GACACCAAGCAUUCUAUUGUUAUAC |
| 1017 | HPV51_5393 | GCCUUAUGUUCCCCACACUUCCAUU |
| 1018 | HPV51_5368 | UAUUGCCCACAUCUCCUACAGUAUG |
| 1019 | HPV51_5343 | CCUAUUCAUACAGGGCCUGAUGUGG |
| 1020 | HPV51_5281 | CUUCAUCUAUGUCUUCAUCUUAUGC |
| 1021 | HPV51_5247 | CACUCCUCUUUGUCUAGGCAGUUGC |
| 1022 | HPV51_5189 | UGAUUUAGAUGAAGCUGAAACAGGU |
| 1023 | HPV51_5142 | CAGCCUUUACUUUCACCUUCUAAUA |
| 1024 | HPV51_5117 | UGCACCAGCUGAUGAACUUGAAAUG |
| 1025 | HPV51_4967 | UCUGGAUAUUAUUACACUGCACCGC |
| 1026 | HPV51_4926 | ACUUUUGAGGAACCUGAUGCUGUUG |
| 1027 | HPV51_4901 | UUUUGAGCCUAUUGACACAUCCAUA |
| 1028 | HPV51_4825 | CCUACACACAGGUUAAAGUUACAAA |
| 1029 | HPV51_4800 | GCUGCUCCCCGCUUGUAUAGUAAGU |
| 1030 | HPV51_4762 | CUAUUAGCAGCACACCUACUCCAGG |
| 1031 | HPV51_4733 | UGCAUCCAAUGUCAGUACUGGUACU |
| 1032 | HPV51_4676 | UUUACUAGUACACUACUCUGGUACU |
| 1033 | HPV51_4633 | CAUCCAUUGAGGCUCCACAAUCUGG |
| 1034 | HPV51_4578 | GGUACUGUACAUGUUUCUAGUACUA |
| 1035 | HPV51_4526 | UACUUCAUCUUCCACAACAACCCCU |
| 1036 | HPV51_4463 | GGUCUCCUAUACCUACCUUUACUGG |
| 1037 | HPV51_4458 | GAGGACUCUAGUAUUAUUCAGUCUG |
| 1038 | HPV51_4425 | CACCAUACUGAACCUUCUAUAGUAA |
| 1039 | HPV51_4400 | GCCACCUAUUAUAAUUGACCUAUGG |
| 1040 | HPV51_4373 | AGGCGUGGUGGAUAUUGCUCCUGCA |
| 1041 | HPV51_4337 | UACUGGAUAUAUCCCUUUAGGUGGU |
| 1042 | HPV51_4253 | GGCCGAUAAAAUAUUACAGUGGAGU |
| 1043 | HPV51_4223 | UGUUGUGAAUAAGGUUGAAGGUACU |
| 1044 | HPV51_4131 | AAUAUGGUGGCUACACGUGCACGGC |
| 1045 | HPV51_4009 | UGUUGCAACAUCCCAAUUAACUACA |
| 1046 | HPV51_3964 | CGUGUUUGCAGCUGCCUUAUUAUUA |
| 1047 | HPV51_3939 | UGUUGCCGCUACUGCUGUCCCAAUA |
| 1048 | HPV51_3861 | GACAUAUUGUAACCAUUGCAGUGUU |
| 1049 | HPV51_3816 | GUACAUAUAUACUGUCACAAGCCAA |
| 1050 | HPV51_3778 | GGGAAUUAUGACACUGUAACUAGUG |
| 1051 | HPV51_3714 | GUGCACAUCAACGGGAAACAUUUAU |
| 1052 | HPV51_3689 | GGCAUUGUUACCAUUGUGUUUGACA |
| 1053 | HPV51_3552 | CAACUCAGACUGCGUUUAUAGUGCA |
| 1054 | HPV51_3495 | CAAACAACCAAAUACACUGUGGAAG |
| 1055 | HPV51_3463 | CUCCACAAUCUCCCCACUGUCCGUG |
| 1056 | HPV51_3438 | GACAGCGACUUACUGAGCCCGACUC |
| 1057 | HPV51_3413 | GAAGCCCAGACACAACAGCGAAAAC |
| 1058 | HPV51_3379 | GACCAAUCCCCUUACCACCUGCGUG |
| 1059 | HPV51_3354 | UUGAACAACUAUCAAACACCCCAAC |
| 1060 | HPV51_3329 | GACGCGUUAUCCACUACUACAACUG |
| 1061 | HPV51_3284 | GGUACUGUAAUAACAUGUCCUGAAU |
| 1062 | HPV51_3259 | ACAACAGUGGGAGGUCUAUAUGUAU |
| 1063 | HPV51_3234 | AAGAUGAAGCCAAAAUAUAUGGGGC |
| 1064 | HPV51_3176 | GACUAUACGGGUAUAUAUUACACUG |
| 1065 | HPV51_3151 | GUGGGUAAAGACAAAUGGAAAUGUG |
| 1066 | HPV51_3102 | CAAUGGACUAUACAAGCUGGAAAUU |
| 1067 | HPV51_3017 | GAACUAUGGUGUGUGGCUCCCAAGC |
| 1068 | HPV51_2992 | AUGGACAAUGCGGGAGACAUGUUAU |
| 1069 | HPV51_2967 | ACAAAUCAGACUAUAACAUGGAACC |
| 1070 | HPV51_2942 | AUGCACAUGGCCUUACAAUCGCUUA |
| 1071 | HPV51_2914 | AAAACAAAAGGCCUGUCAAGCAAUU |
| 1072 | HPV51_2889 | AGGUAGUACCAGCAACAACAGUAUC |
| 1073 | HPV51_2864 | AGAAACUUACGAACAAUCAAUCACC |
| 1074 | HPV51_2829 | GAUAUGAAGCUGCUAUGUUUUAUGC |
| 1075 | HPV51_2623 | GGGAAUGCUGUGUAUACAUUGAAUG |
| 1076 | HPV51_2545 | GAGGAUGCAAACCUAAUGUAUUUAC |
| 1077 | HPV51_2363 | AGCCACUAGAGGAUGCUAAAAUAGC |
| 1078 | HPV51_2307 | GUUUAUGCAAGGGUCCAUUAUUUCA |
| 1079 | HPV51_2280 | GUCAUUAUUUGCAAUGAGCCUAAUG |
| 1080 | HPV51_2243 | AUUGCAUAGUCAUAUAUGGCCCACC |
| 1081 | HPV51_2121 | UGAUAGAGCAAAGGAUGGAGGCAAC |
| 1082 | HPV51_2089 | UUAUCUAUGUCAGCCUGGAUAAGGU |
| 1083 | HPV51_2061 | GCAUUACAAACGAGCACAAAGAAAA |
| 1084 | HPV51_2036 | UAAAAGAUUGUGGGACCAUGGCACG |
| 1085 | HPV51_1927 | GACCAUGAAGUAUUAGAUGAUAGUG |
| 1086 | HPV51_1854 | ACGACAAACGCAACUACAACAUAGU |
| 1087 | HPV51_1819 | AGCAAUACAUAUGGAGAGACACCUG |
| 1088 | HPV51_1600 | CCAUUUUGCAUGUACUACCAUAUAC |
| 1089 | HPV51_1559 | UUUCCCCAAUGGUAGCAGAAAAUUU |
| 1090 | HPV51_1534 | GAUUGGGUUUGUGCAUUGUUUGGCG |
| 1091 | HPV51_1489 | AAUGAGUUGGUACGGGUGUUUAAAA |
| 1092 | HPV51_1438 | GCAAAAGCAACGUUAAUGGCAAAAU |
| 1093 | HPV51_1386 | CUGUGCAAAUGUAGAACUAAACAGU |
| 1094 | HPV51_1317 | UGGCGGUUCACAGAACAGUGUGUGU |
| 1095 | HPV51_1228 | AGGAGAUUACUGGACAGUUAUCCGG |
| 1096 | HPV51_1203 | UCAGGCAAACGAGUCACAAGUUAAA |
| 1097 | HPV51_1178 | AUCAAAACAACACACACAGCCAUAG |
| 1098 | HPV51_1130 | GAAAGUUUCUAGUCAGCCCGCGAAG |
| 1099 | HPV51_1101 | AAACAAAGAGGCUGUGCAUCAGUUA |
| 1100 | HPV51_1076 | UGUUUCAGGCCCAAGAAUUACAGGC |
| 1101 | HPV51_1047 | UCAGGCGGAACAGGAGACAGCACGG |
| 1102 | HPV51 982 | GAAAAUGCAGAUGAUACAGGAUCUG |
| 1103 | HPV51_957 | AGAUAAUGUUUCGGAUGAUGAGGAU |
| 1104 | HPV51_862 | CUAGCAACGGCGAUGGACUGUGAAG |
| 1105 | HPV51_832 | AAGCCUGGUUUGCCCGUGUUGUGCG |
| 1106 | HPV51_800 | CGCGUUGUACAGCAGAUGUUAAUGG |
| 1107 | HPV51_770 | CUGGCAGUGGAAAGCAGUGGAGACA |
| 1108 | HPV51_746 | UUGCAGGUGUUCAAGUGUAGUACAA |
| 1109 | HPV51_720 | CGUGUUACAGAAUUGAAGCUCCGUG |
| 1110 | HPV51_686 | GACCAGCUACCAGAAAGACGGGCUG |
| 1111 | HPV51_661 | GGAGGAUGAAGUAGAUAAUAUGCGU |
| 1112 | HPV51_552 | AUAAAGCCAUGCGUGGUAAUGUACC |
| 1113 | HPV51_503 | GCGCUAAUUGCUGGCAACGUACACG |
| 1114 | HPV51_418 | AGACCACUUGGGCCUGAAGAAAAGC |
| 1115 | HPV51_348 | UGGUACUACAUUAGAGGCAAUUACU |
| 1116 | HPV51_323 | AUAGACGUUAUAGCAGGUCUGUGUA |
| 1117 | HPV51_209 | GUAGAGCAGAUGUAUAUAAUGUAGC |
| 1118 | HPV51_160 | UCUAUGCACAAUAUACAGGUAGUGU |
| 1119 | HPV51_103 | GAAGACAAGAGGGAAAGACCACGAA |
| 1120 | HPV51_75 | GGUAAAAGUAUAGAAGAACACCAUG |

Also described is an isolated polynucleotide for specific hybridization to HPV 52 consisting essentially of a sequence or a complement thereof selected from the group consisting of SEQ ID NOs: 1121-1252 (See Table 9). Also described is a set of polynucleotides for specific hybridization to HPV 52, wherein the set comprises at least one polynucleotide consisting essentially of a sequence or a complement thereof selected from the group consisting of: SEQ ID NOs: 1121-1252. In certain embodiments, the methods of the present invention utilize a set of polynucleotide probes for specific hybridization to HPV 52 comprising SEQ ID NOs: 1121-1252.

**Table 9: Polyribonucleotide probes for determining HPV 52 nucleic acid.**

| SEQ ID NO: | Name | Sequence |
|---|---|---|
| 1121 | HPV52_7871 | UGUUACUCACCAGGUGUGCACUACA |
| 1122 | HPV52_7837 | CGCCAAAUAUGUCUUGUAAAACAUG |
| 1123 | HPV52_7812 | UGUUGGCUUACACAAGUACAUCCUA |
| 1124 | HPV52_7732 | CAAUACAUUGCCUAACAUUGCAUGU |
| 1125 | HPV52_7701 | GCUGACUCACAGGUCCUGCAGUGCA |
| 1126 | HPV52_7676 | GUUGUCCCGCCUAAACUGACUUCUU |
| 1127 | HPV52 7651 | UCCUGCAGUCCACUGGUCUACACUU |
| 1128 | HPV52_7540 | CCAUUUUAAAUCCUAACCGAAUUCG |
| 1129 | HPV52_7509 | CUCUCCAUUUUGUACCAUUUUGUAC |
| 1130 | HPV52_7473 | GUGUCCUACUUUGUUACACUACUAA |
| 1131 | HPV52_7448 | UACCCUGUGUCCCCUGCCCUACCCU |
| 1132 | HPV52_7421 | GCUCCUAAUCUAUUGCAUCUCCUGC |
| 1133 | HPV52_7396 | CACCCACAUGAGUAACAAUACAGUU |
| 1134 | HPV52_7307 | CAGUUCCUGUAUGUAUGUUUUGUGU |
| 1135 | HPV52_7266 | UUUGCAUGUUAUGUAUGUGUGUGCA |
| 1136 | HPV52_7241 | AUUGUUUUGUGUGUGUACUGUGUUG |
| 1137 | HPV52_7200 | UGUCAAACACAGGUUAAAAGGUAAC |
| 1138 | HPV52_7168 | GGUAAUUGUCUGUGUCAUGUAUGUG |
| 1139 | HPV52_7143 | GUUAAAAGGUAACCAUUGUCUGUUG |
| 1140 | HPV52_7112 | GGCCCCACGUACCUCCACAAAGAAG |
| 1141 | HPV52_7087 | CCAAACUAAAACGCCCUGCAUCAUC |
| 1142 | HPV52_7062 | UUACAGGCAGGGCUACAGGCUAGGC |
| 1143 | HPV52_6977 | AAAGGACUAUAUGUUUUGGGAGGUG |
| 1144 | HPV52_6949 | CACCACCUAAAGGAAAGGAAGAUCC |
| 1145 | HPV52_6915 | GUCACUUCUACUGCUAUAACUUGUC |
| 1146 | HPV52_6880 | CACCGUCUGCAUCUUUGGAGGACAC |
| 1147 | HPV52 6828 | CAUAAGAUGGAUGCCACUAUUUUAG |
| 1148 | HPV52_6484 | AAGGGUCUAACUCUGGCAAUACUGC |
| 1149 | HPV52_6459 | CCUGUGCCAGGUGAUUUAUAUAUAC |
| 1150 | HPV52_6368 | CGAGCCAUAUGGUGACAGUUUGUUC |
| 1151 | HPV52_6326 | UAGCAGUGUAUGUAAGUAUCCAGAU |
| 1152 | HPV52_6275 | GGAUUUUAAUACCUUGCAAGCUAGU |
| 1153 | HPV52_6216 | CAGCUCAUUAACAGUGUAAUACAGG |
| 1154 | HPV52_6058 | CUGGUAAACCUGGUAUAGAUAAUAG |
| 1155 | HPV52_6026 | GUUUGAUGAUACUGAAACCAGUAAC |
| 1156 | HPV52_5540 | GCUCCAUCUACAUCUAUUAUUGUUG |
| 1157 | HPV52_5515 | UCCUUUUGUUCCUAUAGCCCCUACA |
| 1158 | HPV52_5490 | CAUUACCUUCGUUACCCACACAUAC |
| 1159 | HPV52_5460 | CUAUGUCCAUUGAGUCAGGUCCUGA |
| 1160 | HPV52_5435 | GGUAUUGACUUUGUAUAUCAACCCA |
| 1161 | HPV52_5385 | CUUCCACACUUUCUACCCAUAAUAA |
| 1162 | HPV52_5360 | UUGCAGCAACCCACGUUUCACUUAC |
| 1163 | HPV52_5314 | CCCUUACACUAUUAAUGAUGGUUUG |
| 1164 | HPV52_5289 | AACCUUUAUUACCACAGUCUGUGUC |
| 1165 | HPV52_5264 | GAAGUUCAGGAAGACAUAGAAUUGC |
| 1166 | HPV52_5239 | UGAUAUUAGUCCUAUCCAGCCUGCU |
| 1167 | HPV52_5076 | AACUUUUACCUGCACCGGAUCCUGA |
| 1168 | HPV52_5036 | GGCGUUGAUACAGAUGAAACUAUAA |
| 1169 | HPV52_4990 | GUCAUCACCACAGAAAUUAGUAACA |
| 1170 | HPV52_4933 | CCUUGGUUUAUAUAGCCGUGCCACA |
| 1171 | HPV52_4884 | GCAGUGUAACAAGUAGUACACCUAU |
| 1172 | HPV52_4859 | ACAUUUGUUACCUCUACUGACAGCA |
| 1173 | HPV52_4821 | CUAUUAGUACACACACCUAUGAAGA |
| 1174 | HPV52_4796 | GGUCAUGUAUUGUUUUCUAGUCCAA |
| 1175 | HPV52_4742 | CCUACAUUCACUGAACCAUCUAUAA |
| 1176 | HPV52_4710 | CAUCUGUACAAUCAGUUUCUACACA |
| 1177 | HPV52_4655 | ACAACAUCUGCAAAUAAUACUCCUG |
| 1178 | HPV52_4628 | AUUCCAUCAGCAACAGGGUUUGAUG |
| 1179 | HPV52_4593 | CAACAUUUAUUGAGUCUGGCGCACC |
| 1180 | HPV52_4556 | CCCUUAGAACCAUCUAUAGUUUCUA |
| 1181 | HPV52_4504 | UAGUAUUACCACGUCCACCAUUCGU |
| 1182 | HPV52_4479 | CAUUGUCCACUCGUCCUCCCACUAG |
| 1183 | HPV52_4452 | GCUCUGGUGGUAGGGCAGGCUAUGU |
| 1184 | HPV52_4424 | GGAGGUUUGGGUAUAGGUACAGGUG |
| 1185 | HPV52_4392 | UUUUAAAAUAUGGCAGCCUAGGGGU |
| 1186 | HPV52_4250 | UAGCUUGUCGCAAUGAGAUACAGAC |
| 1187 | HPV52_4157 | AUAACUGUACAUGUAGAUUGGCUAC |
| 1188 | HPV52_4114 | UGUUUUGUAUUCACUGUCAUGCACA |
| 1189 | HPV52_4055 | AUCUAUUGGGUCACCAUUUAAAGUG |
| 1190 | HPV52_4017 | UAUGCGCAGGUGUUGGUGCUGGUGC |
| 1191 | HPV52_3982 | CAGUGCUUAGGCCGCUCUUGCUAUC |
| 1192 | HPV52_3887 | AACACCCAACACAAGCCAAUAUUGC |
| 1193 | HPV52_3832 | GGUGUCAUGUCAUUGUGAUAUUUGU |
| 1194 | HPV52_3762 | CAGUGAUGAAACACAACGUCAACAA |
| 1195 | HPV52_3681 | GUAUGUUCAAAUUUCAUCUACCUGG |
| 1196 | HPV52_3593 | CAACUUGUACUGCACCUAUAAUACA |
| 1197 | HPV52_3541 | CGGGGACUCGUCACUGCAACUGAGU |
| 1198 | HPV52_3509 | UGCGGGGACAACAAUCCGUGGACAG |
| 1199 | HPV52_3484 | AACACCAAGUACCCCAACAACCUUU |
| 1200 | HPV52_3437 | UACAACCACCACAGAAACGACGACG |
| 1201 | HPV52_3406 | GCAGUGUCCGUGGGUGCCAAAGACA |
| 1202 | HPV52_3381 | AUGCACCGAAACCUCCAAGACCUCC |
| 1203 | HPV52_3208 | GGGUUAUAUUAUUGGUGUGAUGGAG |
| 1204 | HPV52_3176 | GUACAAUUGUAGAAGGACAAGUAGA |
| 1205 | HPV52_3125 | CUAUGGAUUAUACAAACUGGAAGGA |
| 1206 | HPV52_3081 | UGGGUAUACAAUAACAGUGCAAUAC |
| 1207 | HPV52_3036 | UCUAGAAAUGUGGCGUGCAGAACCA |
| 1208 | HPV52_3009 | AGAUGGAUGGACAUUACAACAAACA |
| 1209 | HPV52_2975 | CAUUGGAGGCAUUAAACAAAACACA |
| 1210 | HPV52_2887 | CUGGGAAUAACUCAUAUAGGCCACC |
| 1211 | HPV52_2847 | GACUCGAAUGGAAUGUGUUUUGUUU |
| 1212 | HPV52_2815 | GACCUAAACGCACAAAUUGAACAUU |
| 1213 | HPV52_2788 | CUAGAUCUAUACGAAGCUGAUAGUA |
| 1214 | HPV52_2578 | GGCCAUAUUUACAUAGUAGAUUGGU |
| 1215 | HPV52_2548 | CAAAUACAAAUGCAGGAACAGAUCC |
| 1216 | HPV52_2403 | GUGGGUAUGAUAGAUGAUGUAACAC |
| 1217 | HPV52_2327 | GUUCUUAAGUGGAUGUGUAAUAUCC |
| 1218 | HPV52_2143 | AUAGAAUAGAUGAUGGUGGAGAUUG |
| 1219 | HPV52_1909 | GCAUAUUCGAUUUUGGAGAAAUGGU |
| 1220 | HPV52_1822 | CAGGUUUGUCUAAUAUUAGUGAGGU |
| 1221 | HPV52_1789 | GAAGUGCUACCUGUGCAUUAUAUUG |
| 1222 | HPV52 1753 | CAGAAACACAUAUGGUAAUAGAACC |
| 1223 | HPV52_1723 | CCAAACUAAUGUCACAGCUGUUAAA |
| 1224 | HPV52_1670 | GCUUAUACUGCUGCUAAUUAGGUUU |
| 1225 | HPV52_1585 | CAUCAGUUGCAGAAGGAUUAAAAGU |
| 1226 | HPV52_1560 | UGUAUUAUAGGAAUGGGAGUAACAC |
| 1227 | HPV52_1387 | GUAUAGAGGACAAUGAGGAAAAUAG |
| 1228 | HPV52_1330 | GUAACAGUAGUCAAUCAAGUGGGGU |
| 1229 | HPV52_1237 | CAUGUCACGUAGAAGACAGCGGCUA |
| 1230 | HPV52_1207 | AUACAGAGUGUGUUUUACCAAAACG |
| 1231 | HPV52_1143 | GAAAGUGCUGGGCAAGAUGGUGUAG |
| 1232 | HPV52_1099 | UACAUGCUGUGUCUGCAGUAAAACG |
| 1233 | HPV52_1035 | AAUGAACAGGCAGAACAUGAGGCAG |
| 1234 | HPV52_981 | GCAUAUGAUAGUGGAACAGAUCUAA |
| 1235 | HPV52_899 | GGGAUGUACAGGCUGGUUUGAAGUA |
| 1236 | HPV52_853 | ACAACCCUGCAAUGGAGGACCCUGA |
| 1237 | HPV52_781 | GACCUUCGUACUCUACAGCAAAUGC |
| 1238 | HPV52_746 | GCACACUACGGCUAUGCAUUCAUAG |
| 1239 | HPV52_590 | UAGAUCUGCAACCUGAAACAACUGA |
| 1240 | HPV52_557 | GUGGAGACAAAGCAACUAUAAAAGA |
| 1241 | HPV52_532 | CUGUGACCCAAGUGUAACGUCAUGC |
| 1242 | HPV52_483 | AUUAUGGGUCGUUGGACAGGGCGCU |
| 1243 | HPV52_453 | CAUGUUAAUGCAAACAAGCGAUUUC |
| 1244 | HPV52_417 | UGUCAAACGCCAUUAUGUCCUGAAG |
| 1245 | HPV52_352 | AUGGGAAAACAUUAGAAGAGAGGGU |
| 1246 | HPV52_280 | AUGGCGUGUGUAUUAUGUGCCUACG |
| 1247 | HPV52_216 | CGAAGAGAGGUAUACAAGUUUCUAU |
| 1248 | HPV52_170 | GCAUGAAAUAAGGCUGCAGUGUGUG |
| 1249 | HPV52_145 | UGUGUGAGGUGCUGGAAGAAUCGGU |
| 1250 | HPV52_120 | ACACGACCCCGGACCCUGCACGAAU |
| 1251 | HPV52_95 | CACGGCCAUGUUUGAGGAUCCAGCA |
| 1252 | HPV52_70 | UAUAUAGAACACAGUGUAGCUAACG |

Also described is an isolated polynucleotide for specific hybridization to HPV 56 consisting essentially of a sequence or a complement thereof selected from the group consisting of SEQ ID NOs: 1253-1367 (See Table 10). Also described is a set of polynucleotides for specific hybridization to HPV 56, wherein the set comprises at least one polynucleotide consisting essentially of a sequence or a complement thereof selected from the group consisting of: SEQ ID NOs: 1253-1367. In certain embodiments, the methods of the present invention utilize a set of polynucleotide probes for specific hybridization to HPV 56 comprising SEQ ID NOs: 1253-1367.

**Table 10: Polyribonucleotide probes for determining HPV 56 nucleic acid.**

| SEQ ID NO: | Name | Sequence |
|---|---|---|
| 1253 | HPV56_7754 | GUCAGUAUCUGUUUUGCAAACAUGU |
| 1254 | HPV56_7729 | AAUACACUAUGUAGGCCAAGUAUCU |
| 1255 | HPV56_7697 | UGUGUCUGCAACUUUGGUGUUUUGG |
| 1256 | HPV56_7605 | GUACCGCACCCUGUAUUACUCACAG |
| 1257 | HPV56_7532 | GGCCCUUUUCAGCAGAACAGUUAAU |
| 1258 | HPV56 7506 | GCCUAGUGCCAUUAUUUAAACCAAA |
| 1259 | HPV56_7431 | CAUUUUGUACAUGCAACCGAAUUCG |
| 1260 | HPV56_7366 | GUGUACUAUGUGUAUUGUGCAUACA |
| 1261 | HPV56_7322 | GUGUGUCAUUAUUGUGGCUUUUGUU |
| 1262 | HPV56_7271 | GUCUGUAAUAAACAUGAAUGAGUGC |
| 1263 | HPV56_7111 | UUUGUGUAACUGUGUUUGUGUGUUG |
| 1264 | HPV56_7083 | GUAAAAGGCGGUAGUGUGUUGUUGU |
| 1265 | HPV56_7058 | ACCUCCACCUCUACACCAGCAAAAC |
| 1266 | HPV56_7015 | GUCAAAGCCUGCUGUAGCUACCUCU |
| 1267 | HPV56_6872 | UGUCAACGGGAACAGCCACCAACAG |
| 1268 | HPV56_6823 | CACCAGCCUAGAAGAUAAAUAUAGA |
| 1269 | HPV56_6767 | AAUAUGAAUGCUAACCUACUGGAGG |
| 1270 | HPV56 6727 | CAAAAUUACUUUGUCUGCAGAGGUU |
| 1271 | HPV56_6612 | CUAACAUGACUAUUAGUACUGCUAC |
| 1272 | HPV56_6489 | UGAUUACGUCUGAGGCACAGUUAUU |
| 1273 | HPV56_6421 | UUUAAAGGGUAGCAAUGGUAGAGAA |
| 1274 | HPV56_6393 | UUGGGGAAACAAUACCUGCAGAGUU |
| 1275 | HPV56_6253 | ACCUUUAGACAUUGUACAAUCCACC |
| 1276 | HPV56_6212 | GCUAUGGACUUUAAGGUGUUGCAGG |
| 1277 | HPV56_6151 | GCCUCUUGCAUUAAUUAAUACACCU |
| 1278 | HPV56_6119 | AAGUCCACACAAGUUACCACAGGGG |
| 1279 | HPV56_6094 | ACAUUGGACUAAAGGUGCUGUGUGU |
| 1280 | HPV56_6031 | UAUAUCAGUUGAUGGCAAGCAAACA |
| 1281 | HPV56_5860 | UAUUUAUAAUCCGGACCAGGAACGG |
| 1282 | HPV56_5776 | CAUUCCCAAAGUUAGUGCAUAUCAA |
| 1283 | HPV56_5750 | GUGACUAAGGACAAUACCAAAACAA |
| 1284 | HPV56_5524 | UCCUCCUUUGCAUUAUGGCCUGUGU |
| 1285 | HPV56_5471 | CCUUUGUUCCUCAGUCUCCUUAUGA |
| 1286 | HPV56_5419 | CCAUUUUAUUCAGGUCCUGACAUAG |
| 1287 | HPV56_5394 | CCCUUUAGGUAAUGUGUGGGAAACA |
| 1288 | HPV56_5369 | CUAGUAACACCACUAAUGUAACUGC |
| 1289 | HPV56_5334 | ACACUUACCUAUAAAGCCUUCCACA |
| 1290 | HPV56_5306 | CUAGCCAGUCAGUUGCUACACCUUC |
| 1291 | HPV56_5131 | ACUAUACAAACACGUAGAGGCACAC |
| 1292 | HPV56_4953 | ACCUGCAACAUUAGUAUCUGCUGAU |
| 1293 | HPV56_4885 | GCAGCUCCUAGAUUAUAUAGAAAAG |
| 1294 | HPV56_4818 | AUUUGCUGUUCACGGUUCUGGUACA |
| 1295 | HPV56_4754 | GCAAUAUUUUAAUUAGCACACCCAC |
| 1296 | HPV56_4682 | GUACCCAUAUAACCAAUCCGUUAUU |
| 1297 | HPV56_4657 | ACCUCUAGUACUGUACAUGUCAGUA |
| 1298 | HPV56_4572 | AGGGAUUCCUAAUUUUACUGGGUCU |
| 1299 | HPV56_4546 | GAGUCCAGUGUUAUAGAAUCUGGUG |
| 1300 | HPV56_4474 | ACUCCGGCGCGACCACCUAUUGUUG |
| 1301 | HPV56_4429 | GGCUAUGUUCCAUUGGGGUCUAGGC |
| 1302 | HPV56_4206 | UAGUACUGUUACUACUAUGGUUGCC |
| 1303 | HPV56_4150 | CUGUGCUGUGUAUAUAUUUACAUGC |
| 1304 | HPV56_4082 | GUUUUGGUUUGUUAUAGCCACAUCC |
| 1305 | HPV56_4045 | CCUCUGUGUUUUCCAGUUGUAUAUU |
| 1306 | HPV56_4018 | GUCAUGUUGUCCCGCUUUUGCUAUC |
| 1307 | HPV56_3993 | UGCUUUUGUGUUUGUUUGCUUGUGU |
| 1308 | HPV56_3937 | UGCUACGCAUAUAUAUUGCAACCAU |
| 1309 | HPV56_3912 | GUGAAGUGUACCUGCCAUACAUUGC |
| 1310 | HPV56_3844 | CAAAUGAGUUUUCCAUAAAGUGCUG |
| 1311 | HPV56_3819 | CAGUAGUGUACAGGUUAGUUUGGGA |
| 1312 | HPV56_3717 | CAUAUCAUUGGACAAGUACAGACAA |
| 1313 | HPV56_3571 | CAGUAGAAGUAGAAGUAUCAACAAC |
| 1314 | HPV56_3546 | ACAUCAGCGACACAGACAAUACCGA |
| 1315 | HPV56_3488 | GAAUCAGAAUUUGACUCCUCCAGAG |
| 1316 | HPV56_3463 | ACCAGGAAAACGACCCAGACUACGG |
| 1317 | HPV56_3438 | ACCAAGACGCCGCAGUAUCCCACAG |
| 1318 | HPV56_3390 | AAUACAACACCCACAAGACCACCAC |
| 1319 | HPV56_3247 | CUACACAGACUUUGAACAAGAGGCC |
| 1320 | HPV56_3197 | GGGGUAGACUAUAGAGGUAUAUAUU |
| 1321 | HPV56_3129 | GUAUGCAAUAUGUAGCCUGGAAAUA |
| 1322 | HPV56_3024 | CAUUAAGAGACACAUGCGAGGAACU |
| 1323 | HPV56_2978 | GCACUGGAAUCAUUAAGUACAACAA |
| 1324 | HPV56_2896 | CAUUACUGUACUAAACCACCAGAUG |
| 1325 | HPV56_2738 | AGAAAACAAUGGAGACGCUUUCCCA |
| 1326 | HPV56_2683 | AAUGUUUCUUUACAAGGACGUGGUC |
| 1327 | HPV56_2562 | CCUAUGCUAGAUGCUAAAUUACGAU |
| 1328 | HPV56_2530 | GUCCACCAUUACUAAUUACAACCAA |
| 1329 | HPV56_2398 | AUGCUAAACUUGGGUUGUUGGAUGA |
| 1330 | HPV56_2269 | GUUUGGUACUUUGUGGACCGCCAAA |
| 1331 | HPV56_2124 | CAGUGGAUAAAGCACAUAUGUAGUA |
| 1332 | HPV56_1957 | AAGUAACAGAUGAUAGCCAAAUUGC |
| 1333 | HPV56_1896 | CACAGUUUACAGGAUAGUCAAUUUG |
| 1334 | HPV56_1837 | AUAUUAGUGAUGUGUAUGGAGACAC |
| 1335 | HPV56_1756 | CACAGGAGCAAAUGUUAAUUCAACC |
| 1336 | HPV56_1436 | GCAGGACUUGUUUAAAAGUAGCAAU |
| 1337 | HPV56_1411 | ACAAUGAAACGCCAACACAACAAUU |
| 1338 | HPV56_1377 | GAGGACUCUGUAAUACAUAUGGAUA |
| 1339 | HPV56_1346 | CUCACAAAACAGUACCUAUAGUAAC |
| 1340 | HPV56_1321 | GGUGCGGGAAUACACAAAAUGGAGG |
| 1341 | HPV56_1296 | GUAGAUGAAGAGGUACAGGGACGUG |
| 1342 | HPV56_1265 | UACAUUGGAAACUCUGGAAACACCA |
| 1343 | HPV56_1231 | UUUUAUCAGACCUACAAGACAGCGG |
| 1344 | HPV56_1170 | CCAUUAAGGGAUAUUAGUAAUCAGC |
| 1345 | HPV56_1108 | UACAAACAGCACAUGCAGAUAAACA |
| 1346 | HPV56_1078 | GACGCAGAAACAGUCAACAAUUGUU |
| 1347 | HPV56_993 | AGAUGAUGAAAGUGACGAGGAGGAU |
| 1348 | HPV56_943 | UGGUUUGAAGUAGAGGCAAUUGUAG |
| 1349 | HPV56_874 | CGCAUCAAGUAACUAACUGCAAUGG |
| 1350 | HPV56_807 | CCAAAGAGGACCUGCGUGUUGUACA |
| 1351 | HPV56_778 | GUUUGUGGUGCAGUUGGACAUUCAG |
| 1352 | HPV56_751 | AAUACACGUACCUUGUUGUGAGUGU |
| 1353 | HPV56_722 | AGACAAGCUAAACAACAUACGUGUU |
| 1354 | HPV56_619 | ACCUCAAACAGAAAUUGACCUACAG |
| 1355 | HPV56_594 | UGCAAGACGUUGUAUUAGAACUAAC |
| 1356 | HPV56_529 | GGAGACAAACAUCUAGAGAACCUAG |
| 1357 | HPV56_504 | UGGACCGGGUCAUGUUUGGGGUGCU |
| 1358 | HPV56_479 | ACGAUUUCAUCUAAUAGCACAUGGU |
| 1359 | HPV56_423 | AGAUGUCAAAGUCCGUUAACUCCGG |
| 1360 | HPV56_362 | UGGAGCUACACUAGAAAGUAUAACU |
| 1361 | HPV56_292 | CAGUGUGCAGAGUAUGUUUAUUGUU |
| 1362 | HPV56_267 | GUGUAUAGGGAUGAUUUUCCUUAUG |
| 1363 | HPV56_222 | ACACGUGCUGAGGUAUAUAAUUUUG |
| 1364 | HPV56_150 | CACUUGAGUGAGGUAUUAGAAAUAC |
| 1365 | HPV56_115 | UCAACAAUCCACAGGAACGUCCACG |
| 1366 | HPV56_77 | CAGCUUAUUCUGUGUGGACAUAUCC |
| 1367 | HPV56_15 | UACUUUUAUAUAUUGGGAGUGACCG |

Also described is an isolated polynucleotide for specific hybridization to HPV 58 consisting essentially of a sequence or a complement thereof selected from the group consisting of SEQ ID NOs: 1368-1497 (See Table 11). Also described is a set of polynucleotides for specific hybridization to HPV 58, wherein the set comprises at least one polynucleotide consisting essentially of a sequence or a complement thereof selected from the group consisting of: SEQ ID NOs: 1368-1497. In certain embodiments, the methods of the present invention utilize a set of polynucleotide probes for specific hybridization to HPV 58 comprising SEQ ID NOs: 1368-1497.

**Table 11: Polyribonucleotide probes for determining HPV 58 nucleic acid.**

| SEQ ID NO: | Name | Sequence |
|---|---|---|
| 1368 | HPV58_7715 | GUUUGUUAUGCCAAACUAUGUCUUG |
| 1369 | HPV58_7678 | CUUUCAAUGCUUAAGUGCAGUUUUG |
| 1370 | HPV58_7596 | UCAUAUAUACAUGCAGUGCAGUUGC |
| 1371 | HPV58_7571 | UUUUGCCUAUACUUGCAUAUGUGAC |
| 1372 | HPV58_7546 | UUAAUCCUUUCCCUUCCUGCACUGC |
| 1373 | HPV58_7472 | CAUUUUGUGCAUGUAACCGAUUUCG |
| 1374 | HPV58_7444 | CAGUACUGCCUCCAUUUUACUUUAC |
| 1375 | HPV58_7384 | CUGCCUAUUAUGCAUACCUAUGUAA |
| 1376 | HPV58_7359 | UGUCCCUAAAUUGCCCUACCCUGCC |
| 1377 | HPV58_7334 | UUGGGUGUAUCUAUGAGUAAGGUGC |
| 1378 | HPV58_7266 | CUUGUCAGUUUCCUGUUUCUGUAUA |
| 1379 | HPV58_7232 | GUUAUGUGUCAUGUUUGUGUACAUG |
| 1380 | HPV58_7097 | UACCCGUGCACCAUCCACCAAACGC |
| 1381 | HPV58_7070 | GCCCAGACUAAAACGUUCGGCCCCU |
| 1382 | HPV58_6784 | CACUAACUGCAGAGAUAAUGACAUA |
| 1383 | HPV58_6722 | GGAAUAUGUACGUCAUGUUGAAGAA |
| 1384 | HPV58_6676 | GCACUGAAGUAACUAAGGAAGGUAC |
| 1385 | HPV58_6625 | AGUUAUUUGUUACCGUGGUUGAUAC |
| 1386 | HPV58_6533 | CUCUAUAGUUACCUCAGAAUCACAA |
| 1387 | HPV58_6488 | UACUGCAGUUAUCCAAAGUAGUGCA |
| 1388 | HPV58_6453 | GUCCCGGAUGACCUUUAUAUUAAAG |
| 1389 | HPV58_6428 | UAGGGCUGGAAAACUUGGCGAGGCU |
| 1390 | HPV58_6395 | ACGUGAGCAGAUGUUUGUUAGACAC |
| 1391 | HPV58_6177 | AAUGCAGCUGCUACUGAUUGUCCUC |
| 1392 | HPV58_6055 | CACAGCCAGGGUCUGAUAACAGGGA |
| 1393 | HPV58_6030 | ACUGAAACCAGUAACAGAUAUCCCG |
| 1394 | HPV58_5840 | AUCAGGCUUACAGUAUAGGGUCUUU |
| 1395 | HPV58_5590 | UAGCUAUUUUAUUUUGCGUCGCAGA |
| 1396 | HPV58_5562 | UGGAUGGUGCUGAUUUUAUGUUGCA |
| 1397 | HPV58_5532 | CUCCACUAACUCCUUUUAAUACCAU |
| 1398 | HPV58_5502 | CAUCUAUGUCUAGUCCAUUUAUUCC |
| 1399 | HPV58_5477 | GGUCCAGACAUUGCAUCUUCUGUAA |
| 1400 | HPV58_5452 | CACUCCUCUUGUGUCAUUGGAACCU |
| 1401 | HPV58_5423 | GUGUCCAUACCAUUAAAUACUGGAU |
| 1402 | HPV58_5398 | CUUUGCCACCACACGUACCAGUAAU |
| 1403 | HPV58_5373 | AGAGUCCUCUGCACUCACAUACGUC |
| 1404 | HPV58_5345 | GACGAUGCUGAUACUAUACAUGAUU |
| 1405 | HPV58_5304 | CUCCCUAUAGUAUUAAUGAUGGACU |
| 1406 | HPV58_5258 | CAACAGCAGCAACAAUUUGAAUUAC |
| 1407 | HPV58_5225 | UUAAGUCCCAUACAGCCUGUCCAGG |
| 1408 | HPV58_5200 | GGCUAAAGUACAUUACUACCAAGAC |
| 1409 | HPV58_5161 | AAAGGCUACACUUCGUACUCGCAGU |
| 1410 | HPV58_5050 | ACAUAGUGACAUAUCGCCUGCUCCU |
| 1411 | HPV58_5025 | ACCCUGAGGACACAUUGCAGUUUCA |
| 1412 | HPV58_4977 | CUCCUCAUAGACUUGUAACAUAUGA |
| 1413 | HPV58_4929 | GUCGCAACACCCAACAAGUUAAGGU |
| 1414 | HPV58_4867 | CAAUGUCACGUCUAGCACACCCAUU |
| 1415 | HPV58_4842 | CCUUUGUUAUUUCUACUGACAGUGG |
| 1416 | HPV58_4809 | GCACACAUAGUUAUGAAAACAUACC |
| 1417 | HPV58_4776 | CUGGACAUUUAAUAUUUUCCUCUCC |
| 1418 | HPV58_4741 | AUCCGUACUCCGCCCUCCUGCACCU |
| 1419 | HPV58_4716 | AUUUAAAUCCCUCCUUUACUGAGCC |
| 1420 | HPV58_4658 | CCUGCAAUACUUAAUGUUUCCUCUA |
| 1421 | HPV58_4633 | UAUUACCACCUCUGCAGAUACUACA |
| 1422 | HPV58_4608 | CAAUUCCCACUCCAUCUGGUUUUGA |
| 1423 | HPV58_4583 | AUAGACGCCGGUGCACCAGCCCCAU |
| 1424 | HPV58_4470 | GUACCCCACCGUCUGAGGCUAUACC |
| 1425 | HPV58_4375 | AUUACGAUAUGGUAGCUUAGGGGUG |
| 1426 | HPV58_4278 | CAUCUGCUACACAACUUUACCAAAC |
| 1427 | HPV58_4139 | CACAUGGUGGUAUGGUAUUGUAAAU |
| 1428 | HPV58_4114 | CAAGACUAACUGUAUACUGGUUCUG |
| 1429 | HPV58_4015 | GUGUCUGUGGGGUCGGCUCUACGAA |
| 1430 | HPV58_3990 | GCUGGUGUUGGUGUUGCUGCUUUGG |
| 1431 | HPV58_3954 | GCCAUUGGUGCUAUCUAUUUCUAUA |
| 1432 | HPV58_3845 | ACUGUAUGUAAACCACAAGCCAAUA |
| 1433 | HPV58_3799 | GCAAAUAAGUACUGGUGUUAUGUCA |
| 1434 | HPV58_3737 | ACAUACACAACGGAAACACAACGAC |
| 1435 | HPV58_3711 | GUGACAAAGUAGGAAUUGUUACUGU |
| 1436 | HPV58_3579 | CUAAAGUUUCACCUAUCGUGCAUUU |
| 1437 | HPV58_3544 | UAACUGUACAUACAAAGGGCGGAAC |
| 1438 | HPV58_3487 | GUAUACAGACUGCGCCGUGGACAGU |
| 1439 | HPV58_3462 | GAGACAACACCCAGUACUCCACAAA |
| 1440 | HPV58_3437 | CGACGACUCGAUUUACCAGACUCCA |
| 1441 | HPV58_3412 | CGAAAGUACACAGGGGACAAAGCGA |
| 1442 | HPV58_350 | CCUAGUGAUCAAAUAUCCACUACUG |
| 1443 | HPV58_3288 | CUAAAACACAAUUAUGGGAGGUACA |
| 1444 | HPV58_3209 | GACUAUGUGGGGUUGUAUUAUAUAC |
| 1445 | HPV58_3184 | AUGUACUUUGGUAGCAGGAGAAGUU |
| 1446 | HPV58_3116 | GACAAUGAUAAAGCAAACACAAUGG |
| 1447 | HPV58_3046 | CUUAGAAGUGUGGUUAUCAGAGCCA |
| 1448 | HPV58_2985 | CAUUAGAGACAUUAAAUGCAUCACC |
| 1449 | HPV58_2943 | CAUCAAAGACUAAAGCGUUUCAAGU |
| 1450 | HPV58_2898 | UGGGAAUAUCACAUUUGUGCCACCA |
| 1451 | HPV58_2873 | GCUAUAAUGUAUACAGCCAGACAAA |
| 1452 | HPV58_2842 | UGAACAUUGGAAACUAAUACGCAUG |
| 1453 | HPV58_2794 | AAUCCUAGACAUAUACGAAGCUGAU |
| 1454 | HPV58_2717 | AAUUAGGCUUAAUAGAGGAAGAGGA |
| 1455 | HPV58_2598 | GCACAGUAGACUAACAGUAUUUGAA |
| 1456 | HPV58_2573 | GCAAAGAUUCACGAUGGCCAUAUUU |
| 1457 | HPV58_2516 | GGGCAUUAGUACAAUUAAAAUGUCC |
| 1458 | HPV58_2482 | GAUGGUAACGACAUUUCAAUAGAUG |
| 1459 | HPV58_2404 | GAUGCUAAACUAGGUAUGAUAGAUG |
| 1460 | HPV58_2278 | AUGUUACUGUGUGGCCCAGCAAAUA |
| 1461 | HPV58_2109 | AAAGCGUGGUAUGACAAUGGGACAA |
| 1462 | HPV58_1885 | AGAUUAACAGUGUUACAGCAUAGCU |
| 1463 | HPV588_1852 | GAUGUGCAAGGGACAACACCAGAAU |
| 1464 | HPV58_1800 | AAGUCAAGCAUGUGCCUUAUAUUGG |
| 1465 | HPV58_1770 | AUGUAUGAUUAUCGAGCCACCAAAA |
| 1466 | HPV58_1643 | AUACACACCUACAAUGUUUAACGUG |
| 1467 | HPV58 1590 | AAGUCCCUCCGUAGCAGAAAGUUUA |
| 1468 | HPV58_1565 | AUUGGUGUAUAACAGGGUAUGGAAU |
| 1469 | HPV58_1498 | GAAGCUUAUGGAGUAAGUUUUAUGG |
| 1470 | HPV58_1456 | CAUAACAGUAAUACUAAAGCAACGC |
| 1471 | HPV58_1402 | ACGGAUGUAGACAGUUGUAAUACUG |
| 1472 | HPV58_1349 | UAAAUGACUCGGAGUCUAGUGGGGU |
| 1473 | HPV58_1313 | CACACCAGGUAGAAAGCCAAAAUGG |
| 1474 | HPV58_1196 | CAAAUGUGUGUGUAUCGUGGAAAUA |
| 1475 | HPV58_1108 | GUGGACGAUAUAAAUGCUGUGUGUG |
| 1476 | HPV58_1083 | AGCGUUGUUUAAUGUACAGGAAGGG |
| 1477 | HPV58_1005 | CGAUAGUGGUACAGAUUUAAUAGAG |
| 1478 | HPV58_958 | CGAAGAACAGGAGAUAAUAUUUCAG |
| 1479 | HPV58_933 | GUUUGAGGUAGAAGCGGUAAUAGAA |
| 1480 | HPV58_837 | UACCAUUGUGUGCCCUAGCUGUGCA |
| 1481 | HPV58_799 | GACGUACGAACCCUACAGCAGCUGC |
| 1482 | HPV58_774 | UUUGUGUAUCAACAGUACAACAACC |
| 1483 | HPV58_749 | GUUACACUUGUGGCACCACGGUUCG |
| 1484 | HPV58_719 | CCACAGCUAAUUACUACAUUGUAAC |
| 1485 | HPV58_667 | UCAGACGAGGAUGAAAUAGGCUUGG |
| 1486 | HPV58_620 | AUCCUGAACCAACUGACCUAUUCUG |
| 1487 | HPV58_585 | CAACCCAACGCUAAGAGAAUAUAUU |
| 1488 | HPV58_560 | CCUGUAACAACGCCAUGAGAGGAAA |
| 1489 | HPV58_533 | CCCCGACGUAGACAAACACAAGUGU |
| 1490 | HPV58_481 | GUUUCAUAAUAUUUCGGGUCGUUGG |
| 1491 | HPV58_360 | AUGGAGACACAUUAGAACAAACACU |
| 1492 | HPV58_302 | GUGUGCUUACGAUUGCUAUCUAAAA |
| 1493 | HPV58_261 | GAAUAGUGUAUAGAGAUGGAAAUCC |
| 1494 | HPV58_184 | AAUCGAAUUGAAAUGCGUUGAAUGC |
| 1495 | HPV58_159 | AGGCGUUGGAGACAUCUGUGCAUGA |
| 1496 | HPV58_134 | CCACGGACAUUGCAUGAUUUGUGUC |
| 1497 | HPV58_104 | AGGACUAUGUUCCAGGACGCAGAGG |

Also described is an isolated polynucleotide for specific hybridization to HPV 59 consisting essentially of a sequence or a complement thereof selected from the group consisting of SEQ ID NOs: 1498-1646 (See Table 12). Also described is a set of polynucleotides for specific hybridization to HPV 59, wherein the set comprises at least one polynucleotide consisting essentially of a sequence or a complement thereof selected from the group consisting of: SEQ ID NOs: 1498-1646. In certain embodiments, the methods of the present invention utilize a set of polynucleotide probes for specific hybridization to HPV 59 comprising SEQ ID NOs: 1498-1646.

**Table 12: Polyribonucleotide probes for determining HPV 59 nucleic acid.**

| SEQ ID NO: | Name | Sequence |
|---|---|---|
| 1498 | HPV59_7826 | CAAGUACAUGCACACUUUCUACUUA |
| 1499 | HPV59_7735 | ACUACUGUGCAAUCCAAGAAUGUGU |
| 1500 | HPV59_7657 | CGCCCUUGUUAAUAAAACAGCUUUU |
| 1501 | HPV59_7632 | AACAAUACUUGCAUAACUUUGGUGG |
| 1502 | HPV59_7592 | ACGCCAAAUAGUUAGUCAUCAUCCU |
| 1503 | HPV59_7567 | CCUAGACUACUAACACAACUUACAA |
| 1504 | HPV59_7542 | UCCCCAUCUUGUUUCCUCCUACACG |
| 1505 | HPV59_7474 | UCGGUUACCUUGGUUUAACCUUACC |
| 1506 | HPV59_7429 | GUCCAUUUUAUCCUUUAAAUCCUCC |
| 1507 | HPV59_7392 | CCUGAAUGUCCAGUUUUGCAUUUGC |
| 1508 | HPV59_7367 | AGGUGUGUUUGUUCCUUCAUUUUGU |
| 1509 | HPV59_7340 | CAUUAUUACACAUUGCCCUACUUAC |
| 1510 | HPV59_7309 | GUCCCUUUAUUGUUUCUUUGUCCUU |
| 1511 | HPV59_7218 | GUUUGUCUGCUGUAUGUGUGUAUUU |
| 1512 | HPV59_7152 | GUAUGUGUGCAUGUUGUAUGUUUUG |
| 1513 | HPV59_7117 | GUCUUCCAGAAAAUAGUGUUGUUUG |
| 1514 | HPV59_7086 | CCCCAUCACCAAAACGUGUUAAGCG |
| 1515 | HPV59_7027 | AGCUAGACCUAAGCCCACUAUAGGC |
| 1516 | HPV59_6965 | GAAAGGUUUUCUGCAGAUCUUGAUC |
| 1517 | HPV59_6940 | AAAGUUUUGGCCUGUAGAUCUUAAG |
| 1518 | HPV59_6915 | UUAAACAGGACCCUUAUGACAAACU |
| 1519 | HPV59_6877 | UGCUGCUGUAACUUGUCAAAAGGAC |
| 1520 | HPV59_6852 | UUGACACAUACCGUUUUGUUCAAUC |
| 1521 | HPV59_6688 | UAAAGAAUAUGCCAGACAUGUGGAG |
| 1522 | HPV59_6663 | CUAAUGUAUACACACCUACCAGUUU |
| 1523 | HPV59_6638 | UGUGCUUCUACUACUUCUUCUAUUC |
| 1524 | HPV59_6463 | AGGCAGUUAUUUAUAUUCCCCUUCC |
| 1525 | HPV59_6408 | GUGAUCAACUUCCUGAAUCACUAUA |
| 1526 | HPV59_6248 | GAUAACAAAAGUGAAGUACCAUUGG |
| 1527 | HPV59_6223 | GGCUAUGGACUUUAAAUUGUUGCAG |
| 1528 | HPV59_6136 | UACUACUGUGGUUCAGGGCGAUUGU |
| 1529 | HPV59_6020 | GAUACCAAAGAUACACGUGAUAAUG |
| 1530 | HPV59_5991 | CUGAAAACUCUCAUGUAGCAUCUGC |
| 1531 | HPV59_5912 | GUAGGUGUUGAAAUCGGUCGGGGCC |
| 1532 | HPV59_5882 | CCUAACUCUCAACGCUUGGUCUGGG |
| 1533 | HPV59_5857 | CCUUCCAGAUAACACAGUAUAUGAU |
| 1534 | HPV59_5798 | GUGUCUGCAUAUCAAUACAGAGUAU |
| 1535 | HPV59_5765 | AAAGGUGGUAAUGGUAGACAGGAUG |
| 1536 | HPV59_5701 | UAUUUUCUACCACGCAGGCAGUUCC |
| 1537 | HPV59_5676 | CUGAUGAGUAUGUCACCCGUACCAG |
| 1538 | HPV59_5642 | CUACCUCCACCUUCGGUAGCUAAGG |
| 1539 | HPV59_5498 | CCUUUACCACCAUACAGUCUAUUAA |
| 1540 | HPV59_5473 | GUUGAACCCACUUAUUCUACUACAC |
| 1541 | HPV59_5441 | GACCCGAUAUAGUUUUACCUAAUAC |
| 1542 | HPV59_5416 | GCCUGGGAUGUUCCUGUAAAUACAG |
| 1543 | HPV59_5382 | CACCUUUUCAAAUGUAACUGUUCCU |
| 1544 | HPV59_5357 | UGUCAUUAACACGGUCGGCAUCUAG |
| 1545 | HPV59_5315 | CCAACACUGCAUUUACAAUUCCUAA |
| 1546 | HPV59_5290 | ACAGAUGAAGCACCUACUAGUACUG |
| 1547 | HPV59_5250 | GGCUGCUACUGAUGAUAUAUAUGAU |
| 1548 | HPV59_5225 | AAUUGCAACCUCUUGUUUCUUCCCA |
| 1549 | HPV59_5200 | CCUAUACCACAUGCUGAAGAUAUUG |
| 1550 | HPV59_5088 | AACAUCCAGACGCAGCACUGUAAGG |
| 1551 | HPV59_5046 | CCCGGACUUUAUGGAUAUAGUUCGU |
| 1552 | HPV59_5013 | AUUAACUUUUGACCCCUCAUCAGAG |
| 1553 | HPV59_4988 | CUGCUUAUGAUCCAAUUGAUACUAC |
| 1554 | HPV59_4958 | GUCCAUCCACAUUUGUUACAUAUGA |
| 1555 | HPV59_4923 | ACAAGUUCGGGUGUCUAACGCUGAC |
| 1556 | HPV59_4896 | ACCUAGAUUGUACAGUAGGGCUAAU |
| 1557 | HPV59_4871 | AUCCAACAGUACGUCGUGUGGCUGG |
| 1558 | HPV59_4740 | CCAAACAGGUGAAAUUUCUGGUAAU |
| 1559 | HPV59_4685 | GUAGCUCUAGUUUUAUAAAUCCUGC |
| 1560 | HPV59_4660 | ACCCCAACCUCUUCUGUUCAAAUUA |
| 1561 | HPV59_4607 | CAGGAUUUGAAAUAUCUACCUCUAG |
| 1562 | HPV59_4555 | GAUUCUAGUGUUAUAACAUCUGGAG |
| 1563 | HPV59_4522 | CCUACAGAUCCAUCUAUAGUUACAU |
| 1564 | HPV59_4495 | CCACCAGUAGUUAUUGAACCUGUUG |
| 1565 | HPV59_4470 | UAUAGUAGAUGUAUCGCCUGCUAAA |
| 1566 | HPV59_4362 | AUUGCAGUGGACCAGCCUAGGAAUA |
| 1567 | HPV59_4238 | CCCAUCGUGCUGCUCGUCGUAAACG |
| 1568 | HPV59_4109 | GCAAUACUGUCCAUACAAUAAUUGC |
| 1569 | HPV59_4084 | UCCACUGUUACUACUAUAUGCCCAU |
| 1570 | HPV59_4029 | UGGUUAUCACCUCCUCAUAUGAGUG |
| 1571 | HPV59_3991 | GUGUGCAUAUACAUGGUUACUAGUA |
| 1572 | HPV59_3966 | UCCCGCUUCUGCAAUCUGUCUAUAU |
| 1573 | HPV59_3913 | AACCCUUGUAUUUGUGUGUUGUGUU |
| 1574 | HPV59_3858 | UGCAAAUGUAACACAAGCCAAUACU |
| 1575 | HPV59_3832 | GGUAUAUGAGUGUGUAAUGGUUGUU |
| 1576 | HPV59_3754 | UAACAUAUACAAGCGAAACACAACG |
| 1577 | HPV59_3718 | GAAACAGAGGAUCAGCCAAAACAGG |
| 1578 | HPV59_3686 | UGAAAAUAUUUCCUCUACCUGGCAU |
| 1579 | HPV59_3589 | UCCCUUGCAGUAACACUACGCCUAU |
| 1580 | HPV59_3562 | AUCCAGGCAACAACCCGCGACGGCA |
| 1581 | HPV59_3537 | UGUGACAACCCAGUCGUCCGUUUGC |
| 1582 | HPV59_3512 | GUCUACCAGCGUGUCAGUGGACUAC |
| 1583 | HPV59_3474 | AAGCGACCAAGACAGUGUGGAUACA |
| 1584 | HPV59_3392 | GCAACUAUCAUACCCCUCCGCAACG |
| 1585 | HPV59_3354 | ACCAGUGACGAGCAAGUAUCCACUG |
| 1586 | HPV59_3319 | GCAAGGUUAUUGAUUGUUAUGACUC |
| 1587 | HPV59_3291 | ACAGACAAGUGGGAAGUGCAUUAUA |
| 1588 | HPV59_3237 | GAGGAACAGGUGUACUAUGUAAAAU |
| 1589 | HPV59_3204 | GUGGACUUUUGGGGACUAUAUUAUA |
| 1590 | HPV59_3170 | UGAUGUAGGACAGUGGUGUAAAACC |
| 1591 | HPV59_3134 | GCAUUACACAAGCUGGACAUUUAUA |
| 1592 | HPV59_3109 | CCAUCUGCAGCAAGGAAAACACAAU |
| 1593 | HPV59_3050 | UGUUUCUUGCAUUGUCCAUUGCUCA |
| 1594 | HPV59_3023 | AGGUGCUGUUUGCCAUAGUUCUUGG |
| 1595 | HPV59_2980 | ACCGUACUUCCACUGUAAUGCCCUG |
| 1596 | HPV59_2948 | CAAGGCAUGUGAAGCUAUUGAACUG |
| 1597 | HPV59_2881 | CAGCAAGAGAGAACAAUAUACAUAC |
| 1598 | HPV59_2757 | CUUUCGCAGCGUUUAAGUGUGUUAC |
| 1599 | HPV59_2732 | GAAGAUGCAGACAGUGAUGGACACC |
| 1600 | HPV59_2706 | GCAGAUUAGAUUUGAACGAGGAAGA |
| 1601 | HPV59_2577 | GGUGGCCAUAUUUAAAUAGCAGAUU |
| 1602 | HPV59_2508 | GGCACCUAGUACAAAUUAAAUGUCC |
| 1603 | HPV59_2450 | GAUACAUAUAUGCGAAAUGCUUUGG |
| 1604 | HPV59_2396 | GAUCGUAAAUUAGCUAUGCUAGACG |
| 1605 | HPV59_2371 | UCACUUUUGGCUAGAACCUUUAACA |
| 1606 | HPV59_2264 | AAUUGCAUUGUGCUGUGUGGGCCAG |
| 1607 | HPV59_2123 | CAGUGGAUAAAAUGGAGAUGUGAUA |
| 1608 | HPV59_2002 | AGAUAGUAAUAGUAACGCCGCUGCA |
| 1609 | HPV59_1909 | UAGCGUGUUUGACCUGUCAGAAAUG |
| 1610 | HPV59_1838 | AUUAGUGAAGUUAUAGGGGAAACGC |
| 1611 | HPV59_1754 | CCAGAUACGUGCAUGUUAAUUGAAC |
| 1612 | HPV59_1729 | AGGACUUAGCACAUUACUACAUGUA |
| 1613 | HPV59_1662 | CAUGGGGAGUAGUAAUAUUAGCAUU |
| 1614 | HPV59_1614 | UAAUACAACCCUAUGUGCUAUAUGC |
| 1615 | HPV59_1585 | UCCAACUGUAGCAGAAGGAUUUAAA |
| 1616 | HPV59_1374 | GUAGCGACAGCAGUAACAUGGAUGU |
| 1617 | HPV59_1348 | UGUUUGUAGCGACAGUCAAAUAGAC |
| 1618 | HPV59_1323 | CUGGAAAUGGGGAUAGCAAUGGCAG |
| 1619 | HPV59_1298 | GAGACUCAGGUAACCGUGGAGAAUA |
| 1620 | HPV59_1242 | GAAGGUUAAUAACAGUGCCAGACAG |
| 1621 | HPV59_1212 | CAGUAAAUGUUAACCACCCAAAAGU |
| 1622 | HPV59_1155 | ACAGUAGUGAGAAAGCGGCGGCAGG |
| 1623 | HPV59_1130 | CGAAAGUUUGGGUGCAGUAUAGAAA |
| 1624 | HPV59_1105 | UGCACGGGAAAUGCAUGUUUUAAAA |
| 1625 | HPV59_1073 | GCCUUGUUUAAUGUGCAGGAAGCCC |
| 1626 | HPV59_954 | CAGGUGACAAAAUUUCAGAUGACGA |
| 1627 | HPV59_814 | GUUUAUGGACACACUAUCCUUUGUG |
| 1628 | HPV59_773 | GUAGAAACCUCGCAAGACGGAUUGC |
| 1629 | HPV59_684 | AUCCUUUGCUACUAGCUAGACGAGC |
| 1630 | HPV59_632 | UUACCUGACUCCGACUCCGAGAAUG |
| 1631 | HPV59_605 | GAAGUUGACCUUGUGUGCUACGAGC |
| 1632 | HPV59_569 | GACAUUGUUUUAGAUUUGGAACCAC |
| 1633 | HPV59_541 | AAUGCAUGGACCAAAAGCAACACUU |
| 1634 | HPV59_499 | AGACAGCAACGACAAGCGCGUAGUG |
| 1635 | HPV59_459 | AGGACAGUGUCGUGGGUGUCGGACC |
| 1636 | HPV59_379 | CUAAAACCUCUAUGUCCAACAGAUA |
| 1637 | HPV59_354 | GCUGCUGAUACGCUGUUAUAGAUGC |
| 1638 | HPV59_329 | CUGAAACCAAGACACCGUUACAUGA |
| 1639 | HPV59_304 | UCCGUGUAUGGAGAAACAUUAGAGG |
| 1640 | HPV59_228 | CUGUACACCGUAUGCAGCGUGUCUG |
| 1641 | HPV59_169 | CUGCAAGAAAGAGAGGUAUUUGAAU |
| 1642 | HPV59_130 | CAUGAUAUUCGCAUCAAUUGUGUGU |
| 1643 | HPV59_105 | GAGCACAACAUUGAAUAUUCCUCUG |
| 1644 | HPV59_74 | CUACACAACGACCAUACAAACUGCC |
| 1645 | HPV59_49 | AACGGCAUGGCACGCUUUGAGGAUC |
| 1646 | HPV59_24 | UAAAGGUAGUUGAAAAGAAAAGGGC |

Also described is an isolated polynucleotide for specific hybridization to HPV 66 consisting essentially of a sequence or a complement thereof selected from the group consisting of SEQ ID NOs: 1647-1767 (See Table 13). Also described is a set of polynucleotides for specific hybridization to HPV 66, wherein the set comprises at least one polynucleotide consisting essentially of a sequence or a complement thereof selected from the group consisting of: SEQ ID NOs: 1647-1767. In certain embodiments, the methods of the present invention utilize a set of polynucleotide probes for specific hybridization to HPV 66 comprising SEQ ID NOs: 1647-1767.

**Table 13: Polyribonucleotide probes for determining HPV 66 nucleic acid.**

| SEQ ID NO: | Name | Sequence |
|---|---|---|
| 1647 | HPV66_7794 | GUCGUGCUAAAACAGGUUUCUUUUA |
| 1648 | HPV66_7737 | GUAUCUGUCUUGCAAAUAUGUAACC |
| 1649 | HPV66_7712 | GUGUAGCCCUUAUUGUAUAAGCCAA |
| 1650 | HPV66_7687 | GGUGUUUGCAAUAUAUUUUGUUGGC |
| 1651 | HPV66_7611 | UUACUCACCUGUAUUUCUGUGCCAA |
| 1652 | HPV66_7586 | GGUAUGUACACUGCCUUACCCUGUA |
| 1653 | HPV66_7521 | CAAAACGACUUUUCAGCAAAACAGU |
| 1654 | HPV66_7496 | CUAGCCUUUUGUCCUUAUUUAAACC |
| 1655 | HPV66_7466 | CAUUUUAUGCAUGCAACCGAAUUCG |
| 1656 | HPV66_7441 | CAAACUCCAUUUUAGUGCUGUACGC |
| 1657 | HPV66_7416 | GUUUGUAUGCACUAUAGUAACACAC |
| 1658 | HPV66_7377 | GUGGUGUUCCUUACUGUUUAAUGUU |
| 1659 | HPV66_7352 | CCUUGGGCAGUGUGUGUCAGGUUAG |
| 1660 | HPV66_7299 | AACAUGCAUGGUUACUUUUACGCGU |
| 1661 | HPV66_7246 | GCUAUGUGUAUGUAUGACUGUAUGU |
| 1662 | HPV66_7183 | UGUAUGGUUGUGCUUGUACUGUAUG |
| 1663 | HPV66_7122 | UUCCUCUUCUUCACCAGCUAAACGU |
| 1664 | HPV66_7097 | CUAAAAGGCGGGCGGCUCCUACCUC |
| 1665 | HPV66_7071 | UAGACCCAAGGCUAGUGUAUCUGCC |
| 1666 | HPV66_7000 | AGCUUUUCUGCAGACCUGGAUCAGU |
| 1667 | HPV66_6956 | AUCCCCUGGCUAAAUAUAAGUUUUG |
| 1668 | HPV66_6858 | AUCCCCACCAGUUGCAACUAGCUUA |
| 1669 | HPV66_6720 | CAAUCAAUACCUUCGCCAUGUGGAG |
| 1670 | HPV66_6692 | CAUUAACUAAAUAUGAUGCCCGUGA |
| 1671 | HPV66_6666 | CAUGACUAUUAAUGCAGCUAAAAGC |
| 1672 | HPV66_6540 | GAUUACCUCUGAGGCCCAAUUAUUU |
| 1673 | HPV66_6498 | UCCUCCCAGUUCUGUAUAUGUUGCU |
| 1674 | HPV66_6466 | UUGUAUUGGAAGGGUGGCAAUGGCA |
| 1675 | HPV66_6433 | GCAGGUAAUGUUGGGGAAGCCAUUC |
| 1676 | HPV66_6274 | AAGCUAUUACAGGAAUCAAAGGCUG |
| 1677 | HPV66_6220 | ACCCCGAUAGAGGACGGUGACAUGG |
| 1678 | HPV66_6195 | UUGUCCACCUCUUGCAUUAGUUAAU |
| 1679 | HPV66_6170 | AGUCUACACCAGGUAAUACAGGGGA |
| 1680 | HPV66_6145 | CAUUGGACUAAGGGCGCGGUGUGUA |
| 1681 | HPV66_6061 | AUAGAAGAUAGCCGGGACAAUAUAU |
| 1682 | HPV66_6031 | GAGGUCUCUAAUUUAGCAGGUAAUA |
| 1683 | HPV66_5999 | GUCAUCCAUUAUUUAAUAGGCUGGA |
| 1684 | HPV66_5904 | UCCAUCUUUCUAUAAUCCUGACCAG |
| 1685 | HPV66_5836 | GUUAGUGCAUAUCAGUAUAGAGUGU |
| 1686 | HPV66_5811 | UGGUACCAAAACAAACAUCCCUAAA |
| 1687 | HPV66_5783 | GCCAUCCUUAUUACUCUGUUUCCAA |
| 1688 | HPV66_5718 | GGAUACAUAUGUAAAACGUACCAGU |
| 1689 | HPV66_5565 | AUACAGGGAGCUACAUUUGCACUAU |
| 1690 | HPV66_5520 | CCCUUCGUACCUCAGUCUCCUUCUG |
| 1691 | HPV66_5469 | CCAUUUUAUUCAGGUCCUGAUAUAG |
| 1692 | HPV66_5427 | ACAGCUAAUGUUACUGCCCCUUUGG |
| 1693 | HPV66_5400 | CCUUCUACAUUAUCCUUUGCUAGUA |
| 1694 | HPV66_5374 | CACCUUCUGCACAAUUACCUAUUAA |
| 1695 | HPV66_5187 | CAAACACGUAGGGGUACGCAAAUAG |
| 1696 | HPV66_5128 | CAUUUACUACACGUAGAACAGGUGU |
| 1697 | HPV66_5003 | CCCCACAACAUUAAUAUCUGCUGAU |
| 1698 | HPV66_4943 | CAGGUUAUAUAGUAGGGCUUUUCAG |
| 1699 | HPV66_4918 | CAGGUUUUAGACGCCUUGCUGCUCC |
| 1700 | HPV66_4873 | CUAUACACGGUACUGGCAACGAACC |
| 1701 | HPV66_4831 | CUGGAAUACAUAGCUAUGAGGAAAU |
| 1702 | HPV66_4801 | CUGGUAAUAUUUUGAUUAGCACUCC |
| 1703 | HPV66_4760 | UGAUCCUCCAGUAAUUGAGGCUCCA |
| 1704 | HPV66_4729 | GUAGUACUACUAUAACAAACCCACU |
| 1705 | HPV66_4704 | CCCACAUCUAGUACUGUACAUGUAA |
| 1706 | HPV66_4617 | GGGGCUGGUGUUCCCAAUUUUACUG |
| 1707 | HPV66 4544 | UGUGGUGGAGUCAGUUGGGCCUACA |
| 1708 | HPV66_4509 | ACUAUAGUUGAUGUCACUCCUGCAC |
| 1709 | HPV66_4209 | GUGUAUAUAUUGCCAUGCUUUGUGG |
| 1710 | HPV66_4038 | UGCGCUUUGCUUUUGUGUUUGUCUG |
| 1711 | HPV66_3990 | GUAAUCGCCAUAUAUUGCAACCAUU |
| 1712 | HPV66_3965 | AUUGUAACACUGGGAAAGGUAACGU |
| 1713 | HPV66_3915 | GCUAAGCAUAUAUAUUGCACCCAUU |
| 1714 | HPV66_3890 | UGAAGUGUAAUUGCCAUACAUUGCU |
| 1715 | HPV66_3821 | CAAAUGAGUUGUCCAUAAAGUGUUG |
| 1716 | HPV66_3796 | ACCUAGUGUACAGGUUAUUUUGGGA |
| 1717 | HPV66_3702 | GGACAAGUACAGAUAAUAAAGACAG |
| 1718 | HPV66_3586 | UGAUAAAACUACGCCUGUAAUCCAU |
| 1719 | HPV66_3536 | AACAACGCCAACAGUAGAAGUCCAC |
| 1720 | HPV66_3470 | GAAUCAGAACCUGACUCCUCCAGAG |
| 1721 | HPV66_3445 | ACCAGGAAAACGACCCAGAGCAAGU |
| 1722 | HPV66_3296 | ACCGAGAGUAUUUACUGUCCUGACU |
| 1723 | HPV66_3228 | AUUACACAGACUUUGAACAGGAGGC |
| 1724 | HPV66_3181 | GGUGGAUUACAGAGGCAUAUAUUAU |
| 1725 | HPV66_3144 | AUAAUGGAGAGUGUGGGUGGUGUAA |
| 1726 | HPV66_3109 | UUGUAUGGAAUAUGUGGUGUGGAAA |
| 1727 | HPV66_3017 | ACAUGUGAUGAACUGUGGCGCACGG |
| 1728 | HPV66_2961 | CACUGGAAGCAAUAAGUAACACAAU |
| 1729 | HPV66_2878 | CAUUAAUGUACUAAACCACCAGAUG |
| 1730 | HPV66_2614 | CCAUUAGAUAACAAUGGUAAUCCUG |
| 1731 | HPV66_2411 | CAGAUACGUGUUGGAGAUACAUAGA |
| 1732 | HPV66_2374 | CUAGACAAUGCCAAAUUAGGUUUGC |
| 1733 | HPV66_2254 | UUGGUACUGUGUGGACCACCAAAUA |
| 1734 | HPV66_2104 | UGCCAGUGGAUAAAGCAUAUAUGUA |
| 1735 | HPV66_1941 | AGUAACAGAUGAUAGCCAAAUUGCC |
| 1736 | HPV66_1875 | GCAACACAGUUUACAAGACAAUCAA |
| 1737 | HPV66_1739 | CACAAGAGCAAAUGUUAAUUCAACC |
| 1738 | HPV66_1649 | GGGGAGUAAUUGUAAUGAUGCUAAU |
| 1739 | HPV66_1612 | UGUGUGUACUAUCAUAUGCAAUGCU |
| 1740 | HPV66_1532 | GUUGUAACGAUUGGAUAUGUGCAAU |
| 1741 | HPV66_1484 | GAGUGCCAUAUACAGAGUUGGUGCG |
| 1742 | HPV66_1436 | GUAGUAACGUACAAGGAAGAUUACA |
| 1743 | HPV66_1403 | CACCAACACACCAAUUGCAGGAACU |
| 1744 | HPV66_1363 | CACUCGGUAUCAAAUAUGGAUAUAG |
| 1745 | HPV66_1332 | UGGAGGCUCGCAAAACAGUAAUUGU |
| 1746 | HPV66_1298 | ACGAAAAGGGAAAUGGGUGCGGGAG |
| 1747 | HPV66_1273 | UUGGAAACAUCACAACAGGUAGAAU |
| 1748 | HPV66_1226 | GGCUAAUAUUAUCAGAAGACAGCGG |
| 1749 | HPV66_1165 | GGUAGUCCCUUAAGUGAUAUUAGUA |
| 1750 | HPV66_1106 | AAGUACAAACAGCACAUGCAGAUGC |
| 1751 | HPV66_939 | UGGAUGGUUUCAGGUAGAAGCAAUU |
| 1752 | HPV66_874 | CGCAUCAUCUAAAUAACUGCAAUGG |
| 1753 | HPV66_819 | UACGUGUGGUACAACAGCUGCUUAU |
| 1754 | HPV66_791 | UUGGACAUUCAGAGUACCAAAGAGG |
| 1755 | HPV66_759 | UACCUUGUUGUAAGUGUGAGUUGGU |
| 1756 | HPV66_604 | UAUAUUAGAACUUGCACCGCAAACG |
| 1757 | HPV66_579 | GUAAAGUACCAACGUUGCAAGAGGU |
| 1758 | HPV66_554 | AGAAUCUACAGUAUAACCAUGCAUG |
| 1759 | HPV66_529 | GGAGACAUACGAGUAGACAAGCUAC |
| 1760 | HPV66_504 | UGGACCGGGUCAUGUUUGCAGUGUU |
| 1761 | HPV66_462 | CACUGUGAACAUAAAAGACGAUUUC |
| 1762 | HPV66_346 | AUAAAUAUUCAGUGUAUGGGGCAAC |
| 1763 | HPV66_291 | GCAGUAUGUAGGGUAUGUUUAUUGU |
| 1764 | HPV66_150 | CAUCUGAGCGAGGUAUUACAAAUAC |
| 1765 | HPV66_115 | UCAGCAAUACACAGGAACGUCCACG |
| 1766 | HPV66_88 | GCCUGUAGAUAUCCAUGGAUUCCAU |
| 1767 | HPV66_63 | GUACAUAUAAAAGGCAGCCUGUUGU |

Also described is an isolated polynucleotide for specific hybridization to HPV 68 consisting essentially of a sequence or a complement thereof selected from the group consisting of SEQ ID NOs: 1768-1875 (See Table 14). Also described is a set of polynucleotides for specific hybridization to HPV68, wherein the set comprises at least one polynucleotide consisting essentially of a sequence or a complement thereof selected from the group consisting of: SEQ ID NOs: 1768-1875. In certain embodiments, the methods of the present invention utilize a set of polynucleotide probes for specific hybridization to HPV 68 comprising SEQ ID NOs: 1768-1875.

**Table 14: Polyribonucleotide probes for determining HPV 68 nucleic acid.**

| SEQ ID NO: | Name | Sequence |
|---|---|---|
| 1768 | HPV68 7798 | CUGAACACAGCAGUUCUCUAUACUA |
| 1769 | HPV68_7696 | GGCACACAUACCAAUACUUUUACUU |
| 1770 | HPV68_7661 | CUACAUCCAUAAAUUUGUGCAACCG |
| 1771 | HPV68_7628 | UGUCUGGUAGUGUAAGUUAUACAGU |
| 1772 | HPV68_7597 | GCCAGUAUAACUACUUUUGCAUUCA |
| 1773 | HPV68_7527 | CCUCCCUUGUAAUAAAACUGCUUUU |
| 1774 | HPV68 7502 | CAAUAGUUUGGCAACCAACGUAUCU |
| 1775 | HPV68_7452 | UCGUACUGGCGCACCUUAGUUAGUC |
| 1776 | HPV68_7427 | CCCACAUAGUUGGCACCAGUAACAG |
| 1777 | HPV68_7352 | GUCGUUGGUACUAUUUGCUUUUAGA |
| 1778 | HPV68_7325 | UGGCCGGGUUGUGUGCGACCGCUUU |
| 1779 | HPV68_7300 | AACUAUACCGUGUGGCCAUUUUGUA |
| 1780 | HPV68_7258 | CCUAAGGUGUGUUACAUUAUAUGCA |
| 1781 | HPV68_7186 | CUGUGACUAACAUAUGUCCUUGUUU |
| 1782 | HPV68_7159 | UAUGUCCGUGUCCUUUGUGGUUGCA |
| 1783 | HPV68_7108 | GUGUAUGUUUGCAAGUAUGUGUGUA |
| 1784 | HPV68_7078 | GUGUAUGUGCAUGUAUGUGUAUGUG |
| 1785 | HPV68_7053 | UGUGUCAUGUUGGUGUUGGUAUGUU |
| 1786 | HPV68_7028 | UGUUGUUUGUCUGUGUGGUUGUAUA |
| 1787 | HPV68_6961 | ACCACAUCUACCUCUAAACACAAAC |
| 1788 | HPV68_6898 | UUACAGGCAGGUGUUCGCAGACGGC |
| 1789 | HPV68 6873 | AUUCCCAUUAGGACGCAAAUUUCUG |
| 1790 | HPV68_6848 | AAAAGUUUAGUUCUGAACUGGACCA |
| 1791 | HPV68_6809 | CCUAUGAUGGUCUUAACUUUUGGAA |
| 1792 | HPV68_6749 | ACCUACAAUCAGCAGCAAUUACAUG |
| 1793 | HPV68_6719 | CAUCUGCUAGUCUUGUAGAUACAUA |
| 1794 | HPV68_6541 | GUACCAGCUGUGUAUGAUUCUAAUA |
| 1795 | HPV68_6516 | AUUGUCCACUACUACAGACUCUACU |
| 1796 | HPV68_6337 | GAAACUCCUAGUAGUUAUGUGUAUG |
| 1797 | HPV68_6312 | GUAUAUUAAGGGCACUGACAUUCGU |
| 1798 | HPV68_6145 | GUACCUUUGGAUAUAUGUCAAUCUG |
| 1799 | HPV68_6118 | GGUACAUUACAAGAAACGAAAAGCG |
| 1800 | HPV68_6052 | GAAUUGGUAAAUACUCCUAUUGAGG |
| 1801 | HPV68_6016 | CCUACCAAUGUACAACAAGGGGACU |
| 1802 | HPV68_5924 | AUGUUGCAGUGGACUGUAAACAAAC |
| 1803 | HPV68_5874 | UGAAAAUUCCCCGUUUUCCUCUAAU |
| 1804 | HPV68_5743 | CCUGAGUCUACAUUAUAUAAUCCAG |
| 1805 | HPV68_5625 | CCAUCCAUAUUUUAAGGUUCCUAUG |
| 1806 | HPV68_5600 | GUACAUCUAGGUUAUUAACUGUAGG |
| 1807 | HPV68_5380 | CAAUUGAUACAACCUUUGCCAUAAC |
| 1808 | HPV68_5355 | CAGUUGCCUUUAACACCCUCUACUC |
| 1809 | HPV68_5326 | CUGAUGUUGUAUUACCAUCUACAAC |
| 1810 | HPV68_5301 | UGGAACACGCCUGUAAAUACUGGUC |
| 1811 | HPV68_5270 | UACUAAUACUACCAUUCCUCUUGGU |
| 1812 | HPV68_5245 | UGGCUUCUGCUGCAUCCACUACAUA |
| 1813 | HPV68_5220 | CGUUCCCACAUAUCAGUUCCUUCAU |
| 1814 | HPV68_5158 | CACCUGAUACUGACAAUACUACAGU |
| 1815 | HPV68_5126 | GGACCCUAUGGAUAACUUAUAUGAU |
| 1816 | HPV68_5101 | AACCAUUGGUUGCCCCUGAGCAGGC |
| 1817 | HPV68_5066 | UAGUAACAUUACCCCUGCUGACAGC |
| 1818 | HPV68_5006 | GACCAUGUUUACACGCCGAGGUACA |
| 1819 | HPV68_4973 | AACAGUACGUUUUAGCAGAGUAGGC |
| 1820 | HPV68_4877 | UACUACUCUUACAUAUGAACCUGCU |
| 1821 | HPV68_4823 | AACGCACCCUUCAUCAUUUGUAACA |
| 1822 | HPV68_4692 | GUAUUUGCAACACAUGGCACUGGUA |
| 1823 | HPV68_4636 | UGUUUGUAAGUACCCCUACAUCAGG |
| 1824 | HPV68_4595 | UAUAAUAGAAGUGCCACAAACAGGU |
| 1825 | HPV68_4570 | CUAACCCUGCAUUUACAGACCCGAC |
| 1826 | HPV68_4498 | CUACCACUACACCGGCAGUUUUAGA |
| 1827 | HPV68_4452 | GUACCAACAUUUACAGGCACCUCUG |
| 1828 | HPV68_4427 | CAGUGUUAUUACAUCUGGGACACCA |
| 1829 | HPV68_4395 | GAACCCUCCAUUGUGCAAUUGGUGG |
| 1830 | HPV68_4323 | GGAAAACCUAAUACUGUUGUGGAUG |
| 1831 | HPV68_4206 | GGUACUACACUUGCAGACAAAAUAU |
| 1832 | HPV68_4046 | CAGUAACUGUUAUAGUGUGCAUUUG |
| 1833 | HPV68_4012 | GUGGUUAUUACACAGUCUUACUCUU |
| 1834 | HPV68_3966 | CAUUUGAGGUGUUUGCUGUAUACCU |
| 1835 | HPV68_3867 | GCAUGUAUAUAUGUUGCACUGUCCC |
| 1836 | HPV68_3796 | CCCACACUGUACACUAUAUGUAUAU |
| 1837 | HPV68_3766 | GGGGUAUAUGACAUUAUAAGUGUGU |
| 1838 | HPV68_3728 | GAAACUGUUAAACUACCAUCUAGUG |
| 1839 | HPV68_3697 | UGUUUCAGAAGCACAACGUGACAAG |
| 1840 | HPV68_3514 | AAGACGGAGCCUUUGUUGUGGUGAC |
| 1841 | HPV68_3489 | UCAGUAGAAGUGCAGGCCAAAACAA |
| 1842 | HPV68_3438 | AGCCCUCUGAGCCCGACAACGUGUC |
| 1843 | HPV68_3316 | UACUGAAUCUGUUGCCGACCUACAG |
| 1844 | HPV68_3291 | GUACCACUGACGGAAAAGUAUCCAC |
| 1845 | HPV68_3188 | UAUUACGAAAGGUUUAUGCAGGAUG |
| 1846 | HPV68_3129 | AAACCCAAGGGCGUGUGGAUUACUG |
| 1847 | HPV68_3079 | UGUAGUGUGGGGUACAAUUUACUUU |
| 1848 | HPV68_3054 | GGGACAAGAGUAACUCAAUGCAUUA |
| 1849 | HPV68_2978 | AGUAAUGAACUAUGGCAUACAAAGC |
| 1850 | HPV68_2927 | AGCCUUGCUAAAACUGCAUAUAGUG |
| 1851 | HPV68_2776 | UAACUAUUGGAAUUGUGUGCGACUG |
| 1852 | HPV68_2523 | GUAUUUACAUAGUAGACUAACCGUG |
| 1853 | HPV68_2496 | UAACCCUGUAGAAGACAAUAGGUGG |
| 1854 | HPV68_2429 | GUUUAGAUAGAAAACACAGACACCU |
| 1855 | HPV68_2301 | UUCAGCAAGUCACUUUUGGUUAGAG |
| 1856 | HPV68_2186 | AAGGCACGCCAAAACGAAAUUGUAU |
| 1857 | HPV68_1684 | UUGCAUGUUCCAGACAGCUGUAUGC |
| 1858 | HPV68_1358 | CACCUACUACCCAACUUAAAGUAUU |
| 1859 | HPV68_1333 | GAUAGUGAAAACCAGGAUCCUAAAU |
| 1860 | HPV68_1166 | CAAGACAACCGGCGUAUACAGUGCC |
| 1861 | HPV68_1141 | UCACUAAAUGUAAGCAGUACACAGG |
| 1862 | HPV68_1116 | AGCAAAGUCGCCAUUACAGGAAUUA |
| 1863 | HPV68_1091 | CAGACAGUAUAGAAAGCAGUCCUUU |
| 1864 | HPV68_897 | UAAACAAACAGGUGACACAGUCUCA |
| 1865 | HPV68_772 | UCACUAAAUUUUGUGUGUCCGUGGU |
| 1866 | HPV68_745 | CGGACACUACAACAGCUGUUUAUGG |
| 1867 | HPV68_685 | CUGUGUUGUAAGUGUAACAAGGCAC |
| 1868 | HPV68_518 | UGUUAGAGCUAUGUCCAUACAAUGA |
| 1869 | HPV68_487 | CAUGGACCAAAGCCCACCGUGCAGG |
| 1870 | HPV68_358 | CACCUAACAACAAAACGAAGAUUAC |
| 1871 | HPV68_253 | GUGUAUGCAACUACAUUAGAAACCA |
| 1872 | HPV68_228 | GGAACUACGAUAUUACUCGGAAUCG |
| 1873 | HPV68_150 | UGACCUAUGUGUAGUGUAUAGAGAC |
| 1874 | HPV68_117 | ACAACGGACAGAGGUAUAUGAAUUU |
| 1875 | HPV68_3 | GGCGCUAUUUCACAACCCUGAGGAA |

Also described is an isolated polynucleotide for specific hybridization to HPV 82 consisting essentially of a sequence or a complement thereof selected from the group consisting of SEQ ID NOs: 1876-2026 (See Table 15). Also described is a set of polynucleotides for specific hybridization to HPV 82, wherein the set comprises at least one polynucleotide consisting essentially of a sequence or a complement thereof selected from the group consisting of: SEQ ID NOs: 1876-2026. In certain embodiments, the methods of the present invention utilize a set of polynucleotide probes for specific hybridization to HPV 82 comprising SEQ ID NOs: 1876-2026.

**Table 15: Polyribonucleotide probes for determining HPV 82 nucleic acid.**

| SEQ ID NO: | Name | Sequence |
|---|---|---|
| 1876 | HPV82_7835 | UUGUGUUUUGCCUAUGCUUGCAACA |
| 1877 | HPV82_7785 | AUGUAUUACUCAUCUGCAGGUGUGC |
| 1878 | HPV82_7760 | GCCAAGUUUCUAUCCUACCUAUAAA |
| 1879 | HPV82_7735 | GGCAGGUCAUGAACUAAAUGUCUCU |
| 1880 | HPV82_7662 | CCGCCCUGUAAUAAUUUAUAUGCUU |
| 1881 | HPV82_7612 | CACACCACAUUACUCAUUUGUACUU |
| 1882 | HPV82_7550 | UGGUAUGUACAUCCCGCCCGCCCAC |
| 1883 | HPV82_7525 | GGCAUAACCCUUAAUUCUUUUGGCA |
| 1884 | HPV82_7494 | CAACUUUUGAACCACACUACCUAUG |
| 1885 | HPV82_7408 | GCAUGUACCACAGGAUUCCAUUUUG |
| 1886 | HPV82_7373 | GCAGCACACUUGUAUAUAUAUGUUC |
| 1887 | HPV82_7348 | AUUGCCCUACCCAUAUUUGUGGCUU |
| 1888 | HPV82_7319 | GUUAAGGGUGGUGUUUAGGUGGCGU |
| 1889 | HPV82_7191 | GUAUGGUUUCUGUGUGGUUUACUAA |
| 1890 | HPV82_7130 | GUGUGCGUGUUGUGUGUAUUUGUGU |
| 1891 | HPV82_7086 | CGCCCUGCCUAUGUAUGUGUUUGUG |
| 1892 | HPV82_7030 | CCCCAUCCUCUUCCGCUUCCUCGUC |
| 1893 | HPV82_7001 | CAAACCCAGACCAGGCCUUAAAAGG |
| 1894 | HPV82_6939 | UCUUUGGAUUUGGAUCAGUUUGCAU |
| 1895 | HPV82_6880 | CUAAAGAAGACCCUUUGGCAAAAUA |
| 1896 | HPV82_6755 | GGAUUCUACAAUUUUAGAACAGUGG |
| 1897 | HPV82_6651 | UUUAAGCAGUACAUUAGGCAUGGGG |
| 1898 | HPV82_6626 | UGCACAAACAUUUACUCCAGCAAAC |
| 1899 | HPV82_6589 | CCAAUUUAACCAUUAGCACUGCUGU |
| 1900 | HPV82_6459 | GGUUCUAUGAUAACCUCUGAUUCUC |
| 1901 | HPV82_6433 | GUUAUAUUUAUUCAGCUACUCCCAG |
| 1902 | HPV82_6408 | GGUGCUGGCCGCGACCCUAUUAGUA |
| 1903 | HPV82_6383 | AGACAAGGCUUAUAUUAAGGGUACU |
| 1904 | HPV82_6358 | CUGGUGUGGUUGGUGAUGCCAUUCC |
| 1905 | HPV82_6281 | AGCAGAUACAUAUGGCAAUUCUAUG |
| 1906 | HPV82_6247 | CUGUGUGUAAAUACCCUGAUUACUU |
| 1907 | HPV82_6210 | GCUACUAAAUCAGAUGUUCCAUUGG |
| 1908 | HPV82_6137 | UGUGUCUACUGUCAUUGAGGAUGGC |
| 1909 | HPV82_6039 | AUUAUAGGCUGCGCUCCUCCUAUUG |
| 1910 | HPV82_6012 | GUGGACAACAAACAAACUCAGUUAU |
| 1911 | HPV82_5840 | UAAUCCAGACACAGAUCGUUUGGUG |
| 1912 | HPV82_5815 | UUGGUCUUCCUGAUCCUAAUUUGUU |
| 1913 | HPV82_5738 | UACACGUGCUGAAAUACCUAAGGUA |
| 1914 | HPV82_5654 | AACCCGCACCGGCAUAUAUUAUUAU |
| 1915 | HPV82_5628 | CGCAUUGUCAACACAGAAGAAUAUA |
| 1916 | HPV82_5603 | GUAUUUACCACCUGCACCAGUGUCA |
| 1917 | HPV82_5519 | UAUACAUAUUUGUUACGCAAACGCC |
| 1918 | HPV82_5494 | GGUGGGGAUUACUACUUUGUGGCCG |
| 1919 | HPV82_5467 | GACACACAACAUGCUAUUGUUAUAC |
| 1920 | HPV82_5442 | GCCCUUUAUUCCACACACAUCUAUU |
| 1921 | HPV82_5417 | UGUUACCUACUUCACCCACUGUGUG |
| 1922 | HPV82 5392 | CCUAUUCAUACGGGUCCUGAUGUUG |
| 1923 | HPV82_5345 | CAUCUUAUGCUAAUGUUACUAUCCC |
| 1924 | HPV82_5320 | CCUUCAUUGUCUUCCUCUGUUUCUU |
| 1925 | HPV82_5294 | CAUUUUCUCCUUUGUCUACACAACU |
| 1926 | HPV82_5269 | CAAACCACACCUAUGCUUCGCUCUC |
| 1927 | HPV82_5244 | UGAAACAGGUUUUAUGCAGCCUACA |
| 1928 | HPV82_5182 | CCUUUACUUUCCCCUUCUACUAAUA |
| 1929 | HPV82_5143 | AUAAGUAGUAUUGCACCUGCUGAGG |
| 1930 | HPV82_5009 | AUAUUAUUAAACUGCACCGCCCUGC |
| 1931 | HPV82_4976 | CUACUGAUGUUGCACCAGAUCCUGA |
| 1932 | HPV82_4951 | GAUACAUCAUUGUCCUUUGAGGAAC |
| 1933 | HPV82_4898 | UUAGUAAGCCCUCUACAUUUGUUAC |
| 1934 | HPV82_4872 | GGUUAAGGUUACUAAUCCAGACUUU |
| 1935 | HPV82_4847 | IGUUUAUAUAGCAGGGCAUUUUCACA |
| 1936 | HPV82_4790 | GUAAGGAACCCAUUAGCAGUACACC |
| 1937 | HPV82_4765 | GUAUUUGCCUCCAAUGUUACUACUG |
| 1938 | HPV82_4706 | AUAUAUUUACCAGUACCCCUACGUC |
| 1939 | HPV82_4667 | CAUUUAUUGAGGCACCACAAUCAGG |
| 1940 | HPV82_4627 | ACAAGCACUAACAUUGAAAAUCCCU |
| 1941 | HPV82_4558 | AUUACUUCCUCUUCUACAACAACUC |
| 1942 | HPV82_4511 | AUUCAGGCUCUACUAUACCUACCUU |
| 1943 | HPV82_4425 | UCCGGCCAGGCCUCCAAUUAUUAUU |
| 1944 | HPV82_4400 | GACGGCCUGGUGUUGUAGAUAUUGC |
| 1945 | HPV82_4259 | UUAUUCCUAAGGUAAAGGGCACUAC |
| 1946 | HPV82_4214 | AAUUAUAUUCCACAUGCAAAGCUGC |
| 1947 | HPV82_4165 | ACAAUGGUGGCUGCACGUGCACGGC |
| 1948 | HPV82_4036 | CCACAUCACCUUUAACUACAUUUAC |
| 1949 | HPV82_3976 | AAUCCCAAUAUGUGUUUGCAGCAGC |
| 1950 | HPV82_3876 | UGUAUAUAGUUACUCGCAACCAUUG |
| 1951 | HPV82_3801 | GUCAUUGGGUAUUAUGACAGUGUAA |
| 1952 | HPV82_3776 | UUAAAGUACCAUCAAGUGUGACAGU |
| 1953 | HPV82_3746 | CACACCAACGUCAAAAGUUUAUUGA |
| 1954 | HPV82_3704 | GUAAUACAAAAGCAGGCAUUGUUAC |
| 1955 | HPV82_3668 | UGUUUAAAGAAGUGUCAUCUACCUG |
| 1956 | HPV82_3580 | GCAACUAAAACUGCGUUUAUAGUUC |
| 1957 | HPV82_3544 | GGAACUGCAGGCCCAAACACCGGAG |
| 1958 | HPV82_3519 | CACCUGCGACCACCAAAUACACUGU |
| 1959 | HPV82_3487 | GACUCCUCCACAGUCACCCCGCUGU |
| 1960 | HPV82_3449 | CACCACAACAACGAAAACGACAGCG |
| 1961 | HPV82_3404 | CGACCAAUACCUAUUCCGCCUCCGC |
| 1962 | HPV82_3362 | CACCCUCUACUACAACUGUUGAACA |
| 1963 | HPV82_3337 | GUAUCUAGUACCUACAGCACCCCGU |
| 1964 | HPV82_3295 | GAGGUAUAUAUGUGUGGCAAUGUAA |
| 1965 | HPV82_3198 | CGUGGACUAUACAGGUAUUUAUUAC |
| 1966 | HPV82_3131 | UGGACUAUACAUGUUGGACAUAUGU |
| 1967 | HPV82_3105 | GUUUGAUGGGAAUAAGGACAAUACA |
| 1968 | HPV82_3036 | AUGCUAUGAACUAUGGGGCGAGGCC |
| 1969 | HPV82_2977 | GCAUUAGAAUCGCUAAACAAAUCUG |
| 1970 | HPV82_2937 | AUCAAAACAAAAGGCCUGCCAAGCC |
| 1971 | HPV82_2912 | AUCAAGUAGUACCAGCAUCGGCAGU |
| 1972 | HPV82_2887 | GAAAGAAACAUGCAAACCCUUAACC |
| 1973 | HPV82_2751 | GACCCUAUGUCAUCGUUUAAAUGUG |
| 1974 | HPV82_2650 | GGAAUCCUGUAUAUGCACUAAAUGA |
| 1975 | HPV82_2519 | GCUGCAAAUUGUAUGCCCACCAUUG |
| 1976 | HPV82_2454 | GACCAGUACCUAAGAAAUUUCCUAA |
| 1977 | HPV82_2196 | CGAUACCAGGGUAUUAACUUUAUGU |
| 1978 | HPV82_2138 | GUAUAGAUGUGACAAAGUGCAAGAC |
| 1979 | HPV82_2113 | CACUAACAAUGUCAGCAUGGAUUAG |
| 1980 | HPV82_2088 | CACUACAAACGAGCACAAAGAAAAU |
| 1981 | HPV82_1999 | AAUUGGCUGAUACAGAUAGCAAUGC |
| 1982 | HPV82_1951 | UUGACCAUGAUGUAGUAGACGAUAG |
| 1983 | HPV82_1914 | AGCACGUUUGAACUAUCGCAAAUGG |
| 1984 | HPV82_1889 | ACAACUACAGCACAGUUUUGAUGAU |
| 1985 | HPV82_1841 | CAUUAGUAGCACAUAUGGCGAAACA |
| 1986 | HPV82_1774 | UUAUAGAACCACCUAAGCUACGUAG |
| 1987 | HPV82_1723 | CCAUUGCCAAAUGUUUAGGUACAUU |
| 1988 | HPV82_1685 | ACUGUUAGCUAGAUUUACAUGUGCC |
| 1989 | HPV82_1660 | CAUGUGAUUGGGGUACUAUUGUGCU |
| 1990 | HPV82_1633 | GUAUGUACUACCAUAUACAAUGCCU |
| 1991 | HPV82_1571 | UGCCUUAUUUGGGGUAUCGCCAAUG |
| 1992 | HPV82 1546 | AAACAUGCUGCACGGACUGGGUAUG |
| 1993 | HPV82_1518 | GAGUUGGUAAGGGUAUUUAAAAGUG |
| 1994 | HPV82_1460 | CAAUGCAAAAGCAAUGUUUAUGGCA |
| 1995 | HPV82_1417 | CCAAUGUAGGACUAAACAGUAUAUG |
| 1996 | HPV82_1392 | GACCUGGAAACAAACGAAAAUGCUA |
| 1997 | HPV82_1320 | GAUGGGCAAAAUGACGGGUCACAAC |
| 1998 | HPV82_1294 | AGACUGUGGAAGGACCCUUACAGGU |
| 1999 | HPV82_1242 | AGGAGAUUACUGGACAGUUAUCCGG |
| 2000 | HPV82_1203 | CAGCAACAACCAAAACAGGCAAACC |
| 2001 | HPV82_1156 | GCAGCCCAUUAAAAGACAUUACAAA |
| 2002 | HPV82_1093 | AAACACAGGCACACAAAGAGGCUGU |
| 2003 | HPV82_1068 | GCACAGGCGUUGUUGCAGGUCCAAG |
| 2004 | HPV82_1035 | AAUAGUAUUUGUAGUCAGGCGGAAC |
| 2005 | HPV82_987 | GAUACAAAUGAUACAGGGUCUGAUA |
| 2006 | HPV82_955 | CGGGAGAUAAUAUAUCAGACGAUGA |
| 2007 | HPV82_867 | ACAUCGGCAAUGGACAGUGAAGGUA |
| 2008 | HPV82_833 | UAAGCCUGGUGUGCCCGUGGUGUGC |
| 2009 | HPV82_807 | AUUUCAGCAAAUGUUACUGGGCGAC |
| 2010 | HPV82_782 | AAAGCAGUGGAGACAGCCUUCGCAU |
| 2011 | HPV82_748 | UGCAGGUGUUCGAGUGUUGUACAGC |
| 2012 | HPV82_723 | GUGUUACAGAAUUAAAGUGCACUGU |
| 2013 | HPV82_608 | UAACACCACAACCUGAAAUUGACUU |
| 2014 | HPV82_583 | CAAUUAAAGGACAUAGUGUUGGAGU |
| 2015 | HPV82_539 | UAGUGAAACCCAGGUGUAAUAACGC |
| 2016 | HPV82_514 | AUUGCAGAAAACCACCAAGACAACG |
| 2017 | HPV82_440 | AGAAAAGCAAAAGGUGGUGGACGAC |
| 2018 | HPV82_415 | GAUGUCAGAGACCACUUGGGCCUGA |
| 2019 | HPV82_361 | CAUUAGAGGCCAUUACUAACAAAAG |
| 2020 | HPV82_332 | AAGGUAUAGUAGGUCUGUGUAUGGU |
| 2021 | HPV82_265 | GGGACAAUACGCCAUAUGCAGCAUG |
| 2022 | HPV82_234 | GUAGCAUUUACAGAACUUAGGAUUG |
| 2023 | HPV82_209 | GUUGUGUAGAGCAGAUGUGUAUAAU |
| 2024 | HPV82_164 | GUCUAUGCACAAUAUUCAGGUAUUG |
| 2025 | HPV82_139 | ACGAAUUAUGUGAAGCCUGCAAUAC |
| 2026 | HPV82_105 | UUUGAAGACAUAAGAGAAAGACCAC |

### Hybridization

The methods of the present invention comprises contacting the polynucleotide probes with the sample under a hybridization condition sufficient for the polynucleotide probes to hybridize to the target nucleic acid in the sample to form double-stranded nucleic acid hybrids. Preferably, the polynucleotide probes are diluted in a probe diluent that also can act as a neutralizing hybridization buffer. The diluent can be used to dissolve and dilute the probe and also help restore the sample to about a neutral pH, e.g., about pH 6 to about pH 9, to provide a more favorable environment for hybridization. Sufficient volume of probe diluent, preferably one-half volume, can be used to neutralize one and one-half volume of base-treated sample. Preferably, the probe diluent is a 2-[bis(2-Hydroxyethyl) amino] ethane sulfonic acid (BES, Sigma, St. Louis, Mo.)/sodium acetate buffer. Most preferably, the probe diluent is a mixture of 2 M BES, 1 M sodium acetate, 0.05% of the antimicrobial agent NaN₃, 5 mM of the metal chelating agent EDTA, 0.4% of the detergent Tween™-20 and 20% of the hybridization accelerator dextran sulfate. The pH of the probe diluent can be about 5 to about 5.5.

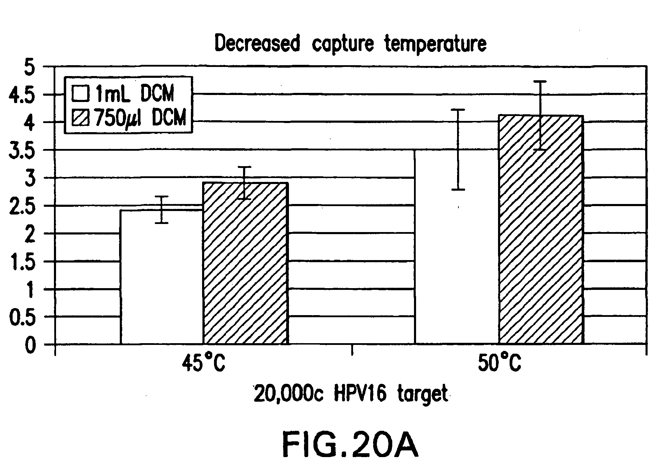

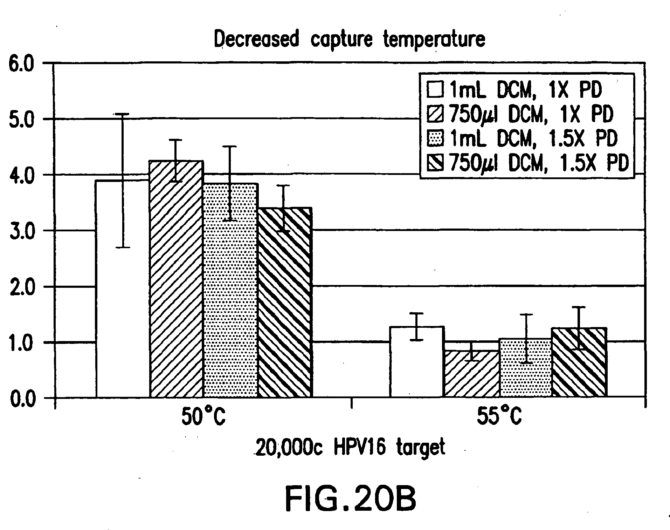

Thus, for example, after treatment with base, an aliquot of sample can be removed from the sample tube and combined with a sufficient amount of probe to allow hybridization to occur under a hybridization condition. The hybridization condition is sufficient to allow the polynucleotide probes to anneal to a corresponding complementary nucleic acid sequence, if present, in the sample to form double-stranded nucleic acid hybrids. The probes and sample nucleic acids can be incubated for a hybridization time, preferably at least about 5 minutes, to allow the polynucleotide probes to anneal to a corresponding complementary nucleic acid sequence. The hybridization condition can comprise a hybridization temperature of at least about 20° C, preferably about 50 to about 80° C. In certain embodiments, the hybridization is performed at a temperature of less than 55° C. In other embodiments when synRNA probes are used and when the sample containing the target nucleic acid contains a large volume of collection medium (i.e. ≥ 1 ml), the hybridization temperature is between 45°C and 55° C and preferably is about 50°C (see figures 20A and 20B). Lowering the hybridization temperature provides the ability to detect 20,000 copies of HPV target nucleic acid in an assay. For any given target to be determined and the polynucleotides employed, one of ordinary skill in the art can readily determine the desired hybridization condition by routine experimentation.

The present invention also allows for hybridization of probes to targets in the presence of anti-hybrid antibody that is immunospecific to double-stranded nucleic acid hybrids (i.e. the anti-hybrid antibody can be added at the same time or before the probes are added to the sample containing the target nucleic acid). This allows for reduction in the time to perform an assay.

### Anti-hybrid Antibodies

The double-stranded nucleic acid hybrids formed in accordance with the present invention can be detected using an antibody that is immunospecific to double-stranded nucleic acid hybrids. The antibody is immunospecifc to double-stranded hybrids, such as but not limited to RNA/DNA; DNA/DNA; RNA/RNA; and mimics thereof, where "mimics" as defined herein, refers to molecules that behave similarly to RNA/DNA, DNA/DNA, or RNA/RNA hybrids. The anti-double-stranded nucleic acid hybrid antibody (*i.e*., "anti-hybrid" antibody) that is utilized will depend on the type of double-stranded nucleic acid hybrid formed. In one embodiment, the antibody is immunospecific to RNA/DNA hybrids.

It will be understood by those skilled in the art that either polyclonal or monoclonal anti-hybrid antibodies can be used and/or immobilized on a solid support or phase in the present assay as described below. Monoclonal antibody prepared using standard techniques can be used in place of the polyclonal antibodies. Also included are immunofragments or derivatives of antibodies specific for double-stranded hybrids, where such fragments or derivatives contain binding regions of the antibody.

For example, a polyclonal RNA:DNA hybrid antibody derived from goats immunized with an RNA:DNA hybrid can be used. Hybrid-specific antibody can be purified from the goat serum by affinity purification against RNA:DNA hybrid immobilized on a solid support, for example as described in Kitawaga et al., Mol. Immunology, 19:413 (1982); and U. S. Patent No. 4,732, 847.

Other suitable methods of producing or isolating antibodies, including human or artificial antibodies, can be used, including, for example, methods which select recombinant antibody (e.g., single chain Fv or Fab, or other fragments thereof) from a library, or which rely upon immunization of transgenic animals (e.g., mice) capable of producing a repertoire of human antibodies (see, *e.g.,* Jakobovits et al., Proc. Natl. Acad. Sci. USA, 90:2551 (1993); Jakobovits et al., Nature, 362: 255 (1993); and U.S. Pat. Nos. 5,545, 806 and 5,545, 807).

The target nucleic acid to be determined is DNA *(e.g.,* HPV 18 genomic DNA), wherein the polynucleotide probes are polyribonucleotides. According to this embodiment, the double-stranded nucleic acid hybrids (*i.e.* DN/RNA hybrids) formed can be detected using an antibody that is immunospecific to RNA:DNA hybrids.

In a preferred embodiment of the present invention, a polyclonal anti-RNA/DNA hybrid antibody is derived from goats immunized with an RNA/DNA hybrid. Hybrid-specific antibody is purified from the goat serum by affinity purification against RNA/DNA hybrid immobilized on a solid support. Monoclonal antibody prepared using standard techniques can be used in place of the polyclonal antibodies.

While any vertebrate may be used for the preparation of anti-RNA/DNA hybrid monoclonal antibodies, goats or rabbits are preferred. Preferably, a goat or rabbit is immunized with a synthetic poly(A)-poly(dT) hybrid by injecting the hybrid into the animal in accordance with conventional injection procedures. Polyclonal antibodies may be collected and purified from the blood of the animal with antibodies specific for the species of the immunized animal in accordance with well-known antibody isolation techniques. For the production of monoclonal antibodies, the spleen can be removed from the animal after a sufficient amount of time, and splenocytes can be fused with the appropriate myeloma cells to produce hybridomas. Hybridomas can then be screened for the ability to secrete the anti-hybrid antibody. Selected hybridomas may then be used for injection into the peritoneal cavity of a second animal for production of ascites fluid, which may be extracted and used as an enriched source of the desired monoclonal antibodies.

In some embodiments, the step of detecting comprises contacting the double-stranded nucleic acid hybrids with a first anti-hybrid antibody to capture the double-stranded nucleic acid hybrids, wherein the first anti-hybrid antibody is immunospecific to double-stranded nucleic acid hybrids. In one embodiment, the first anti-hybrid antibody is immobilized onto a solid support such as a test tube surface. It will be understood by those skilled in the art that a solid support includes polystyrene, polyethylene, polypropylene, polycarbonate or any solid plastic material in the shape of test tubes, beads, microparticles, dip-sticks or the like. Examples of a solid support also includes, without limitation, glass beads, silica beads, glass test tubes, and any other appropriate shape made of glass. A functionalized solid support such as plastic, silica, or glass that has been modified so that the surface contains carboxyl, amino, hydrazide or aldehyde groups can also be used. Immobilization of the antibody can be direct or indirect. Preferably, test tubes are directly coated with anti-hybrid antibody in accordance with methods known to those skilled in the art or briefly described below. The antibody can also be biotinylated and subsequently immobilized on, for example streptavidin coated tubes or silica, or modified by other methods to covalently bind to the solid phase. Solubilized biotinylated antibody can be immobilized on the streptavidin coated tubes before capture of the hybridized samples as described below or in conjunction with the addition of the hybridized samples to simultaneously immobilize the biotinylated antibody and capture the hybrids.

In another embodiment, the first anti-hybrid antibody is attached to the solid phase in accordance with the method of Fleminger et al., Appl. Biochem. Biotech. 23:123 (1990), by oxidizing the carbohydrate portion of the antibody with periodate to yield reactive aldehyde groups. The aldehyde groups are then reacted with a hydrazide-modified solid phase such as MicroBind-HZ™ microtiter plates available from Dynatech Laboratories (Chantilly, Va.). Passive coating of the antibody according to the well known method of Esser, P., Nunc Bulletin No. 6 (November 1988) (Nunc, Roskilde, Denmark) can also be employed.

In other embodiments, Ventrex Star™ tubes (Ventrex Laboratories Inc., Portland, ME) are coated with streptavidin by the method of Haun et al., Anal. Biochem. 191:337-342 (1990). After binding of streptavidin, a biotinylated goat polyclonal antibody as described above, or otherwise produced by methods known to those skilled in the art, is bound to the immobilized streptavidin. Following antibody binding, tubes can be post-coated with a detergent such as Tween™-20 and sucrose to block unbound sites on the tube and stabilize the bound proteins as described by Esser, Nunc Bulletin No. 8, pp. 1-5 (December 1990) and Nunc Bulletin No. 9, pp. 1-4 (June 1991) (Nunc, Roskilde, Denmark) and Ansari, et al., J. Immunol. Methods, 84:117 (1985). Preferably, each tube is coated with between 10 ng and 100 µg biotinylated antibody. Most preferably each tube is coated with approximately 250 ng of biotinylated antibody.

As discussed above, the solid phase can be coated with functional antibody fragments or derivatized functional fragments of the anti-hybrid antibody.

In some embodiments, hybridized samples are incubated in tubes coated with the first anti-hybrid antibody for a sufficient amount of time to allow capture of the double-stranded nucleic acid hybrids by the immobilized capture antibodies. The hybrids can be bound to the immobilized antibodies by incubation, for example incubation for about 5 minutes to about 24 hours at about 15 to about 65° C. In some embodiments, the incubation time is about 30 to about 120 minutes at about 20 to about 40° C, with shaking at about 300 to about 1200 rpm. In another embodiment, capture occurs with incubation at about one hour at about room temperature with vigorous shaking on a rotary platform. It will be understood by those skilled in the art that the incubation time, temperature, and/or shaking can be varied to achieve alternative capture kinetics as desired.

In other embodiments, the first anti-hybrid antibody is coupled to a magnetic bead (e.g., COOH-beads) to capture double-stranded nucleic acid hybrids. Magnetic bead-based technology is well known in the art. In some embodiments, magnetic silica beads having derivatized surfaces for reacting with antibody can be employed.

In one embodiment, the step of detecting further comprises providing a second anti-hybrid antibody that is immunospecific to double-stranded nucleic acid hybrids, wherein the second anti-hybrid antibody is detectably labeled either directly or indirectly.

For example, in some embodiments, an anti-hybrid antibody as described above can be conjugated to a detectable label to provide the second anti-hybrid antibody for detection of the double-stranded nucleic acid hybrids. Conjugation methods for labeling are well known in the art. Preferably, an antibody, such as the mouse monoclonal antibody deposited with the American Type Culture Collection as ATCC Accession number HB-8730, is conjugated to a detectable label such as alkaline phosphatase. It will be understood by those skilled in the art that any detectable label such as an enzyme, a fluorescent molecule, or a biotin-avidin conjugate can be used.

The antibody conjugate can be produced by well known methods such as direct reduction of the monoclonal antibody with dithiothreitol (DTT) to yield monovalent antibody fragments. The reduced antibody can then be directly conjugated to maleimated alkaline phosphatase by the methods of Ishikawa et al., J. Immunoassay 4:209-237 (1983) and Means et al., Chem. 1: 2-12 (1990), and the resulting conjugate can be purified by HPLC.

In another embodiment, the double-stranded nucleic acid hybrids can be detected indirectly, for example using an unlabelled anti-hybrid antibody for which a labeled antibody is specific. For example, the second anti-hybrid antibody can be a mouse immunoglobulin that is detected by a labeled goat anti-mouse antibody.

The double-stranded nucleic acid hybrids can be contacted with the second anti-hybrid antibody under a binding condition that is sufficient to provide for specific antibody-antigen binding (*i.e.,* antibody/double-stranded nucleic acid hybrid binding), while minimizing non-specific binding. The binding condition preferably comprises a binding buffer comprising 0.1 M Tris-HCl, pH 7.5, 0.6 M NaCl to reduce cross reaction of antibody with other nucleic acid species, ZnCl₂ and MgCl₂ for stabilizing alkaline phosphatase, normal goat serum to block non-specific interaction of conjugate with the capture surface, 0.25% of the detergent Tween™-20 to block non-specific binding of conjugate, and sodium azide as a preservative. Reactions can then be washed with a wash buffer (e.g., 0.1 M Tris-HCl, pH 7.5, 0.6 M NaCl, 0.25% Tween™-20, and sodium azide) to remove as much of the unbound or non-specifically bound second anti-hybrid antibody as possible. The second anti-hybrid antibody that is bound to the double-stranded nucleic acid hybrids can subsequently be detected, for example by colorimetry or chemiluminescence methods as described by *e.g.,* Coutlee, et al., J. Clin. Microbiol. 27:1002-1007 (1989). For example, bound alkaline phosphatase conjugate can be detected by chemiluminescence with a reagent such as a Lumi-Phos™ 530 reagent (Lumigen, Detroit, MI) using a detector such as an E**/**Lumina™ luminometer (Source Scientific Systems, Inc., Garden Grove, CA), an Optocomp I™ Luminometer (MGM Instruments, Hamden, CT), or the like.

In some embodiments, the polynucleotides can be conjugated to a label, such as an enzyme, or to a hapten such as biotin, that is then detected with a labeled anti-hapten antibody.

Thus, target-specific oligoribonucleotides can be designed using commercially available bioinformatics software. For example, for the detection of dsDNA targets, DNA can be denatured, hybridized to the RNA probes, and captured via anti-RNA:DNA hybrid antibodies on a solid support. Detection can be performed by various methods, including anti-RNA:DNA hybrid antibodies conjugated with alkaline phosphatase for chemiluminescent detection. Alternatively, other detection methods can be employed, for example using anti-RNA:DNA hybrid antibodies conjugated with phycoerythrin, suitable for detection by fluorescence.

In other embodiments, the methods of the present invention, optionally, further comprise a step of amplification of the target nucleic acid. Amplification techniques are known in the art and may be utilized. For example, Whole Genome Amplification (WGA) may be employed. WGA is an isothermal process that uses non-specific primers to generate amplicons using the target nucleic acid sequence as a template. For example, *Phi* 29 DNA polymerase can be used in combination with non-specific primers to amplify target nucleic acid sequences. The polymerase can move along the target nucleic acid sequence displacing the complementary strand. The displaced strand becomes a template for replication allowing high yields of high-molecular weight DNA to be generated. For example, helicase-dependent amplification may be employed.

### Kits

Also described is a kit comprising the necessary components and reagents for performing the methods of the present invention. The kit can comprise at least one of the following: an inert sample collection device, such as a dacron swab for exfoliated cell sample collection; a sample transport medium for stabilization of the sample during transport to the laboratory for analysis; a base, or a hydrolysis reagent; one or more polynucleotide probes specific for the target nucleic acid to be determined; neutralizing probe diluent; anti-hybrid antibody coated test tubes; and any necessary controls.

Preferably, the sample transport medium is Specimen Transport Medium; the base is 0.415 M NaOH; the neutralizing probe diluent is a BES/sodium acetate buffer, the test tubes are Ventrex Star™ tubes coated with a polyclonal anti-hybrid antibody; and the conjugated anti-hybrid antibody is a mouse monoclonal antibody conjugated to alkaline phosphatase. Preferably, the kit also contains a substrate for the chemiluminescent detection of alkaline phosphatase, such as a CDP-Star® with Emerald II (Applied Biosystems, Bedford, MA).

The present invention will be illustrated in more detail by way of Examples, but it is to be noted that the invention is not limited to the Examples.

### EXAMPLES

### Example 1: Polynucleotide probes for determining HPV 18 or HPV 16 DNA

Oligoarray 2.0 was chosen as the tool with which to identify RNA probes specific for HPV 18 or HPV 16 DNA. A database of sequences to be checked against, in this case, HPV high risk and low risk types: 1, 2, 3, 4, 5, 6, 8, 11, 13, 16, 18, 26, 30, 31, 33, 34, 35, 39, 40, 42, 43, 44, 51, 52, 53, 54, 56, 58, 59, 61, 62, 66 , 67, 68, 69, 70, 71, 72, 73, 74, 81, 82, 83, 84, and 89 was provided and the sequence of interest, *i.e.,* HPV 16 or HPV 18 was then BLASTed against the database to search for any regions of identity, and the similarities were stored. Tm and %GC were then computed for ribonucleotides of a specified length and compared to the parameters, after which secondary structure was examined. Cross hybridization was checked with the Mfold package, using the similarity determined by BLAST.

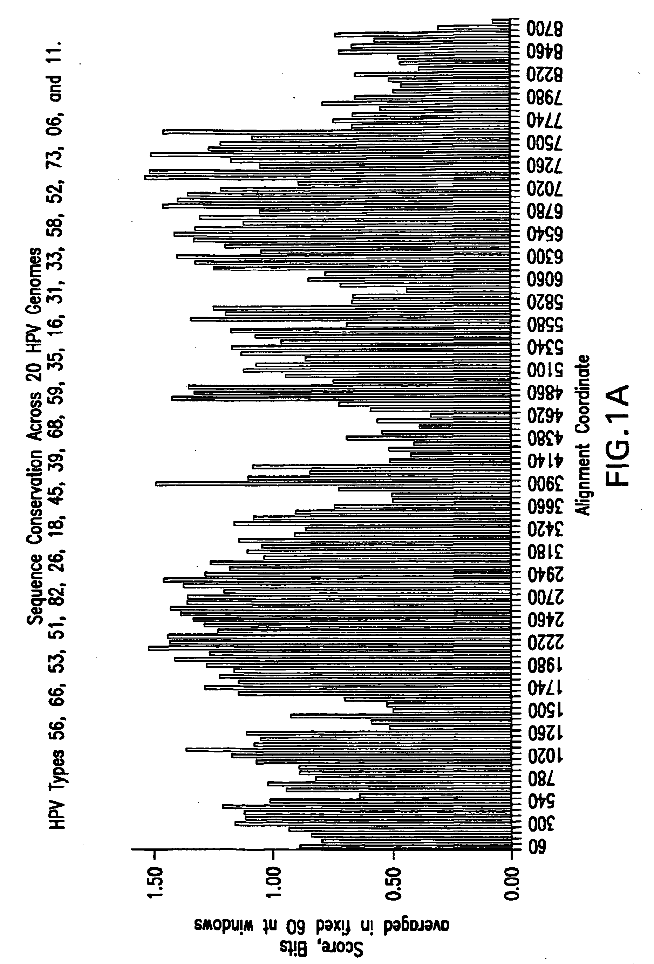

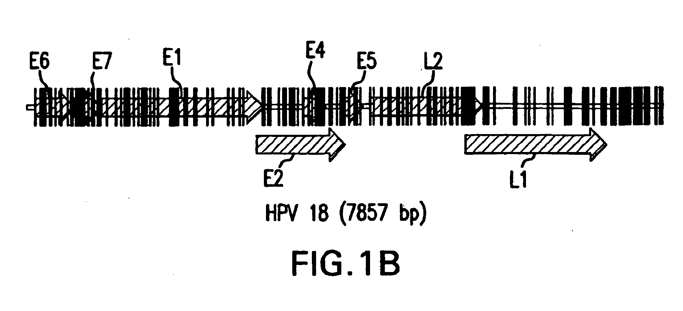

The parameters of the Oligoarray 2.0 program were set to look for ribonucleotides of 25nt length, Tm range of 55-95°C, a GC range of 35-65%, and no secondary structure or cross-hybridization at 55°C or below. Using these parameters to determine ribonucleotide probes for HPV18 (with a modified BLAST database that did not include HPV45, as we are not interested in specificity against that type) resulted in 145 ribonucleotides (for HPV 18) and 127 ribonucleotides (for HPV 16) covering a total of about 3.7kb of the target (*i.e.,* HPV 18 or HPV 16 viral DNA). The sequences of the ribonucleotide probes that were selected are shown Tables 1 and 2 above. Sequence conservation across 20 HPV genomes is shown in Fig. 1a. As schematically shown in Fig. 1b for HPV 18, all regions of the HPV 18 genome were represented in the respective probes.

RNA oligos were ordered from IDT technologies, at the 250 nM scale, with standard desalting. Oligos were stored in Ambion's RNA Storage Solution (1 mM Sodium Citrate, pH 6.4). The synthetic ribonucleotide probes are hereinafter referred to as "synRNA."

### Example 2: Protocol for detecting HPV 18 DNA using HPV 18 synRNA

The hybridization and detection protocol was performed essentially as described in Table 16.

**Table 16: Protocol**

| | | |
|---|---|---|
| Denature | 1 | Sample nucleic acid was denatured with alkali and heat. |
| Hybridize/ Capture | 2 | Synthetic RNA probes were added to sample, hybridized, and neutralized |
| | 3 | Synthetic RNA probe/target DNA hybrids were captured with anti-hybrid antibody immobilized on a substrate |
| Conjugate | 4 | Alkaline phosphatase-conjugated anti-hybrid antibodies were added |
| Wash | 5 | Samples were washed |
| Detection | 6 | Alkaline-phosphatase-activated chemiluminescent substrate was added |
| Read | 7 | Samples were read using a luminometer |

### Example 3: Results

To remove as much variability as possible, data was analyzed as (S-N)/N, expressed as (S/N)-1. When signal = noise, data value = 0.0.

### A. Specificity Demonstrated with HPV18 synRNA

As shown in Table 17, the synthetic RNA probes (synRNAs) designed for HPV 18 showed no cross-reactivity with either HPV 6 or HPV 16 at up to 10⁹ copies/assay (200 ng/ml). synRNA = 3.7kb coverage of HPV 18 DNA; 25 mers @ 1.34 nM final in hybridization.

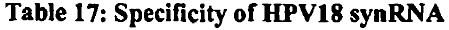

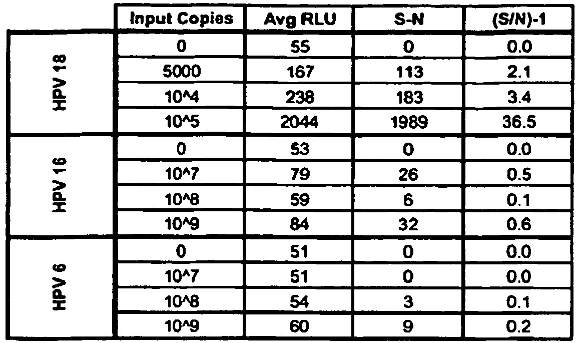

### B. Cross-reactivity of HPV18 synRNA with HPV45

HPV 18 synRNA was not designed to be specific against HPV45 because HPV45 was not part of the specificity design. Accordingly, as shown in Table 18, synRNA for HPV18 showed cross-reactivity against HPV 45 plasmid only starting at between 10⁶ and 10⁷ copies of plasmid. synRNA = 3.7kb coverage of HPV 18 DNA; 25 mers @ 1.34 nM final in hybridization.

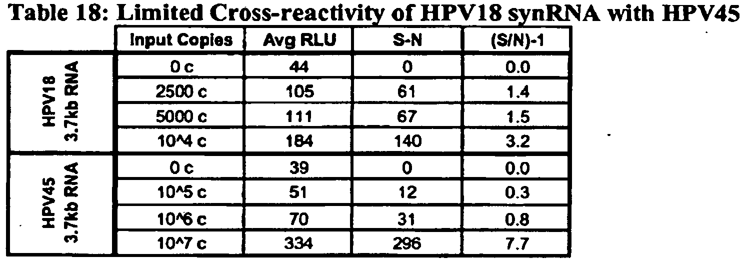

### C. Determining Specificity with HPV16 synRNA

As shown in Table 19, HPV16 synRNA is unable to detect HPVs 6, 18, or 45 at up to 10⁹ copies/assay (200 ng/ml). synRNA = 3.175kb coverage of HPV 16 DNA; 25 mers @ 1.34 nM final in hybridization.

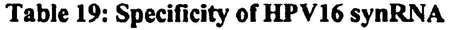

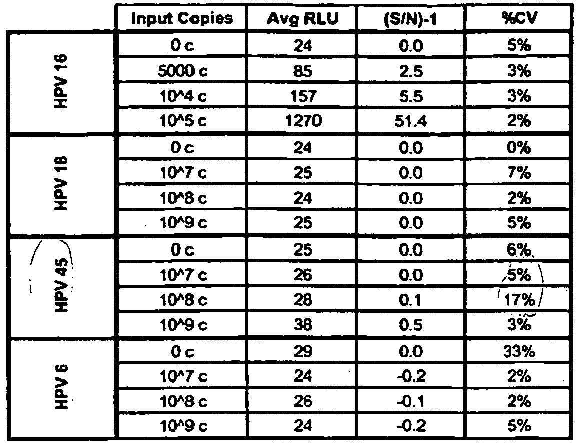

### D. Deterring different HPV Types

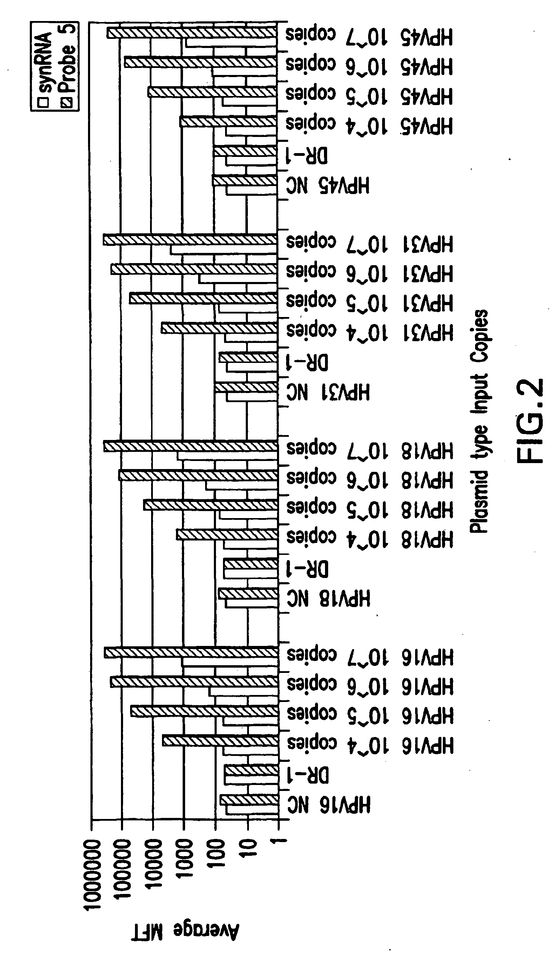

About 0.5kb coverage of specific 25mer probes was provided for HPVs 16, 18, 31, and 45. As shown in Fig. 2, each HPV type was detected at 10⁶ copies. synRNA probes should be equally applicable to detection of whichever HPV types are desired.

### E. Effect of synRNA coverage on sensitivity of detection

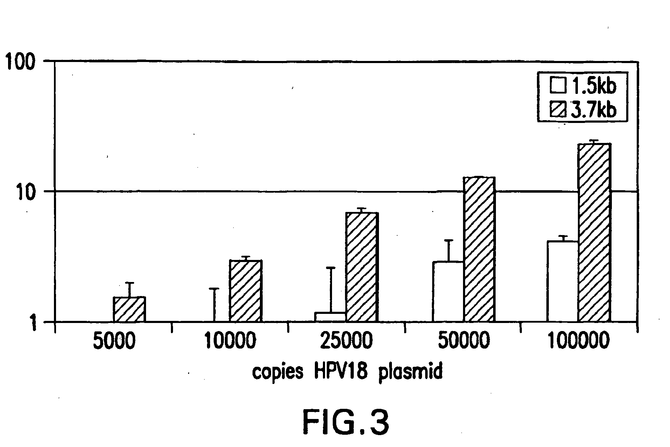

Total coverage of synRNA probe affected signal in the assay. Increasing coverage improved signal in a non-linear fashion, probably due to base-stacking effects and loosening of secondary structure on the single-stranded DNA target as more synRNA probes are hybridized. As shown in Figure 3, at 3.7 kb of coverage, the sensitivity of detection was at 5,000 copies/assay.

### F. Effect of synRNA concentration on sensitivity of detection

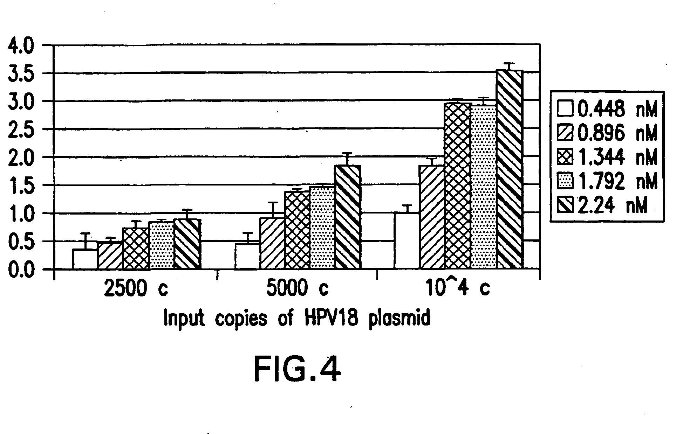

As shown in Figure 4, increasing the concentration of synRNA increased sensitivity of detection. 25mer synRNA oligos had Tms about 45 to about 60°C. Increasing probe concentration raised that Tm, resulting in more efficient hybridization. synRNA = 3.7kb coverage; 25mers @ concentrations shown in Figure 4.

### G. Effect of synRNA size on sensitivity of detection

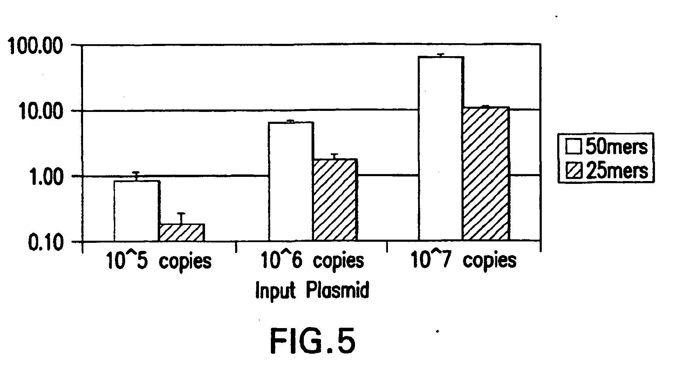

As shown in Figure 5, given equivalent coverage, longer synRNA provided increased sensitivity.

### H. Effect of synRNA contiguity on sensitivity of detection

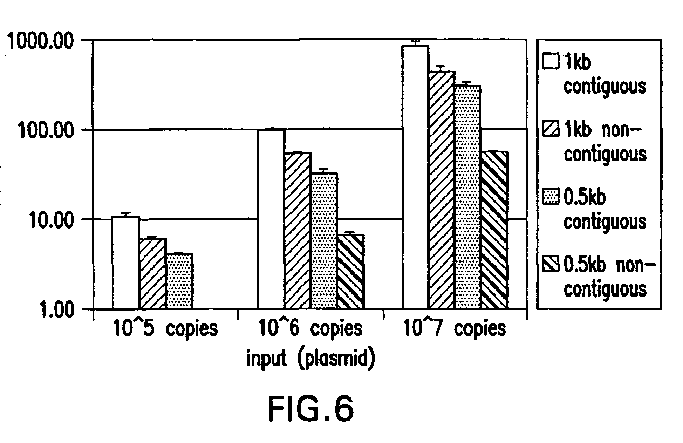

As shown in Figure 6, sensitivity increased as synRNA probes targeted adjacent regions. Without being held to a particular theory, it is believed that hybridization efficiency improved as the binding of one probe relaxed secondary structure on the target strand, providing a more accessible template for hybridization of the adjacent synRNA.

### I. HPV16 and HPV18 are detected at equivalent levels

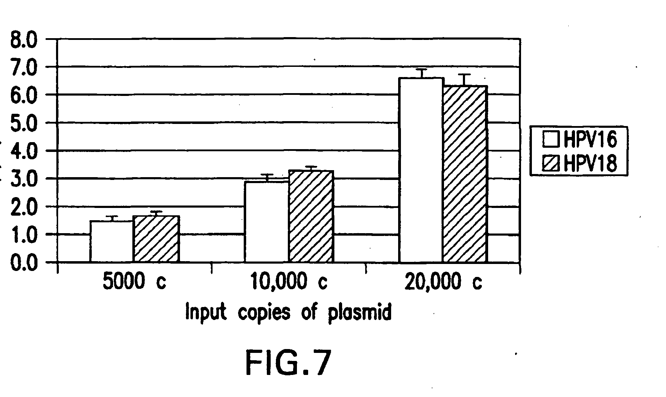

As shown in Figure 7, HPV16 synRNA, with about 3.175kb coverage, and HPV18, with about 3.7kb coverage, gave about similar results. Both synRNAs were able to detect their respective targets at a concentration of 5,000 copies.

### J. Comparison of different synRNA synthesis chemistries

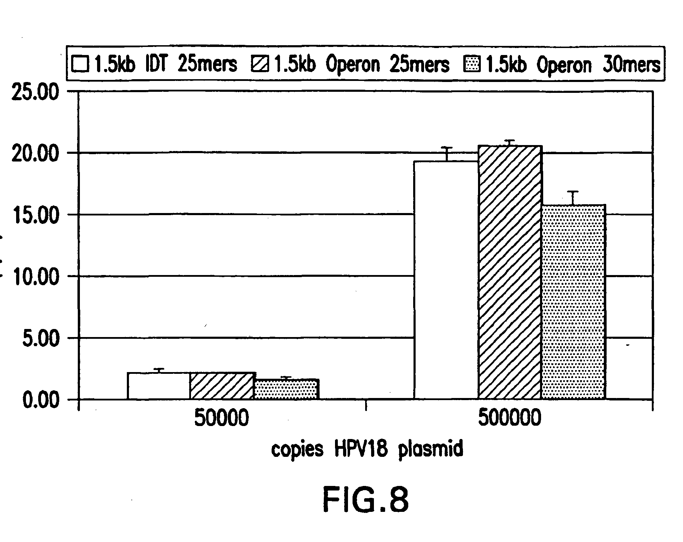

SynRNAs were prepared by TOM amidite chemistry (Operon Biotechnologies, Inc., Huntsville, AL) or by tBDMS chemistry (Integrated DNA Technologies (IDT)). As shown in Figure 8, 25mers of comparable quality can be provided using different chemical synthesis methods.

### K. Detection at different temperatures

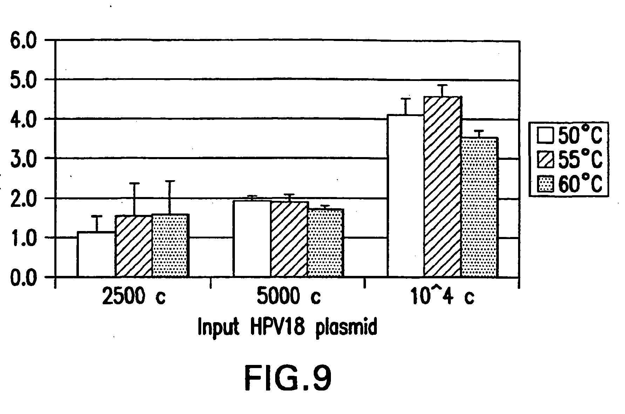

With no RNA-dependant background occurring from synRNA, the hybridization temperature can be reduced, if desired, to provide a more tolerable condition for antibody/antigen interactions (Figure 9).

### L. Exogenous RNase is unnecessary for detection

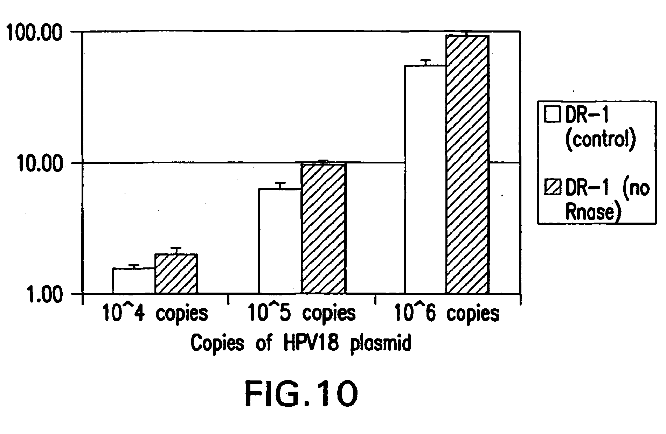

synRNAs are largely devoid of secondary structure. This eliminates non-specific RNA-based background arising from anti-RNA:DNA hybrid antibodies recognizing long RNA secondary structures. With RNA not bound to DNA no longer contributing to background signal, the use of RNase A in the assay becomes unnecessary (Figure 10).

### M. Discussion

The method provided specificity and decreased background, and does not require RNase and is compatible with various media including SurePath, PC, STM and DCM.

The method provided a LOD with a 0.5kb target coverage is of 5pg/mL for HPV18 with an S/N=3, whereas 2.5 kb target coverage could allow target detection to 1pg/mL.

### Example 4: Target capture and amplification

The inclusion of a target amplification component provided enhanced sensitivity. The method detected as low as 10 copies of HPV plasmids or 10 SiHa cells comprising HPV nucleic acid target. The method also provided robust specificity, the ability to distinguish HPV 16 or HPV18 plasmid from all other high- and low-risk HPV types.

Target amplification can involve e.g., generating short amplicons with sequence-specific primers (e.g. Polymerase Chain Reaction) or large amplicons with multiple random primers (e.g. Whole Genome Amplification). Amplified targets can be captured and detected on a variety of different detection platforms.

Hybrid-specific antibodies were coupled to magnetic beads and employed in combination with short type-specific RNA probes for target capture. The sample processing procedure involved capture of targets pre-target amplification and the detection procedure involves capture of targets post-target amplification. To enhance assay sensitivity the isothermal WGA technology was utilized to produce non-specific amplification of any captured targets.

The nucleic acid target of interest was immobilized on a solid support with the use of type-specific RNA probes to form nucleic acid hybrids and anti-RNA:DNA hybrid-specific antibodies to capture, concentrate and purify. The sample preparation process produced single-stranded DNA targets free of amplification inhibitors and non-specific targets and allowed for multiple targets to be captured simultaneously. This was demonstrated by coupling hybrid capture antibodies to magnetic beads and using HPV sequence-specific RNA probes for detection.

Magnetic beads coupled with anti-hybrid antibodies were used to specifically capture amplicons generated by WGA. Short RNA probes were used for specific detection. In addition, anti-RNA:DNA hybrid antibodies coupled with alkaline phosphatase was used for detection.

Table 20 shows a flowchart representing a method steps in accordance with one embodiment. Detection reagent 1 is preferably the detection reagent 1 provided in the digene Hybrid Capture Kit and detection reagent 2 is preferably the detection reagent 2 provided in the digene Hybrid Capture Kit. Detection Reagent 1 comprises alkaline phosphatase-conjugated antibodies to RNA:DNA hybrids and Detection Reagent 2 comprises CDP-Star® with Emerald II (chemiluminescent substrate).

**Table 20. Protocol.**

| **Assay Flow Chart** |
|---|
| **Target Denaturation** |
| **RNA probe hybridization and capture with anti-hybrid antibody** |
| **Wash** |
| **Isothermal Amplification** |
| **Amplicon Denaturation** |
| **RNA probe hybridization and capture with anti-hybrid antibody** |
| **Detection Reagent 1** |
| **Wash** |
| **Detection Reagent 2** |

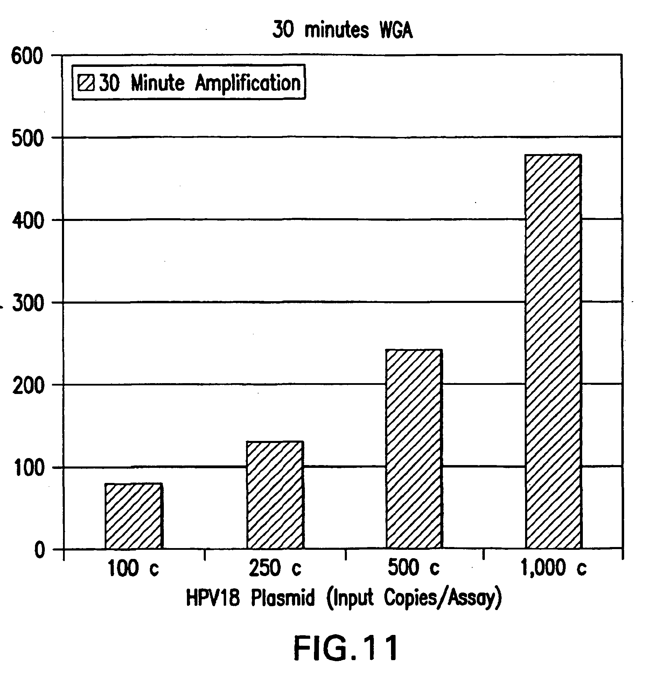

One hundred (100) copies of HPV18 plasmid are obtained after 30 minutes of WGA (Fig. 11)

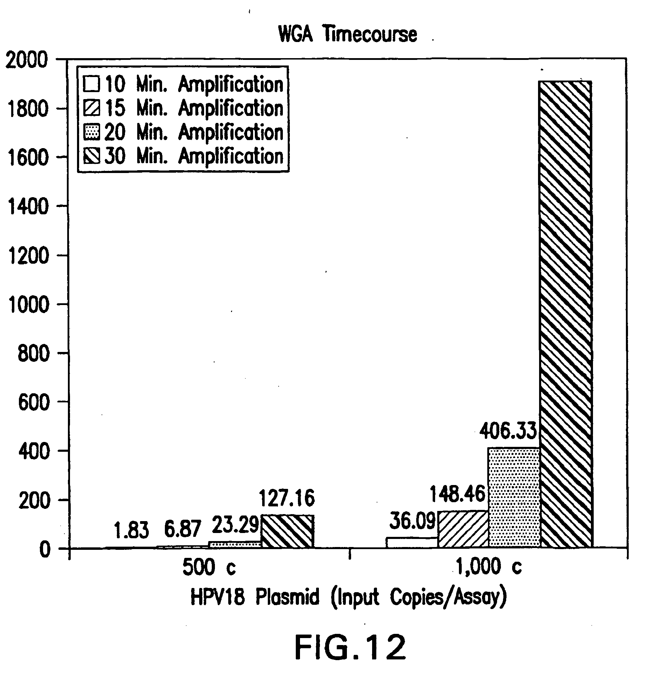

Five hundred (500) copies of HPV18 plasmid are detected after 15 minutes of WGA; and detection of 1000 copies of HPV18 plasmid are obtained after only 10 minutes of WGA (Fig. 12).

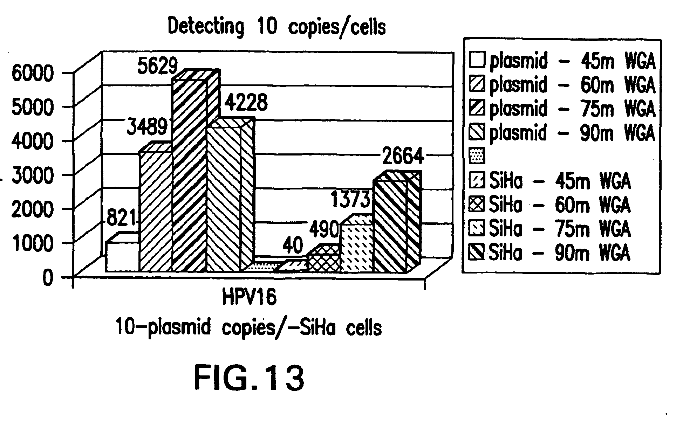

Ten (10) copies of plasmid or 10 SiHa cells comprising HPV nucleic acid are detected with longer amplification times of 45 minutes or greater (Fig. 13).

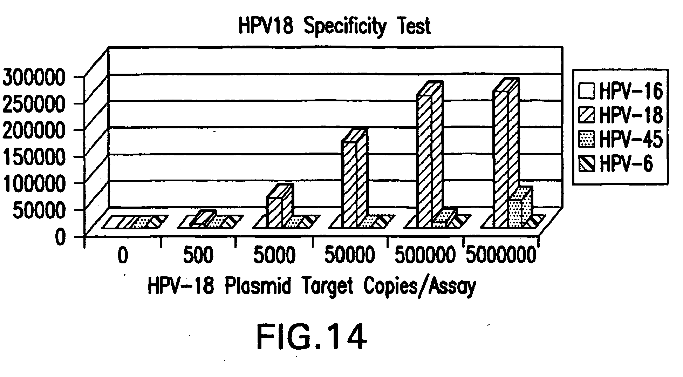

Figure 14 shows specificity for HPV18.

The results demonstrated that after 45 minutes of amplification, as little as 10 copies of plasmid or 10 SiHa cells can be detected; and about 1000 copies of plasmid can be detected after only 10 minutes of amplification.

### Example 5: Synthetic type-specific biotinylated DNA probes DNA probes.

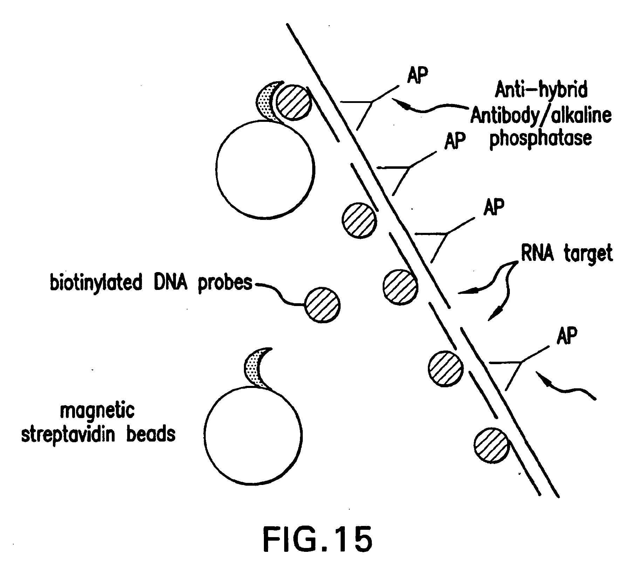

In another embodiment, synthetic type-specific biotinylated DNA probes are used to form double-stranded hybrids with target mRNA (Fig. 15). Hybrids are captured on magnetic streptavidin beads. Signal amplification and detection is performed with anti-hybrid antibody/alkaline phosphatase and the resulting chemiluminescent signal is detected.

### Example 6: Sample Assay Flow.

Predenatured samples are transferred to a multiwell plate. Probes in neutralizing solution are added to the denatured sample and incubated with shaking at room temperature for about 1 minute to neutralize the sample. The neutralized samples are transferred to a plate containing immobilized anti-RNA:DNA hybrid antibodies so that target DNA is allowed to hybridize to the synthetic RNA probes and also to be captured by the immobilized antibodies. The incubation is at about 55°C for about 120 min. Anti-RNA:DNA hybrid antibodies conjugated with alkaline phosphatase are added at room temp and incubated for about 30 min. After the conjugated antibody step, the plate is washed for about 12 min. A dioxetane substrate is added and incubated for 15 minutes. The plate is then read with a luminometer.

Hybridization and hybrid capture by anti-RNA:DNA hybrid antibodies are performed in the same step at about 55°C and may include shaking.

## Claims

1. A method for determining the presence of a target nucleic acid in a sample, wherein the target nucleic acid is a DNA, the method comprising:
a) contacting a probe set comprising more than one polynucleotide probes with the sample under a hybridization condition sufficient for the more than one polynucleotide probes to hybridize to the target DNA in the sample to form double-stranded nucleic acid hybrids, wherein the polynucleotide probes do not hybridize to a variant of the target nucleic acid and wherein the polynucleotide probes of the probe set are short RNA probes having a length from 20 to 60 nucleotides; and
b) detecting the double-stranded nucleic acid hybrids, wherein detecting comprises contacting the double-stranded nucleic acid hybrids with a first anti-hybrid antibody that is immunospecific to double-stranded nucleic acid hybrids to capture the double-stranded nucleic acid hybrids, wherein the first anti-hybrid antibody is directly or indirectly immobilized onto a solid support and wherein the step of detecting further comprises providing a second anti-hybrid antibody that is immunospecific to double-stranded nucleic acid hybrids, wherein the second anti-hybrid antibody is detectably labelled either directly or indirectly, whereby detection of the double-stranded nucleic acid hybrids determines the presence of the target DNA in the sample.

2. The method of Claim 1 wherein the second anti-hybrid antibody is detectably labelled directly.

3. The method of Claim 1 wherein the probe set and the anti-hybrid antibody are added in the same step.

4. The method of Claim 1, wherein the target DNA is an HPV nucleic acid.

5. The method of Claim 4, wherein the HPV nucleic acid is HPV DNA of a high risk HPV type.

6. The method of Claim 5, having one of the following characteristics:
i) the HPV type is HPV 16 and wherein the variant is a nucleic acid of a type selected from the group consisting of: HPV 1, 2, 3, 4, 5, 6, 8, 11, 13, 18, 26, 30, 31, 33, 34, 35, 39, 40, 42, 43, 44, 45, 51, 52, 53, 54, 56, 58, 59, 61, 62, 66, 67, 68, 69, 70, 71, 72, 73, 74, 81, 82, 83, 84, and 89;
ii) the HPV type is HPV 18, wherein the variant is nucleic acid of a type selected from the group consisting of: HPV 1, 2, 3, 4, 5, 6, 8, 11, 13, 16, 26, 30, 31, 33, 34, 35, 39, 40, 42, 43, 44, 45, 51, 52, 53, 54, 50, 58, 59, 61, 62, 66, 67, 68, 69, 70, 71, 72, 73, 74, 81, 82, 83, 84, and 89;
iii) the HPV type is HPV 45, wherein the variant is nucleic acid of a type selected from the group consisting of: HPV 1, 2, 3, 4, 5, 6, 8, 11, 13, 16, 18, 26, 30, 31, 33, 34, 35, 39, 40, 42, 43, 44, 51, 52, 53, 54, 56, 58, 59, 61, 62, 66, 67, 68, 69, 70, 71, 72, 73, 74, 81, 82, 83, 84, and 89.

7. The method of Claim 5, having one of the following characteristics:
i) the HPV type is a hrHPV type and wherein the variant is a nucleic acid of low risk HPV type;
ii) more than one polynucleotide probes consist essentially of a sequence or a complement thereof selected from the group consisting of SEQ ID NOs: 1-2026.

8. The method according to one or more of claims 1 to 7, having one or more of the following features:
i) the set of probes ensures coverage of about 3-4kb of the target nucleic acid;
ii) the polynubleotides In the probe set are determined by identifying a contiguous nucleotide sequence of the target nucleic acid, wherein the contiguous nucleotide sequence is not present in the variant;
iii) the polynucleotide probes have a length of 20 to 50, 25 to 50 or 25 to 30 nucleotides;
iv) the polynucleotide probes in a probe set have the same length and have similar melting temperatures to allow hybridization of all probes in the set under the same hybridization conditions;
v) the polynucleotide probes target adjacent regions in the target DNA which is a HPV DNA.

9. The method according to one or more of claims 1 to 8, wherein a probe mixture comprising multiple sets of probes is used to simultaneously screen for any one of a mixture of desired target nucleic acids, which preferably are high risk HPV types.

10. The method according to one or more of claims 1 to 9, having one or more of the following features:
i) wherein the signal in the assay is increased by increasing the coverage of the RNA probes of the target DNA;
ii) wherein the target DNA is a HPV DNA and wherein the sensitivity is Increased by increasing the concentration of the RNA probes;
iii) wherein the hybridization is performed at about 45 to about 55°C.

11. The method according to one or more of claims 1 to 10, comprising one or more of the following:
i) said first anti-hybrid antibody is directly immobilized onto a solid support;
ii) the method comprises denaturing the DNA, hybridization to the RNA probes and capturing via anti-RNA:DNA hybrid antibodies on a solid support;
iii) the method comprises the step of amplification of the target nucleic acid, preferably whole genome amplification (WGA).

12. A method for determining the presence of HPV 18 DNA in a sample, the method comprising:
a) contacting more than one polynucleotide probes or a complement thereof with the sample under a hybridization condition sufficient to allow the polynucleotides to anneal to a corresponding complementary nucleic acid sequence in the sample to form double-stranded nucleic acid hybrids, wherein the more than one polynucleotide probes is a set of nucleic acid probes, wherein the polynucleotides comprised in the probe set are short RNA probes having a length from 20 to 60 nucleotides and wherein the probe set comprises at least one nucleic acid sequence chosen from the group consisting of: SEQ ID NOs: 163-309; and
b) detecting double-stranded nucleic acid hybrids, wherein detecting comprises contacting the double-stranded nucleic acid hybrids with a first anti-hybrid antibody that is immunospecific to double-stranded nucleic acid hybrids to capture the double-stranded nucleic acid hybrids, wherein the first anti-hybrid antibody is directly or indirectly immobilized onto a solid support and wherein the step of detecting further comprises providing a second anti-hybrid antibody that is immunospecific to double-stranded nucleic acid hybrids, wherein the second anti-hybrid antibody is detectably labelled either directly or indirectly, whereby detection of the double-stranded nucleic acid hybrids indicates the presence of HPV 18 DNA in the sample.

13. A method for determining the presence of HPV 16 DNA in a sample, the method comprising:
a) contacting more than one polynucleotide probes or a complement thereof with the sample under a hybridization condition sufficient to allow the polynucleotides to anneal to a corresponding complementary nucleic acid sequence in the sample to form double-stranded nucleic acid hybrids, wherein the more than one polynucleotide probes is a set of nucleic acid probes, wherein the polynucleotides comprised in the probe set are short RNA probes having a length from 20 to 60 nucleotides and wherein the set comprises at least one nucleic acid sequence chosen from the group consisting of: SEQ ID NOs:I-162; and
b) detecting double-stranded nucleic acid hybrids, wherein detecting comprises contacting the double-stranded nucleic acid hybrids with a first anti-hybrid antibody that is immunospecific to double-stranded nucleic acid hybrids to capture the double-stranded nucleic acid hybrids, wherein the first anti-hybrid antibody is directly or indirectly immobilized onto a solid support and wherein the step of detecting further comprises providing a second anti-hybrid antibody that is immunospecific to double-stranded nucleic acid hybrids, wherein the second anti-hybrid antibody is detectably labelled either directly or indirectly, whereby detection of the double-stranded nucleic acid hybrids indicates the presence of HPV 16 DNA in the sample.

14. A method for determining the presence of HPV 45 DNA in a sample, the method comprising:
a) contacting more than one polynucleotide probes or a complement thereof with the sample under a hybridization condition sufficient to allow the polynucleotides to anneal to a corresponding complementary nucleic acid sequence in the sample to form double-stranded nucleic acid hybrids, wherein the more than one polynucleotide probes is a set of nucleic acid probes, wherein the polynucleotides comprised in the probe set are short RNA probes having a length from 20 to 60 nucleotides and wherein the set comprises at least one nucleic acid sequence chosen from the group consisting of: SEQ ID NOs:842-974; and
b) detecting double-stranded nucleic acid hybrids, wherein detecting comprises contacting the double-stranded nucleic acid hybrids with a first anti-hybrid antibody that is immunospecific to double-stranded nucleic acid hybrids to capture the double-stranded nucleic acid hybrids, wherein the first anti-hybrid antibody is directly or indirectly immobilized onto a solid support and wherein the step of detecting further comprises providing a second anti-hybrid antibody that is immunospecific to double-stranded nucleic acid hybrids, wherein the second anti-hybrid antibody is detectably labelled either directly or indirectly, whereby detection of the double-stranded nucleic acid hybrids indicates the presence of HPV 45 DNA In the sample.

15. A probe set selected from the group consisting of SEQ ID NO; 1-162 (HPV 16); 163-309(HPV 18); 842-974(HPV 45); 310-454(HPV 31); 455-579(HPV 33); 580-722(HPV 35); 723-841(HPV 39); 975- 1120(HPV 51); 1121-1252(HPV 52); 1253-1367(HPV 56); 1368-1497(HPV 58); 1498-1646(HPV 59); 1647-1767(HPV 68); 1768-1875(HPV 68); and 1876-2026(HPV 82).

16. The method of claim 1 wherein the more than one polynucleotide probes is a mixture of probe sets comprising the probes set forth in SEQ ID NO: 1-2026.

## Patentansprüche

1. Ein Verfahren zur Bestimmung des Vorhandenseins einer Target-Nukleinsäure in einer Probe, wobei die Target-Nukleinsäure eine DNA ist, das Verfahren umfassend:
a) Kontaktieren eines Sonden-Sets, umfassend mehr als eine Polynukleotidsonden, mit der Probe unter einer Hybridisierungsbedingung, welche ausreicht, dass die mehr als eine Polynukleotidsonden mit der Target-DNA in der Probe hybridisieren, um doppelsträngige Nukleinsäurehybride zu bilden, wobei die Polynukleotidsonden nicht mit einer Variante der Target-Nukleinsäure hybridisieren, und wobei die Polynukleotidsonden des Sonden-Sets kurze RNA-Sonden mit einer Länge von 20 bis 60 Nukleotiden sind, und
b) Detektieren der doppelsträngigen Nukleinsäurehybride, wobei das Detektieren das Kontaktieren der doppelsträngigen Nukleinsäurehybride mit einem ersten Anti-Hybrid-Antikörper umfasst, der zu doppelsträngigen Nukleinsäurehybriden immunspezifisch ist, um die doppelsträngigen Nukleinsäurehybride zu fangen, wobei der erste Anti-Hybrid-Antikörper direkt oder indirekt auf einem festen Träger immobilisiert ist, und wobei der Schritt des Detektierens ferner das Vorsehen eines zweiten Anti-Hybrid-Antikörpers umfasst, der zu den doppelsträngigen Nukleinsäurehybriden immunspezifisch ist, wobei der zweite Anti-Hybrid-Antikörper entweder direkt oder indirekt nachweisbar markiert ist, wobei die Detektion der doppelsträngigen Nukleinsäurehybride das Vorhandensein der Target-DNA in der Probe bestimmt.

2. Verfahren nach Anspruch 1, wobei der zweite Anti-Hybrid-Antikörperdirekt nachweisbar markiert ist.

3. Verfahren nach Anspruch 1, wobei das Sonden-Set und der Anti-Hybrid-Antikörper im selben Schritt zugegeben werden.

4. Verfahren nach Anspruch 1, wobei die Target-DNA eine HPV-Nukleinsäure ist.

5. Verfahren nach Anspruch 4, wobei die HPV-Nukleinsäure die HPV-DNA eines High-Risk-HPV-Typs ist.

6. Verfahren nach Anspruch 5, eines der folgenden Merkmale aufweisend:
i) der HPV-Typ ist HPV 16, und wobei die Variante eine Nukleinsäure eines Typs ist, ausgewählt aus der Gruppe bestehend aus: HPV 1, 2, 3, 4, 5, 6, 8, 11, 13, 18, 26, 30, 31, 33, 34, 35, 39, 40, 42, 43, 44, 45, 51, 52, 53, 54, 56, 58, 59, 61, 62, 66, 67, 68, 69, 70, 71, 72, 73, 74, 81, 82, 83, 84 und 89;
ii) der HPV-Typ ist HPV 18, und wobei die Variante eine Nukleinsäure eines Typs ist, ausgewählt aus der Gruppe bestehend aus: HPV 1, 2, 3, 4, 5, 6, 8, 11, 13, 16, 26, 30, 31, 33, 34, 35, 39, 40, 42, 43, 44, 45, 1, 52, 53, 54, 56, 58, 59, 81, 82, 66, 67, 68, 69, 70, 71, 72, 73, 74, 81, 82, 83, 84 und 89;
iii) der HPV-Typ ist HPV 45, und wobei die Variante eine Nukleinsäure eines Typs ist, ausgewählt aus der Gruppe bestehend aus: HPV 1, 2, 3, 4, 5, 6, 8, 11, 13, 16, 18, 26, 30, 31, 33, 34, 35, 39, 40, 42, 43, 44, 51, 52, 53, 54, 56, 58, 59, 61, 62, 66, 67, 68, 69, 70, 71, 72, 73, 74, 81, 82, 83, 84 und 89.

7. Verfahren nach Anspruch 5, eines der folgenden Merkmale aufweisend:
i) der HPV-Typ ist ein hrHPV-Typ, und wobei die Variante eine Nukleinsäure eines Low-Risk-HPV-Typs ist;
ii) mehr als eine Polynukleotidsonden bestehen im Wesentlichen aus einer Sequenz oder einem Komplement derselben, ausgewählt aus der Gruppe bestehend aus SEQ ID-Nr. 1 - 2026.

8. Verfahren nach einem oder mehreren der Ansprüche 1 bis 7, aufweisend eines oder mehrere der folgenden Merkmale:
i) das Sonden-Set gewährleistet die Abdeckung von etwa 3 - 4 kb der Target-Nukleinsäure;
ii) die Polynukleotide in dem Sonden-Set werden durch Identifizierung einer zusammenhängenden Nukleotidsequenz der Target-Nukleinsäure bestimmt, wobei die zusammenhängende Nukleotidsequenz in der Variante nicht vorhanden ist;
iii) die Polynukleotidsonden weisen eine Länge von 20 bis 50, 25 bis 50 oder 25 bis 30 Nukleotiden auf;
iv) die Polynukleotidsonden in einem Sonden-Set haben dieselbe Länge und ähnliche Schmelztemperaturen, um die Hybridisierung aller Sonden im Set unter den gleichen Hybridisierungsbedingungen zu ermöglichen;
v) die Polynukleotidsonden zielen auf angrenzende Regionen der Target-DNA ab, welche eine HPV-DNA ist.

9. Verfahren nach einem oder mehreren der Ansprüche 1 bis 8, wobei eine mehrere Sonden-Sets aufweisende Mischung von Sonden für das gleichzeitige Screenen einer beliebigen Mischung von gewünschten Target-Nukleinsäuren verwendet wird, die vorzugsweise High-Risk-HPV-Typen sind.

10. Verfahren nach einem oder mehreren der Ansprüche 1 bis 9, aufweisend eines oder mehrere der folgenden Merkmale:
i) wobei das Signal im Assay durch Erweitern der Abdeckung der RNA-Sonden der Target-DNA erhöht wird;
ii) wobei die Target-DNA eine HPV-DNA ist, und wobei die Empfindlichkeit durch Erhöhung der Konzentration der RNA-Sonden erhöht wird;
iii) wobei die Hybridisierung bei etwa 45 bis etwa 55 °C durchgeführt wird.

11. Verfahren nach einem oder mehreren der Ansprüche 1 bis 10, umfassend eines oder mehrere des Folgenden:
i) der genannte erste Anti-Hybrid-Antikörper wird direkt auf einem festen Träger immobilisiert;
ii) das Verfahren umfasst das Denaturieren der DNA, Hybridisierung an die RNA-Sonden und das Einfangen über Anti-RNA:DNA-Hybrid-Antikörper auf einem festen Träger;
iii) das Verfahren umfasst den Schritt der Amplifikation der Target-Nukleinsäure, vorzugsweise eine Whole Genome Amplification (WGA).

12. Verfahren für das Bestimmen des Vorhandenseins von HPV-18-DNA in einer Probe, das Verfahren umfassend:
a) Kontaktieren mehr als einer Polynukleotidsonden oder eines Komplements derselben mit der Probe unter einer Hybridisierungsbedingung, welche ausreicht, um den Polynukleotiden das Anlagern an eine entsprechende komplementäre Nukleinsäuresequenz in der Probe für das Bilden doppelsträngiger Nukleinsäurehybride zu ermöglichen, wobei die mehr als eine Polynukleotidsonden ein Set von Nukleinsäresonden sind, wobei die Polynukleotide, die in dem Sonden-Set enthalten sind, kurze RNA-Sonden mit einer Länge von 20 bis 60 Nukleotiden sind, und wobei das Sonden-Set mindestens eine Nukleinsäuresequenz aufweist, ausgewählt aus der Gruppe bestehend aus SEQ ID-Nummern 163 - 309, und
b) Detektieren von doppelsträngigen Nukleinsäurehybriden, wobei das Detektieren das Kontaktieren der doppelsträngigen Nukleinsäurehybride mit einem ersten Anti-Hybrid-Antikörper umfasst, der zu doppelsträngigen Nukleinsäurehybriden immunspezifisch ist, um die doppelsträngigen Nukleinsäurehybride zu fangen, wobei der erste Anti-Hybrid-Antikörper direkt oder indirekt auf einem festen Träger immobilisiert ist, und wobei der Schritt des Detektierens ferner das Vorsehen eines zweiten Anti-Hybrid-Antikörpers umfasst, der zu den doppelsträngigen Nukleinsäurehybriden immunspezifisch ist, wobei der zweite Anti-Hybrid-Antikörper entweder direkt oder indirekt nachweisbar markiert ist, wobei die Detektion der doppelsträngigen Nukleinsäurehybride das Vorhandensein von HPV-18-DNA in der Probe anzeigt.

13. Verfahren für das Bestimmen des Vorhandenseins von HPV-16-DNA in einer Probe, das Verfahren umfassend:
a) Kontaktieren mehr als einer Polynukleotidsonden oder eines Komplements derselben mit der Probe unter einer Hybridisierungsbedingung, welche ausreicht, um den Polynukleotiden das Anlagern an eine entsprechende komplementäre Nukleinsäuresequenz in der Probe für das Bilden doppelsträngiger Nukleinsäurehybride zu ermöglichen, wobei die mehr als eine Polynukleotidsonden ein Set von Nukleinsäresonden sind, wobei die Polynukleotide, die in dem Sonden-Set enthalten sind, kurze RNA-Sonden mit einer Länge von 20 bis 60 Nukleotiden sind, und wobei das Set mindestens eine Nukleinsäuresequenz umfasst, ausgewählt aus der Gruppe bestehend aus SEQ ID-Nummern 1 - 162, und
b) Detektieren von doppelsträngigen Nukleinsäurehybriden, wobei das Detektieren das Kontaktieren der doppelsträngigen Nukleinsäurehybride mit einem ersten Anti-Hybrid-Antikörper umfasst, der zu doppelsträngigen Nukleinsäurehybriden immunspezifisch ist, um die doppelsträngigen Nukleinsäurehybride zu fangen, wobei der erste Anti-Hybrid-Antikörper direkt oder indirekt auf einem festen Träger immobilisiert ist, und wobei der Schritt des Detektierens ferner das Vorsehen eines zweiten Anti-Hybrid-Antikörpers umfasst, der zu den doppelsträngigen Nukleinsäurehybriden immunspezifisch ist, wobei der zweite Anti-Hybrid-Antikörper entweder direkt oder indirekt nachweisbar markiert ist, wobei die Detektion der doppelsträngigen Nukleinsäurehybride das Vorhandensein von HPV-16-DNA in der Probe anzeigt.

14. Verfahren für das Bestimmen des Vorhandenseins von HPV-45-DNA in einer Probe, das Verfahren umfassend:
a) Kontaktieren mehr als einer Polynukleotidsonden oder eines Komplements derselben mit der Probe unter einer Hybridisierungsbedingung, welche ausreicht, um den Polynukleotiden das Anlagern an eine entsprechende komplementäre Nukleinsäuresequenz in der Probe für das Bilden doppelsträngiger Nukleinsäurehybride zu ermöglichen, wobei die mehr als eine Polynukleotidsonden ein Set von Nukleinsäresonden ist, wobei die Polynukleotide, die in dem Sonden-Set enthalten sind, kurze RNA-Sonden mit einer Länge von 20 bis 60 Nukleotiden sind, und wobei das Set mindestens eine Nukleinsäuresequenz umfasst, ausgewählt aus der Gruppe bestehend aus SEQ ID-Nummern 842 - 974, und
b) Detektieren von doppelsträngigen Nukleinsäurehybriden, wobei das Detektieren das Kontaktieren der doppelsträngigen Nukleinsäurehybride mit einem ersten Anti-Hybrid-Antikörper umfasst, der zu doppelsträngigen Nukleinsäurehybriden immunspezifisch ist, um die doppelsträngigen Nukleinsäurehybride zu fangen, wobei der erste Anti-Hybrid-Antikörper direkt oder indirekt auf einem festen Träger immobilisiert ist, und wobei der Schritt des Detektierens ferner das Vorsehen eines zweiten Anti-Hybrid-Antikörpers umfasst, der zu den doppelsträngigen Nukleinsäurehybriden immunspezifisch ist, wobei der zweite Anti-Hybrid-Antikörper entweder direkt oder indirekt nachweisbar markiert ist, wobei die Detektion der doppelsträngigen Nukleinsäurehybride das Vorhandensein von HPV-45-DNA in der Probe anzeigt.

15. Sonden-Set, ausgewählt aus der Gruppe bestehend aus SEQ ID-Nr. 1 - 162 (HPV 16), 163 - 309 (HPV 18), 842 - 974 (HPV 45), 310 - 454 (HPV 31), 455 - 579 (HPV 33), 580 - 722 (HPV 35), 723 - 841 (HPV 39), 975 - 1120 (HPV 51), 1121 - 1252 (HPV 52), 1253 - 1367 (HPV 56), 1368 - 1497 (HPV 58), 1498 - 1146 (HPV 59), 1647 - 1767 (HPV 66), 1768 - 1875 (HPV 68) und 1876 - 2026 (HPV 82).

16. Verfahren nach Anspruch 1, wobei die mehr als eine Polynukleotidsonden eine Mischung von Sonden-Sets sind, umfassend die in SEQ ID-Nr. 1 - 2026 beschriebenen Sonden.

## Revendications

1. Procédé permettant de déterminer la présence d'un acide nucléique cible dans un échantillon, ledit acide nucléique cible étant un ADN, ledit procédé comprenant:
a) la mise en contact d'un jeu de sondes comprenant des sondes de plus d'un polynucléotide avec l'échantillon dans des conditions d'hybridation suffisantes pour que les sondes de plus d'un polynucléotide s'hybrident à l'ADN cible dans l'échantillon pour former des hybrides d'acides nucléiques double brin, lesdites sondes polynucléotidiques ne s'hybridant pas à une variante de l'acide nucléique cible et lesdites sondes polynucléotidiques du jeu de sondes étant des sondes d'ARN courtes ayant une longueur allant de 20 à 60 nucléotides; et
b) la détection des hybrides d'acides nucléiques double brin, ladite détection comprenant la mise en contact des hybrides d'acides nucléiques double brin avec un premier anticorps anti-hybride qui est immunospécifique à des hybrides d'acides nucléiques double brin pour capturer les hybrides d'acides nucléiques double brin, ledit premier anticorps anti-hybride étant directement ou indirectement immobilisé sur un support solide et ladite étape de détection comprenant en outre la fourniture d'un second anticorps anti-hybride qui est immunospécifique à des hybrides d'acides nucléiques double brin, ledit second anticorps anti-hybride étant directement ou indirectement marqué de façon à être détectable, grâce à quoi la détection des hybrides d'acides nucléiques double brin détermine la présence de l'ADN cible dans l'échantillon.

2. Procédé selon la revendication 1, ledit second anticorps anti-hybride étant directement ou indirectement marqué de façon à être détectable.

3. Procédé selon la revendication 1, ledit jeu de sondes et ledit anticorps anti-hybride étant ajoutés dans la même étape.

4. Procédé selon la revendication 1, ledit ADN cible étant un acide nucléique de PVH.

5. Procédé selon la revendication 4, ledit acide nucléique de PVH étant un ADN de PVH de type PVH à haut risque.

6. Procédé selon la revendication 5, présentant l'une des caractéristiques suivantes:
i) le type de PVH est le PVH 16 et ladite variante est un acide nucléique d'un type choisi dans le groupe constitué par : le PVH 1, 2, 3, 4, 5, 6, 8, 11, 13, 18, 26, 30, 31, 33, 34, 35, 39, 40, 42, 43, 44, 45, 51, 52, 53, 54, 56, 58, 59, 61, 62, 66, 67, 68, 69, 70, 71, 72, 73, 74, 81, 82, 83, 84 et 89;
ii) le type de PVH est le PVH 18, ladite variante étant un acide nucléique d'un type choisi dans le groupe constitué par : le PVH 1, 2, 3, 4, 5, 6, 8, 11, 13, 16, 26, 30, 31, 33, 34, 35, 39, 40, 42, 43, 44, 45, 51, 52, 53, 54, 56, 58, 59, 61, 62, 66, 67, 68, 69, 70, 71, 72, 73, 74, 81, 82, 83, 84 et 89;
iii) le type de PVH est le PVH 45, ladite variante étant un acide nucléique d'un ns le groupe constitué par: le PVH 1, 2, 3, 4, 5, 6, 8, 11, 13, 16, 18, 26, 30, 31, 33, 34, 35, 39, 40, 42, 43, 44, 51, 52, 53, 54, 56, 58, 59, 61, 62, 66, 67, 68, 69, 70, 71, 72, 73, 74, 81, 82, 83, 84 et 89.

7. Procédé selon la revendication 5, présentant l'une des caractéristiques suivantes :
i) le type de PVH étant un type de PVH à haut risque (PVHhr) et ladite variante ucléique de type HPV à bas risque;
ii) les sondes de plus d'un polynucléotide se composent essentiellement d'une séquence ou d'un complément de celle-ci choisi dans le groupe constitué par les SEQ ID n° : 1 à 2026.

8. Procédé selon l'une ou plusieurs des revendications 1 à 7, présentant l'une ou plusieurs des caractéristiques suivantes:
i) le jeu de sondes assure une couverture d'environ 3 à 4 kb de l'acide nucléique cible;
ii) les polynucléotides dans le jeu de sondes sont déterminés en identifiant une séquence nucléotidique contiguë de l'acide nucléique cible, ladite séquence nucléotidique contiguë n'étant pas présente dans la variante
iii) les sondes polynucléotidiques ont une longueur de 20 à 50, 25 à 50 ou 25 à 30 nucléotides;
iv) les sondes polynucléotidiques dans un jeu de sondes ont la même longueur et ont des températures de fusion similaires pour permettre l'hybridation de toutes les sondes dans le jeu dans les mêmes conditions d'hybridation;
v) les sondes polynucléotidiques ciblent des régions adjacentes dans l'ADN cible qui est un ADN de PVH.

9. Procédé selon l'une ou plusieurs des revendications 1 à 8, un mélange de sondes comprenant de multiples jeux de sondes étant utilisé pour cribler simultanément l'un quelconque d'un mélange d'acides nucléiques cibles souhaités qui sont préférentiellement des types de PVH à haut risque.

10. Procédé selon l'une ou plusieurs des revendications 1 à 9, présentant l'une ou plusieurs des caractéristiques suivantes:
i) ledit signal dans le test est augmenté en augmentant la couverture des sondes d'ARN de l'ADN cible;
ii) ledit ADN cible est un ADN de PVH et ladite sensibilité est augmentée en augmentant la concentration des sondes d'ARN;
iii) ladite hybridation est effectuée à environ 45°C à environ 55°C.

11. Procédé selon l'une ou plusieurs des revendications 1 à 10, comprenant l'une ou plusieurs des caractéristiques suivantes:
i) ledit premier anticorps anti-hybride est directement immobilisé sur un support solide;
ii) ledit procédé comprend la dénaturation de l'ADN, l'hybridation aux sondes d'ARN et la capture via des anticorps anti-hybrides ARN:ADN sur un support solide;
iii) ledit procédé comprend l'étape d'amplification de l'acide nucléique cible, de préférence une amplification génomique totale (WGA).

12. Procédé permettant de déterminer la présence d'ADN de PVH 18 dans un échantillon, ledit procédé comprenant:
a) la mise en contact de sondes de plus d'un polynucléotide ou d'un complément de celles-ci avec l'échantillon dans des conditions d'hybridation suffisantes pour permettre l'annelage des polynucléotides à une séquence d'acides nucléiques complémentaire correspondante dans l'échantillon pour former des hybrides d'acides nucléiques double brin, lesdites sondes de plus d'un polynucléotide étant un jeu de sondes d'acides nucléiques, lesdits polynucléotides compris dans le jeu de sondes étant des sondes d'ARN courtes ayant une longueur allant de 20 à 60 nucléotides et ledit jeu de sondes comprenant au moins une séquence d'acides nucléiques choisie dans le groupe constitué par les SEQ ID n° : 163 à 309; et
b) la détection d'hybrides d'acides nucléiques double-brin, ladite détection comprenant la mise en contact des hybrides d'acides nucléiques double brin avec un premier anticorps anti-hybride qui est immunospécifique à des hybrides d'acides nucléiques double brin pour capturer les hybrides d'acides nucléiques double brin, ledit premier anticorps anti-hybride étant directement ou indirectement immobilisé sur un support solide et ladite étape de détection comprenant en outre la fourniture d'un second anticorps anti-hybride qui est immunospécifique à des hybrides d'acides nucléiques double brin, ledit second anticorps anti-hybride étant directement ou indirectement marqué de façon à être détectable, grâce à quoi la détection des hybrides d'acides nucléiques double brin indique la présence d'ADN de PVH 18 dans l'échantillon.

13. Procédé permettant de déterminer la présence d'ADN de PVH 16 dans un échantillon, ledit procédé comprenant:
a) la mise en contact de sondes de plus d'un polynucléotide ou d'un complément de celles-ci avec l'échantillon dans des conditions d'hybridation suffisantes pour permettre l'annelage des polynucléotides à une séquence d'acides nucléiques complémentaire correspondante dans l'échantillon pour former des hybrides d'acides nucléiques double brin, lesdites sondes de plus d'un polynucléotide étant un jeu des sondes d'acides nucléiques, lesdits polynucléotides compris dans le jeu de sondes étant des sondes d'ARN courtes ayant une longueur allant de 20 à 60 nucléotides et ledit jeu comprenant au moins une séquence d'acides nucléiques choisie dans le groupe constitué par les SEQ ID n° : 1 à 162; et
b) la détection d'hybrides d'acides nucléiques double-brin, ladite détection comprenant la mise en contact des hybrides d'acides nucléiques double brin avec un premier anticorps anti-hybride qui est immunospécifique à des hybrides d'acides nucléiques double brin pour capturer les hybrides d'acides nucléiques double brin, ledit premier anticorps anti-hybride étant directement ou indirectement immobilisé sur un support solide et ladite étape de détection comprenant en outre la fourniture d'un second anticorps anti-hybride qui est immunospécifique à des hybrides d'acides nucléiques double brin, ledit second anticorps anti-hybride étant directement ou indirectement marqué de manière à être détectable, grâce à quoi la détection des hybrides d'acides nucléiques double brin indique la présence d'ADN de PVH 16 dans l'échantillon.

14. Procédé permettant de déterminer la présence d'ADN de PVH 45 dans un échantillon, ledit procédé comprenant:
a) la mise en contact de sondes de plus d'un nucléotide ou d'un complément de celles-ci avec l'échantillon dans des conditions d'hybridation suffisantes pour permettre l'annelage des polynucléotides à une séquence d'acides nucléiques complémentaire correspondante dans l'échantillon pour former des hybrides d'acides nucléiques double brin, lesdites sondes de plus d'un polynucléotide étant un jeu de sondes d'acides nucléiques, lesdits polynucléotides compris dans le jeu de sondes étant des sondes d'ARN courtes ayant une longueur allant de 20 à 60 nucléotides et ledit jeu comprenant au moins une séquence d'acides nucléiques choisie dans le groupe constitué par les SEQ ID n° : 842 à 974; et
b) la détection d'hybrides d'acides nucléiques double brin, ladite détection comprenant la mise en contact des hybrides d'acides nucléiques double brin avec un premier anticorps anti-hybride qui est immunospécifique à des hybrides d'acides nucléiques double brin pour capturer les hybrides d'acides nucléiques double brin, ledit premier anticorps anti-hybride étant directement ou indirectement immobilisé sur un support solide et ladite étape de détection comprenant en outre la fourniture d'un second anticorps anti-hybride qui est immunospécifique à des hybrides d'acides nucléiques double brin, ledit second anticorps anti-hybride étant directement ou indirectement marqué de manière à être détectable, grâce à quoi la détection des hybrides d'acides nucléiques double brin indique la présence d'ADN de PVH 45 dans l'échantillon.

15. Jeu de sondes choisi dans le groupe constitué par les SEQ ID n° : 1 à 162 (PVH 16) ; 163 à 309 (PVH 18); 842 à 974 (PVH 45) ; 310 à 454 (PVH 31) ; 455 à 579 (PVH 33) ; 580 à 722 (PVH 35) ; 723 à 841 (PVH 39) ; 975 à 1120 (PVH 51 ) ; 1121 à 1252 (PVH 52); 1253 à 1367 (PVH 56) ; 1368 à 1497 (PVH 58) ; 1498 à 1646 (PVH 59) ; 1647 à 1767 (PVH 66) ; 1768 à 1875 (PVH 68) et 1876 à 2026 (PVH 82).

16. Procédé selon la revendication 1, lesdites sondes de plus d'un polynucléotide étant un mélange de jeux de sondes comprenant les sondes représentées dans les SEQ ID n° : 1 à 2026.
